# EUROPEAN PATENT APPLICATION

(11) **EP 2 154 130 A1**
(43) Date of publication of application: **17.02.2010**
(21) Application number: 08765230.1
(22) Date of filing: 06.06.2008
(51) Int. Cl.: C07D 213/64, A61K 31/4412, A61K 31/443, A61K 31/4433, A61K 31/4436, A61K 31/4439, A61P 9/00, C07D 213/69, C07D 401/10, C07D 405/12, C07D 409/06, C07D 413/10

(54) **PYRIDONE COMPOUND**

(30) Priority: 07.06.2007 JP 2007151243
(71) Applicant: Astellas Pharma Inc., Tokyo 103-8411 (JP)
(72) Inventor: KAMIKUBO, Takashi, Tokyo 103-8411 (JP); HIRAYAMA, Fukushi, Tokyo 103-8411 (JP); MIURA, Masanori, Tokyo 103-8411 (JP); KOMIYA, Yuriko, Tokyo 103-8411 (JP); OKUDA, Takao, Tokyo 103-8411 (JP); MAKI, Keisuke, Tokyo 103-8411 (JP)
(74) Representative: Gille Hrabal Struck Neidlein Prop Roos
(86) International application number: PCT/JP2008/060417
(87) International publication number: WO 2008/149965

(57) **Abstract**

[Solving Means] The present inventors have conducted extensive studies on an EP4 receptor agonist, and as a result, found that a novel pyridone compound **characterized in that** the 1-position in the pyridone ring is substituted with a group having an acidic group such as a carboxyl group and the 6-position is bonded with an aromatic ring group via lower alkyl, lower alkylene, ether, or thioether, has an excellent EP4 receptor agonistic action, thereby completing the present invention. Since the compound of the present invention has an excellent EP4 receptor agonistic action and a blood flow increasing action in the hindlimb of a rat, it is useful as a pharmaceutical, in particular, an agent for treating peripheral arterial occlusive disease.

## Description

### Technical Field

The present invention relates to a pharmaceutical, in particular, a novel pyridone compound which is useful as an agent for treating peripheral arterial occlusive disease.

### Background Art

Peripheral arterial occlusive disease, caused by artery stenosis/occlusion due to arteriosclerosis and thrombus formation, thus leading the peripheral, in particular, the lower extremities into ischemia, is a disease with symptoms such as coldness, intermittent claudication, pain, ulcers/necrosis of the lower extremities, and the like. As for the diagnosis and treatment of the peripheral arterial occlusive disease, the guidelines are provided in "Trans- Atlantic Inter-Society Consensus for Management of Peripheral Arterial Disease (TASC) II" (Eur. J. Vasc. Endovasc. Surg, 2007, 33 (1), S1). For the improvement of the symptoms of the lower extremities, it is important to improve the blood flow into the ischemic part, and treatment for promoting the resumption of the blood circulation by a pharmaceutical or physical method is carried out. For a drug therapy, drugs having a vasodilating action or a platelet aggregation inhibiting action have been used.

PGE2 is known as one of the metabolites in an arachidonic acid cascade. The PGE2 exhibits various physiological activities such as a pain inducing and increasing action, a pro-inflammatory action, an anti-inflammatory action, an uterine contractile action, a digestive peristalsis promoting action, an awaking action, a gastric acid secretion inhibiting action, a hypotensive action, a platelet aggregation inhibiting action, an angiogenic action, and the like. It has become clear that there are four subtypes of PGE2 receptors, EP 1, EP2, EP3 and EP4, which have wide distributions in various tissues. The activation of the EP1 receptor is believed to cause the increase in intracellular Ca²⁺. For the EP3 receptor, there exist the receptors having different pathways for second-messenger systems. The activation of the EP2 and EP4 receptors is believed to cause the activation of an adenylate cyclase, and thus to increase the intracellular cAMP level (Phsiol. Rev., 1999, 79, 1193).

The EP4 receptor is associated with smooth muscle relaxation through the increase in cAMP (Br. J. Pharmacol., 2001, 134, 313). Further, it is suggested that the platelet aggregation inhibiting action is exhibited via EP4 in that the expression of the EP4 receptors (Circulation, 2001, 104, 1176) and the cAMP increasing action by PGE2 (Prostaglandins, 1996, 52, 175) are also demonstrated in the platelets. From this, the EP4 agonist, which exhibits a blood flow improving action, is expected to be an agent for treating peripheral arterial occlusive disease. In addition to these, it is believed that the EP4 receptor is useful as an agent for treating renal diseases, inflammatory diseases, bone diseases, gastric mucosal protection, glaucoma, and the like, from the viewpoint that it is associated with increase in the renal blood flow (Am. J. Physiol. 279, F755, 2000), inhibition of the mesangium cell proliferation (Kid. Int., 1999, 56, 589), inhibition of the inflammatory cytokine production (Biochem. Pharmacol., 2001, 61, 1153), osteogenesis (Proc. Natl. Acad. Sci. U.S.A., 2002, 99, 4580), secretion of the gastrointestinal mucus (Gastroenterology, 1999, 117, 1352), intraocular pressure control (Patent Documents 1 to 5), and the like.

In Patent Document 1, it has been reported that a compound represented by the following formula (A) has an EP4 receptor agonistic action, and is thus useful for the treatment of glaucoma, osteoporosis, and the like. 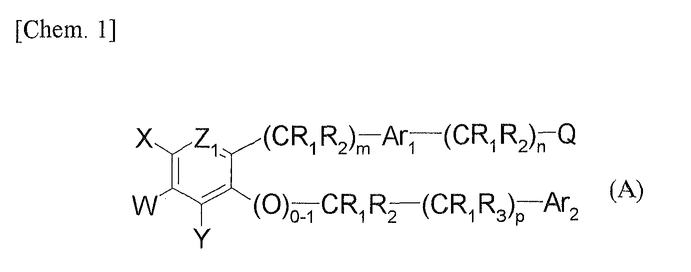 (for the symbols in the formula, refer to the publication.)

In Patent Document 2, it has been reported that a compound represented by the following formula (B) has an EP4 receptor agonistic action, and is thus useful for the treatment of glaucoma, osteoporosis, and the like. 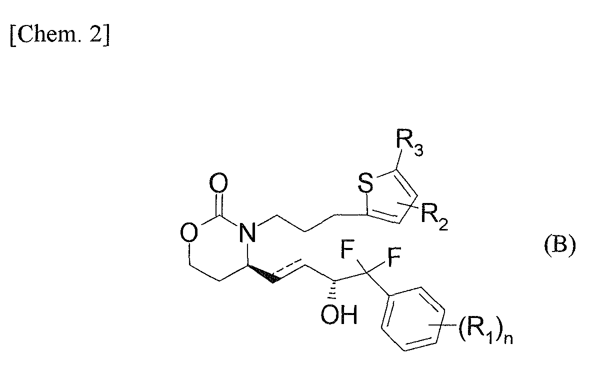 (for the symbols in the formula, refer to the publication.)

In Patent Document 3, it has been reported that a compound represented by the following formula (C) has an EP4 receptor agonistic action, and is thus useful for the treatment of glaucoma, osteoporosis, and the like. 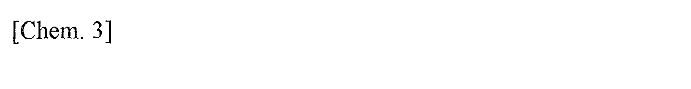 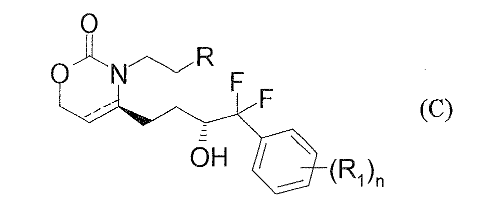 (for the symbols in the formula, refer to the publication.)

In Patent Document 4, it has been reported that a compound represented by the following formula (D) has an EP4 receptor agonistic action, and is thus useful for the treatment of glaucoma, inflammatory bowel disease, and the like. 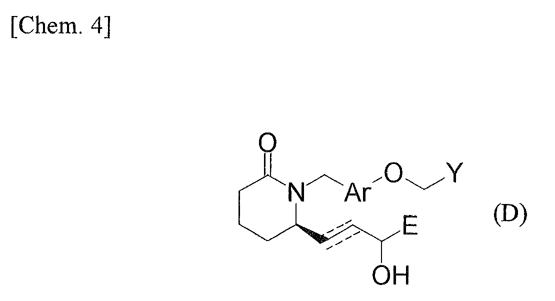 (for the symbols in the formula, refer to the publication.)

In Patent Document 5, it has been reported that a compound represented by the following formula (E) has an EP4 receptor agonistic action, and is thus useful for the treatment of glaucoma, ocular hypertension, and the like. 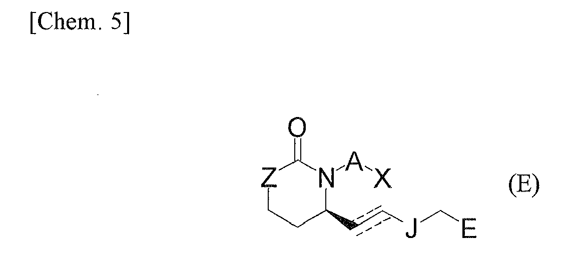 (for the symbols in the formula, refer to the publication.)

In Patent Document 6, it has been reported that a compound represented by the following formula (F) has an EP4 receptor agonistic action, and is thus useful for the treatment of osteoporosis, and other bone diseases. 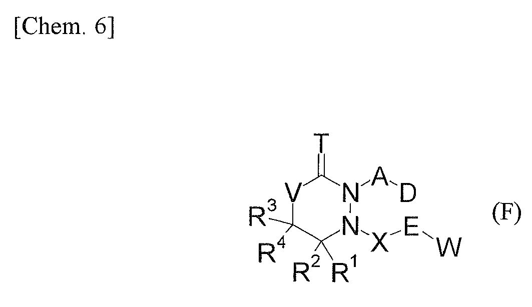 (for the symbols in the formula, refer to the publication.)
In addition, the following compounds have been reported as a pyridone derivative.

In Patent Document 7, it has been reported that a compound represented by the following formula (G) is useful as a plant disease control agent. Further, it has been reported that a compound represented by the following formula (G-1) is useful as a synthesis intermediate. However, there is no disclosure or suggestion of its usefulness as a pharmaceutical. 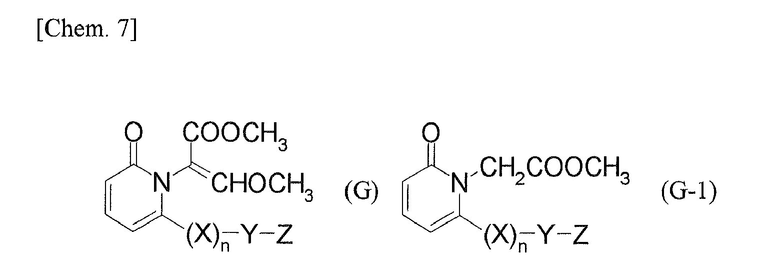 (for the symbols in the formula, refer to the publication.)

In Patent Document 8, it has been reported that a wide range of the compound represented by the following formula (H) exhibit an LXR modulating action, and are thus useful for the treatment of hypercholesterolemia, diabetes, and the like. However, there is no description of specific compounds included in the present invention. In addition, there is no description of the effects on the EP4 receptor and usefulness regarding peripheral arterial occlusive disease. 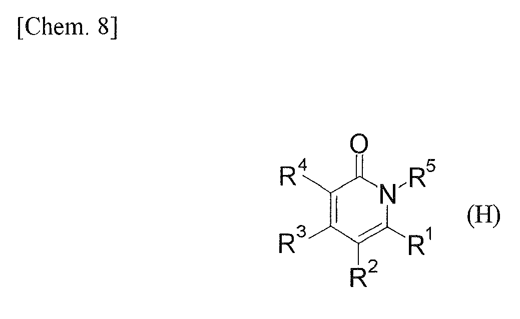 (for the symbols in the formula, refer to the publication.)

[Patent Document 1] Pamphlet of International Publication No. 2005/116010
[Patent Document 2] Pamphlet of International Publication No. 2007/014454
[Patent Document 3] Pamphlet of International Publication No. 2007/014462
[Patent Document 4] Pamphlet of International Publication No. 2006/052630
[Patent Document 5] Pamphlet of International Publication No.2006/014207
[Patent Document 6] Pamphlet of International Publication No.2006/080323
[Patent Document 7] Specification of European Patent Application Publication No. 535980
[Patent Document 8] Pamphlet of International Publication No.2003/059884

### Disclosure of the Invention

### Problem that the Invention is to Solve

It is an object of the present invention to provide a novel pharmaceutical having a selective agonistic action to a prostaglandin EP4 receptor, in particular, a novel compound which is useful as an agent for treating peripheral arterial occlusive disease.

### Means for Solving the Problem

The present inventors have conducted extensive studies on a selective agonist for a prostaglandin EP4 receptor, and as a result, they have found that a novel pyridone derivative **characterized in that** the 1-position in the pyridone ring is substituted with a group having an acidic group and the 6-position is bonded with an aromatic ring group via a linker has an excellent EP4 receptor agonistic action, thereby completing the present invention.
Namely, the present invention relates to a compound of the formula (I) or a pharmaceutically acceptable salt thereof, and a pharmaceutical composition comprising the compound of the formula (I) or a pharmaceutically acceptable salt thereof and a pharmaceutically acceptable excipient 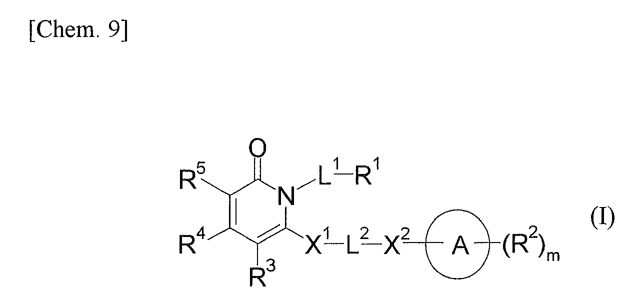 [wherein
Ring A represents aryl or heteroaryl,
X¹, and X² are the same as or different from each other, and represent a single bond, -O-, or -S-,
L¹ represents lower alkylene which may be substituted,
L² represents lower alkylene or lower alkenylene, which may be each substituted,
R¹ represents R⁶ or a group represented by the following formula (II): 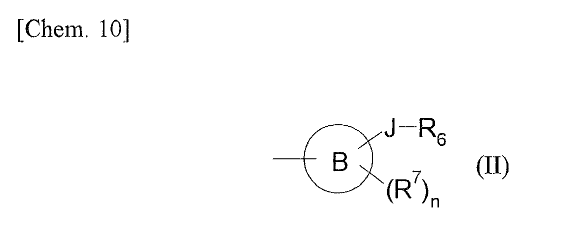 Ring B represents aryl or heteroaryl,
R⁶ represents -CO₂R⁰, -CN, -C(O)-N(R⁰)-S(O)₂-R⁸, -C(O)-N(R⁰)-S(O)₂-N(R⁰)-R⁸, -N(R⁰)-C(O)-N(R⁰)-S(O)₂-R⁸, -C(O)-N(R⁰)-R⁸, or a group represented by the following formula (III) or (IV): 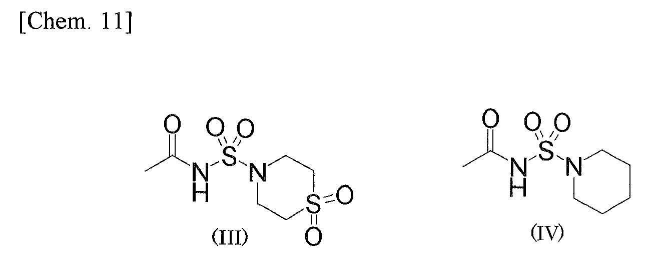 or a group represented by any one of the following formulae (V) to (XIV): 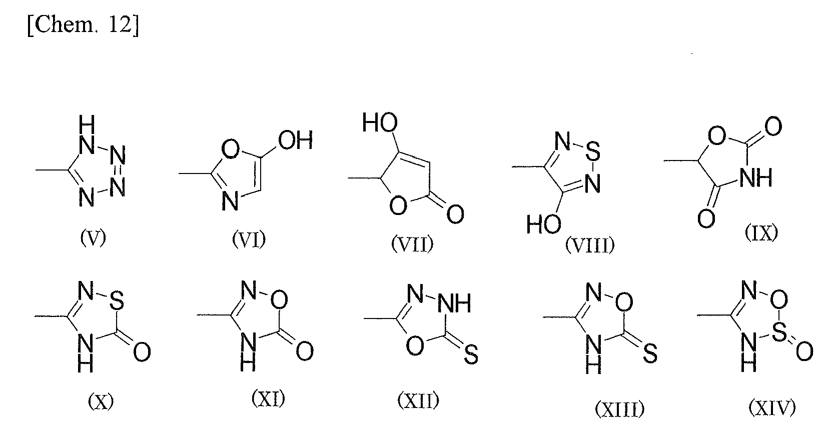 R⁰ are the same as or different from each other, and represent H or lower alkyl,
R⁸ represents H, lower alkyl, halogeno-lower alkyl, cycloalkyl, -(lower alkylene)-OR⁰, -(lower alkylene)-O-C(O)-R⁰, or -(lower alkylene)-CO₂R⁰,
J represents a single bond, lower alkylene, or lower alkenylene,
R² and R⁷ are the same as or different from each other, and represent lower alkyl, halogen, cyano, nitro, halogeno-lower alkyl, -OR⁰, -O-(halogeno-lower alkyl), -O-(cycloalkyl), -O-(lower alkylene)-OR⁰, -N(R⁰)₂, morpholyl, -(lower alkylene)-OR⁰, - (lower alkenylene)-R⁰, or -O-C(O)-R⁰,
m and n are the same as or different from each other, and represent an integer of 0 to 3,
R³, R⁴, and R⁵ are the same as or different from each other, and represent H, halogen, -CN, lower alkyl, lower alkenyl, cycloalkyl, halogeno-lower alkyl, -OR⁰, -O-halogeno-lower alkyl, -CO₂R⁰, -S(O)₂R⁰, or -C(O)N(R⁰)₂,
provided that methyl {6-[(3-methylphenoxy)methyl]-2-oxopyridin-1(2H)-yl}acetate is excluded].

Further, the present invention relates to a pharmaceutical composition for preventing or treating peripheral arterial occlusive disease comprising the compound of the formula (I) or a pharmaceutically acceptable salt thereof, namely, an agent for treating peripheral arterial occlusive disease comprising the compound of the formula (I) or a pharmaceutically acceptable salt thereof.

### Effects of the Invention

Since the compound of the present invention has an EP4 receptor agonistic action, it is useful as an agent for preventing and/or treating peripheral arterial occlusive disease and the like.

### Best Mode for Carrying out the Invention

Hereinbelow, the present invention will be described in detail.
In the present specification, the "lower alkyl" preferably refers to a linear or branched alkyl having 1 to 6 carbon atoms (which is hereinafter simply referred to as C₁₋₆), and specifically, methyl, ethyl, n-propyl, isopropyl, n-butyl, isobutyl, sec-butyl, tert-butyl, n-pentyl, n-hexyl group, or the like. It is more preferably a C₁₋₄ alkyl, and even more preferably methyl or ethyl.

The "lower alkylene" preferably refers to a linear or branched C₁₋₆ alkylene, and specifically, methylene, ethylene, trimethylene, tetramethylene, pentamethylene, hexamethylene, propylene, methylmethylene, ethylethylene, 1,2-dimethylethylene, 1,1,2,2-tetramethylethylene group, or the like. It is more preferably methylene, ethylene, trimethylene, tetramethylene, pentamethylene, or hexamethylene.

The "lower alkenylene" preferably refers to a linear or branched C₂₋₆ alkenylene, and specifically, vinylene, ethylidene, propenylene, butenylene, pentenylene, hexenylene, 1,3-butadienylene, 1,3-pentadienylene group, or the like. It is more preferably C₂₋₄ alkenylene, even more preferably vinylene or propenylene.

The "cycloalkyl" preferably refers to a C₃₋₁₀ saturated hydrocarbon ring group, which may have a bridge. Specifically, it is cyclopropyl, cyclobutyl, cyclopentyl, cyclohexyl, cycloheptyl, cyclooctyl, norbornyl, adamantyl group, or the like. It is preferably cyclopropyl, cyclobutyl, cyclopentyl, or cyclohexyl group.

The "halogen" means F, Cl, Br, or I.
The "halogeno-lower alkyl" refers to C₁₋₆ alkyl substituted with one or more halogen atoms. It is preferably lower alkyl substituted with 1 to 5 halogen atoms, more preferably fluoromethyl, difluoromethyl, trifluoromethyl, 2,2,2-trifluoroethyl, or pentafluoroethyl, and even more preferably trifluoromethyl.

The "aryl" refers to a C₆₋₁₄ monocyclic to tricyclic aromatic hydrocarbon ring group, more preferably phenyl or naphthyl, and even more preferably phenyl.

The "heteroaryl" means a ring group comprising i) a 5- to 6-membered monocyclic heteroaryl comprising 1 to 4 hetero atoms selected from O, S, and N, or ii) a bicyclic 8- to 10-membered heterocycle and a tricyclic 11- to 14-membered heterocycle, each comprising 1 to 5 hetero atoms selected from O, S, and N, which are each formed by condensation of the monocyclic heteroaryl and one or two rings selected from a monocyclic heteroaryl and a benzene ring. The ring atom, S or N, may be oxidized to form an oxide. It is preferably pyrrolyl, imidazolyl, pyrazolyl, triazolyl, tetrazolyl, pyridyl, pyrimidinyl, pyrazinyl, furyl, thienyl, oxazolyl, oxadiazolyl, thiazolyl, thiadiazolyl, indolyl, indazolyl, benzimidazolyl, imidazopyridyl, quinolyl, quinazolyl, quinoxalinyl, naphthylidinyl, benzofuranyl, benzothienyl, benzoxazolyl, benzothiazolyl, or carbazolyl, and more preferably pyridyl, furyl, or thienyl.

The "which may be substituted" refers to "which is not substituted" or "which is substituted with 1 to 5 substituents which are the same as or different from each other". The "which is substituted" refers to "which is substituted with 1 to 5 substituents which are the same as or different from each other". Further, if it has a plurality of substituents, the substituents may be the same as or different from each other.

The substituent at the "lower alkylene" which may be substituted in L¹ is preferably halogen.
The substituent at the "lower alkylene" and the "lower alkenylene", which may be each substituted, in L² is preferably a group selected from the group consisting of halogen and -OR⁰.

The "selective" in the "selective agonist for the EP4 receptor" means that the agonistic action as shown in Test Example 3 to be described later is higher on EP4 than on the subtypes EP1, EP2, and EP3 of the prostaglandin receptor. The difference in the agonistic actions is preferably at least 5-fold, more preferably 10-fold, and even more preferably 100-fold or more.

Preferred embodiments of the compound of the present invention will be described below.
(1) Ring A is phenyl.
(2) -X¹-L²-X²- is preferably lower alkylene, -lower alkylene-O-, or -lower alkenylene-. Further, in another embodiment, -X¹-L²-X²- is lower alkylene, lower alkenylene, -(lower alkylene)-O-, or -(lower alkylene)-S-, and in a further embodiment, it is C₂₋₄ alkylene, C₂₋₄ alkenylene, -CH₂-O-, or -CH₂-S-.
(3) L¹ is linear C₂₋₆ alkylene.
(4) Ring B is phenyl.
(5) R¹ is -CO₂R⁰ or phenyl substituted with -CO₂R⁰. Further, in another embodiment, R¹ is a group represented by the formula (II).
(6) R³ is H or halogen, and in another embodiment, it is halogen. Furthermore, in a further embodiment, R³ is H, Cl, Br, or cyclopropyl.
(7) R⁴ is H.
(8) R⁵ is H or halogen, and in another embodiment, it is halogen. Furthermore, in a further embodiment, R⁵ is H, Cl, Br, or cyclopropyl.
(9) m is 1 or 2.
(10) R⁶ is -CO₂H or a group represented by the formula (IV) in an embodiment. Furthermore, in a further embodiment, R⁶ is -CO₂H.
(11) J is a single bond in an embodiment.
   In other preferred embodiments, the compounds formed by combining each preferred embodiments as described in (1) to (11) above may be mentioned, and it is, for example, the compound as defined in (12) below.

(12) The compound, wherein Ring A is phenyl, -X¹-L²-X²- is a group selected from the group consisting of lower alkylene, lower alkenylene, -(lower alkylene)-O-, and -(lower alkylene)-S-, R¹ is a group represented by the formula (II), R⁴ is H, R³ and R⁵ are each the same as or different from each other, and represent H, Cl, Br, or cyclopropyl, Ring B is phenyl, J is a single bond, and R⁶ is -CO₂H.

Further, examples of the specific compounds included in the present invention include the compound selected from the group shown in (13) and (14) below or pharmaceutically acceptable salts thereof.
(13) 4-(2-{3,5-dichloro-6-[(3-isopropylphenoxy)methyl]-2-oxopyridin-1(2H)-yl}ethyl)benzoic acid,
   4-(2-{3,5-dichloro-6-[2-(2-ethoxyphenyl)ethyl]-2-oxopyridin-1(2H)-yl}ethyl)benzoic acid,
   4-(2-{3,5-dichloro-6-[(E)-2-(3-isopropylphenyl)vinyl]-2-oxopyridin-1(2H)-yl}ethyl)benzoic acid,
   4-{2-[3,5-dichloro-2-oxo-6-{(E)-2-[2-(trifluoromethoxy)phenyl]vinyl}pyridin-1(2H)-yl]ethyl}benzoic acid,
   4-{2-[3,5-dichloro-2-oxo-6-{2-[2-(trifluoromethoxy)phenyl]ethyl}pyridin-1(2H)-yl]ethyl}benzoic acid,
   4-(2-{3,5-dichloro-2-oxo-6-[(3-propylphenoxy)methyl]pyridin-1(2H)-yl}ethyl)benzoic acid,
   4-(2-{3,5-dichloro-6-[(2-isopropoxyphenoxy)methyl]-2-oxopyridin-1(2H)-yl}ethyl)benzoic acid,
   4-(2-{3,5-dichloro-2-oxo-6-[(E)-2-(3-propoxyphenyl)vinyl]pyridin-1(2H)-yl}ethyl)benzoic acid,
   4-(2-{3-chloro-5-cyclopropyl-6-[(3-ethylphenoxy)methyl]-2-oxopyridin-1(2H)-yl}ethyl)benzoic acid,
   4-{2-[3-chloro-5-cyclopropyl-2-oxo-6-{(E)-2-[3-(trifluoromethoxy)phenyl]vinyl}pyridin-1(2H)-yl]ethyl}benzoic acid,
   4-{2-[3-chloro-5-cyclopropyl-2-oxo-6-{2-[3-(trifluoromethoxy)phenyl]ethyl}pyridin-1(2H)-yl]ethyl}benzoic acid,
   4-(2-{5-bromo-6-[(E)-2-(3-ethylphenyl)vinyl]-2-oxopyridin-1(2H)-yl}ethyl)benzoic acid,
   4-(2-{5-chloro-6-[(E)-2-(3-ethylphenyl)vinyl]-2-oxopyridin-1(2H)-yl}ethyl)benzoic acid, and
   4-{2-[5-chloro-2-oxo-6-{(E)-2-[3-(trifluoromethoxy)phenyl]vinyl}pyridin-1(2H)-yl]ethyl}benzoic acid. (14) 4-(2-{3,5-dichloro-6-[(E)-2-(3-methoxyphenyl)vinyl]-2-oxopyridin-1(2H)-yl}ethyl)benzoic acid,
   4-(2-{3,5-dichloro-6-[(3-ethylphenoxy)methyl]-2-oxopyridin-1(2H)-yl}ethyl)benzoic acid,
   4-(2-{3,5-dichloro-2-oxo-6-({[3-(trifluoromethoxy)phenyl]sulfanyl}methyl)pyridin-1(2H)-yl]ethyl}benzoic acid,
   4-{2-[3,5-dichloro-2-oxo-6-{2-[3-(trifluoromethoxy)phenyl]ethyl}pyridin-1(2H)-yl]ethyl}benzoic acid,
   4-{2-[5-bromo-2-oxo-6-{(E)-2-[3-(trifluoromethoxy)phenyl]vinyl}pyridin-1(2H)-yl]ethyl}benzoic acid,
   4-(2-{5-bromo-6-[(E)-2-(3-isopropylphenyl)vinyl]-2-oxopyridin-1(2H)-yl}ethyl)benzoic acid,
   4-(2-{3,5-dichloro-6-[(2-ethoxyphenoxy)methyl]-2-oxopyridin-1(2H)-yl}ethyl)benzoic acid,
   4-(2-{3,5-dichloro-6-[(E)-2-(3-ethylphenyl)vinyl]-2-oxopyridin-1(2H)-yl}ethyl)benzoic acid,
   4-{2-[3,5-dichloro-2-oxo-6-{(E)-2-[3-(trifluoromethoxy)phenyl]vinyl}pyridin-1(2H)-yl]ethyl}benzoic acid, and
   4-(2-{3,5-dichloro-6-[(E)-2-(3-ethoxyphenyl)vinyl]-2-oxopyridin-1(2H)-yl}ethyl)benzoic acid.

The compound of the formula (I) may in some cases exist in the form of other tautomers or geometrical isomers, depending on the kinds of the substituents. In the present specification, the compound may be described in only one form of isomer, but the present invention includes such isomers, isolated forms of the isomers, or a mixture thereof.
Furthermore, the compound of the formula (I) may have asymmetric carbon atoms or axial asymmetries in some cases, and correspondingly, it may exist in the form of optical isomers such as an (R)-form, an (S)-form, and the like. The present invention includes a mixture and an isolated form of these optical isomers.
In addition, the pharmaceutically acceptable prodrugs of the compound of the formula (I) are also included in the present invention. The pharmaceutically acceptable prodrug refers to a compound having a group which can be converted into an amino group, -OH, -CO₂H, or the like, of the present invention, by solvolysis or under a physiological condition. Examples of the group for forming a prodrug include those as described in Prog. Med., 5, 2157-2161 (1985) or "Pharmaceutical Research and Development" (Hirokawa Publishing Company, 1990), vol. 7, Drug Design, 163-198.

Furthermore, the compound of the formula (I) may form an acd addition salt or salt with a base, depending on the kind of the substituents, and the salt is included in the present invention, as long as it is a pharmaceutically acceptable salt. Specifically, examples thereof include acid addition salts with inorganic acids such as hydrochloric acid, hydrobromic acid, hydroiodic acid, sulfuric acid, nitric acid, phosphoric acid, and the like, and with organic acids such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, p-toluenesulfonic acid, aspartic acid, glutamic acid, and the like, and salts with inorganic bases such as sodium, potassium, magnesium, calcium, aluminum, and the like, and organic bases such as methylamine, ethylamine, ethanolamine, lysine, ornithine, and the like, ammonium salts, and others.
Furthermore, the present invention also includes various hydrates or solvates, and polymorphic crystal substances of the compound of the formula (I) and a pharmaceutically acceptable salt thereof. Furthermore, the present invention also includes the compounds labeled with various radioactive isotopes or non-radioactive isotopes.

### (Production Processes)

The compound of the formula (I) and a pharmaceutically acceptable salt thereof can be prepared by applying various known synthesis methods, using the characteristics based on their basic skeletons or the kinds of the substituents. At this time, depending on the types of the functional groups, it is in some cases effective from the viewpoint of the preparation techniques to substitute the functional group with an appropriate protecting group (a group which is capable of being easily converted into the functional group), during the steps from starting materials to intermediates. Examples of such a functional group include an amino group, a hydroxyl group, a carboxyl group, and the like, and examples of the protecting group thereof include those as described in "Protective Groups in Organic Synthesis (4th edition, 2007)", edited by Greene and Wuts, and the like, which may be appropriately selected and used depending on the reaction conditions. In these methods, a desired compound can be obtained by introducing the protecting group to carry out the reaction, and then, if desired, removing the protecting group.
In addition, the prodrug of the compound of the formula (I) can be prepared by introducing a specific group during the steps from starting materials to intermediates, in the same manner as for the above protecting groups, or by carrying out the reaction using the compound of the formula (I) obtained. The reaction can be carried out by applying a method known by a person skilled in the art, such as general esterification, amidation, dehydration, and the like.
Hereinbelow, the representative production processes for the compound of the formula (I) will be explained. Each of the production processes may also be carried out with reference to the References appended in the present description. Further, the production processes of the present invention are not limited to the examples as shown below.

### (Production Process 1)

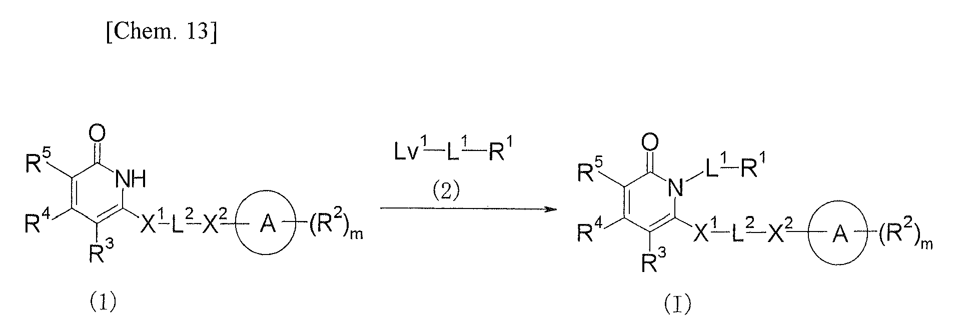

(In the formula, Lv¹ represents a leaving group. The same shall apply hereinafter.)
The present production process is a method for obtaining the compound of the formula (I) by reacting a compound (1) and a compound (2). Here, examples of the leaving group for Lv¹ include halogen, methanesulfonyloxy, p-toluenesulfonyloxy group, and the like.
The reaction is carried out using the compound (1) and the compound (2) in equivalent amounts or either thereof in an excessive amount from under cooling to under heating under reflux, preferably at 0°C to 80°C, usually by stirring for 0.1 hour to 5 days in a solvent which is inert to the reaction or without a solvent. Here, the solvent is not particularly limited, but examples thereof include aromatic hydrocarbons such as benzene, toluene, xylene, and the like, ethers such as diethyl ether, tetrahydrofuran (THF), 1,4-dioxane, 1,2-dimethoxyethane (DME), and the like, halogenated hydrocarbons such as dichloromethane (DCM), 1,2-dichloroethane (DCE), chloroform, and the like, N,N-dimethylformamide (DMF), dimethylsulfoxide (DMSO), ethyl acetate, acetonitrile, water, or a mixture thereof. It may be advantageous in some cases for the smooth progress of the reaction to carry out the reaction in the presence of an inorganic base such as sodium hydride, lithium hydride, n-butyl lithium, potassium carbonate, calcium carbonate, sodium carbonate, sodium hydrogen carbonate, and the like. Further, it may be advantageous in some cases for the smooth progress of the reaction to carry out the reaction in the presence of a phase transfer catalyst such as tetrabutylammonium hydrogen sulfate, a tetrabutylammonium halide, and the like, or a lithium salt such as lithium halide and the like.

### (Production Process 2)

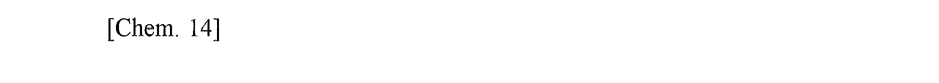

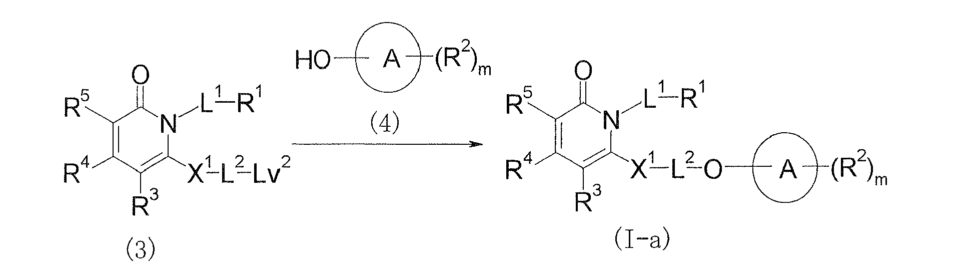

(In the formula, Lv² means a leaving group. The same shall apply hereinafter.)
The present production process is a method for obtaining the compound (I-a) of the present invention, wherein X² is -O-, by reacting a compound (3) and a compound (4). Here, examples of the leaving group for Lv² include halogen, methanesulfonyloxy, p-toluenesulfonyloxy group, and the like.
The reaction is carried out using the compound (3) and the compound (4) in equivalent amounts or either thereof in an excessive amount from under cooling to under heating under reflux, preferably at 0°C to 80°C, usually by stirring for 0.1 hour to 5 days in a solvent which is inert to the reaction in the presence of a base. Here, the solvent is not particularly limited, but examples thereof include aromatic hydrocarbons, ethers, halogenated hydrocarbons, DMF, DMSO, ethyl acetate, acetonitrile, acetone, or a mixture thereof. Examples of the base include organic bases such as triethylamine, diisopropylethylamine (DIPEA), 1,8-diazabicyclo[5.4.0]-7-undecene (DBU), and the like, and inorganic bases such as sodium carbonate, potassium carbonate, sodium hydride, potassium tert-butoxide, and the like.

### (Production Process 3)

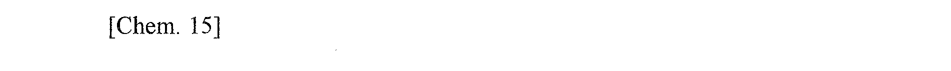

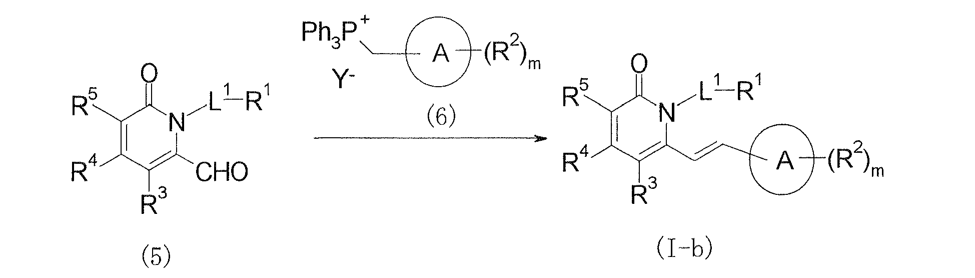

(In the formula, Y⁻ means counter anions such as Cl⁻, Br⁻, and the like. The same shall apply hereinafter.)
The present production process is a method for obtaining the compound (I-b) of the present invention, wherein -X¹-L²-X²- is vinylene, by reacting a compound (5) and a compound (6).
The reaction is carried out using the compound (5) and the compound (6) in equivalent amounts or either thereof in an excessive amount from under cooling to under heating under reflux, preferably at 0°C to 80°C, usually by stirring for 0.1 hour to 5 days in a solvent which is inert to the reaction in the presence of a base. Here, the solvent is not particularly limited, but examples thereof include aromatic hydrocarbons, ethers, or a mixture thereof. Examples of the base include sodium hydride, potassium tert-butoxide, n-butyl lithium, and the like.

### (Production Process 4)

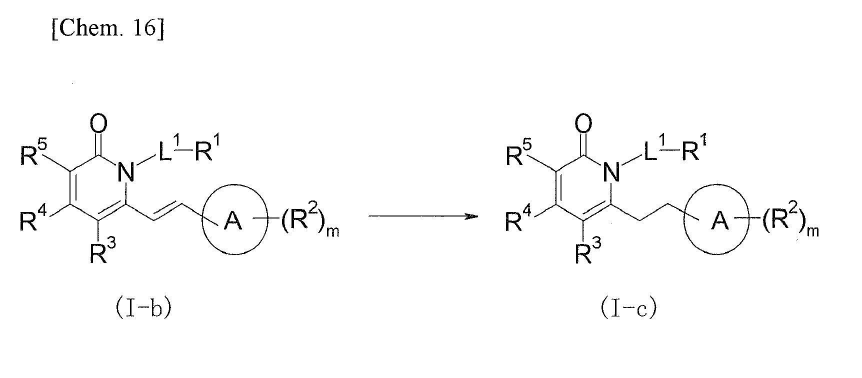

The present production process is a method for obtaining the compound (I-c) of the present invention, wherein -X¹-L²-X²- is ethylene, by hydrogenating the compound (I-b) in the presence of a metal catalyst.
The reaction is carried out using the compound (I-b) from at room temperature to under heating, preferably at room temperature, usually by stirring for 1 hour to 3 days in a solvent which is inert to the reaction in the presence of a catalyst and a hydrogen source. Here, the solvent is not particularly limited, but examples thereof include alcohols, esters, ethers, aromatic hydrocarbons or a mixture thereof. Examples of the catalyst include palladium, rhodium, ruthenium, platinum, and the like. Examples of the hydrogen source include hydrogen, formic acid, ammonium formate, cyclohexene, and the like.

### (Production Process 5)

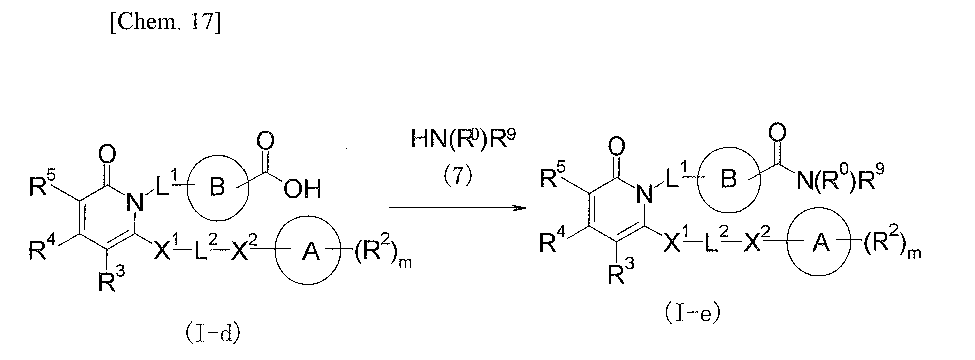

(In the formula, R⁹ means a substituent at the N in the corresponding group represented by R⁶)
The present production process is a method for obtaining the compound (I-e) of the present invention by reacting a compound (I-d) with a compound (7).
In the present reaction, the compound (I-d) and the compound (7) are used in equivalent amounts or in an excessive amount of either thereof, and the mixture thereof is stirred from under cooling to under heating, preferably at -20°C to 60°C, usually for 0.1 hour to 5 days in a solvent which is inert to the reaction in the presence of a condensing agent. Here, the solvent to be used is not particularly limited, but examples thereof include aromatic hydrocarbons such as benzene, toluene, xylene, and the like, halogenated hydrocarbons such as DCM, DCE, chloroform, and the like, ethers such as diethyl ether, THF, dioxane, DME, and the like, DMF, DMSO, ethyl acetate, acetonitrile, or water, and a mixture thereof. Examples of the condensing agent include 1-(3-dimethylaminopropyl)-3-ethylcarbodiimide (WSC), dicyclohexylcarbodiimide (DCC), 1,1'-carbonyldiimidazole (CDI), diphenylphosphoryl azide (DPPA), and phosphorous oxychloride, but are not limited to these. It may be preferable in some cases for the reaction to use an additive (for example, 1-hydroxybenzotriazole) (HOBt). It may be advantageous in some cases for the smooth progress of the reaction to carry out the reaction in the presence of an organic base such as triethylamine, DIPEA, N-methylmorpholine, and the like, or an inorganic base such as potassium carbonate, sodium carbonate, potassium hydroxide, and the like.
Further, a method in which the compound (I-d) is converted into a reactive derivative thereof, followed by reacting with the compound (7) can also be used. Examples of the reactive derivative of the compound (I-d) include an acid halide obtained by the reaction with a halogenating agent such as phosphorus oxychloride, thionyl chloride, and the like, a mixed acid anhydride obtained by the reaction with isobutyl chloroformate or the like, various active esters obtained by the condensation with CDI, HOBt, or the like, and others. The reaction of these reactive derivatives and the compound (7) can be carried out in a solvent which is inert to the reaction, such as halogenated hydrocarbons, aromatic hydrocarbons, ethers, and the like, from under cooling to under heating, preferably at -20°C to 60°C.

### (Production Process 6: other Production Processes)

Moreover, several compounds represented by the formula (I) can be prepared from the compound of the formula (I) obtained above, by any combination of the processes that can be usually employed by a person skilled in the art, such as well-known hydrolysis, amidation, oxidation, reduction, alkylation, and the like. For example, they can be prepared, for example, by the reactions as below, the methods as described in Examples to be described later, the methods known to a skilled person in the art, or a modified method thereof.

### Production Process 6-1: Hydrolysis

A compound having a carboxyl group can be prepared by hydrolyzing a compound having an ester group.
The reaction can be carried out from under cooling to under heating in a solvent such as aromatic hydrocarbons, ethers, halogenated hydrocarbons, alcohols such as methanol, ethanol, and the like, DMF, DMSO, pyridine, water, and the like, or a mixed solvent thereof, in the presence of acids, for example, mineral acids such as sulfuric acid, hydrochloric acid, hydrobromic acid, and the like, organic acids such as formic acid, acetic acid, trifluoroacetic acid, and the like,; or in the presence of bases such as lithium hydroxide, sodium hydroxide, potassium hydroxide, and the like.

### (Production Process of Starting Compound)

The starting materials used for the preparation of the the compound of the formula (I) can be prepared, for example, by the methods as below, the methods as described in Production Examples to be described later, the known methods, or the methods known to a skilled person in the art, or a modified method thereof.

### (Starting Material Synthesis 1)

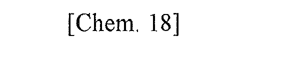

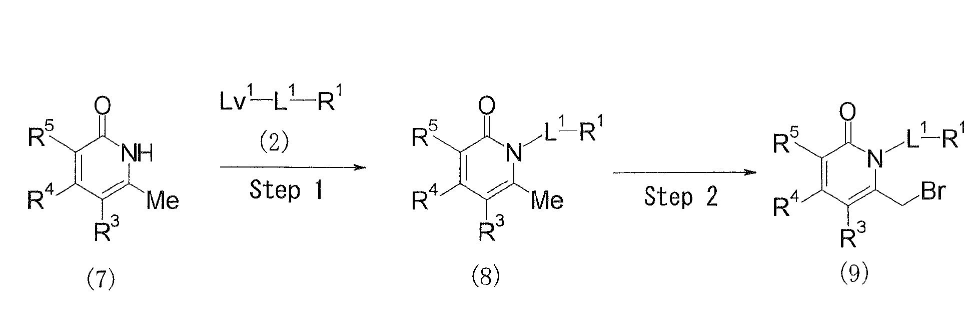

### Step 1

A compound (8) can be obtained by reacting the compound (7) with the compound (2).
The reaction can be carried out in the same manner as in Production Process 1.

### Step 2

A compound (9) can be obtained by the bromidization of the compound (8).
The reaction can be carried out using the compound (8) and N-bromosuccinimide (NBS) under heating in a solvent such as halogenated hydrocarbons, aromatic hydrocarbons, and the like, in the presence of a radical initiator such as 2,2'-azobis(isobutyronitrile) (AIBN), benzoyl peroxide, and the like.

### (Starting Material Synthesis 2)

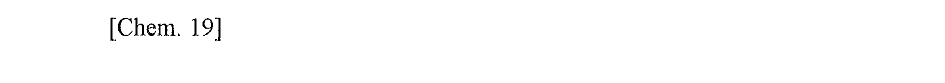

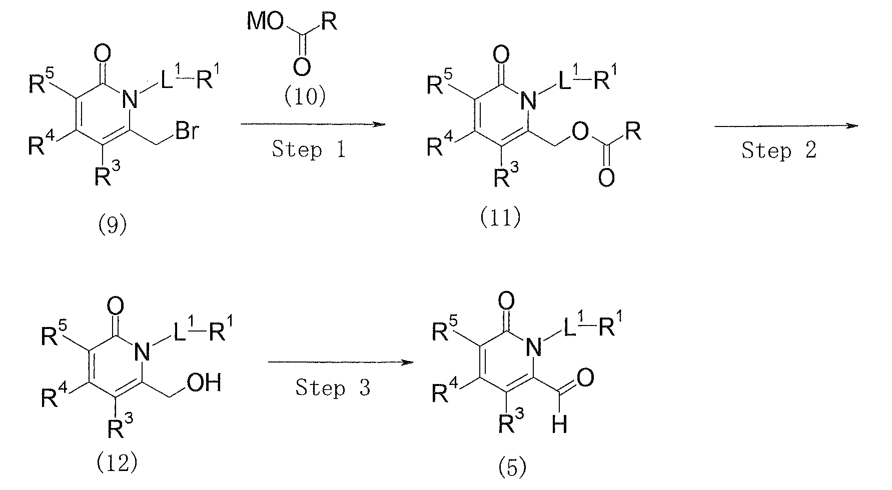

(In the formula, R means lower alkyl and M means an alkali metal such as sodium, potassium, and the like. The same shall apply hereinafter.)

### Step 1

A compound (11) can be obtained by reacting the compound (9) and the compound (10).
The reaction can be carried out from under cooling to under heating under reflux using the compound (9) and the compound (10) in equivalent amounts or either thereof in an excessive amount in a solvent such as aromatic hydrocarbons, ethers, halogenated hydrocarbons, DMF, acetone, and the like. The reaction can also be carried out using a corresponding carboxylic acid compound instead of the compound (10) in the presence of a base such as sodium carbonate, potassium carbonate, sodium hydroxide, and the like.

### Step 2

A compound (12) can be obtained by hydrolyzing the compound (11).
The reaction can be carried out in the same manner as in Production Process 6-1.

### Step 3

The compound (5) can be obtained by oxidizing the compound (12).
The reaction can be carried out using the compound (12) and an oxidizing agent such as manganese dioxide and the like in a solvent such as halogenated hydrocarbons, aromatic hydrocarbons, and the like, from at room temperature to under heating.

### (Starting Material Synthesis 3)

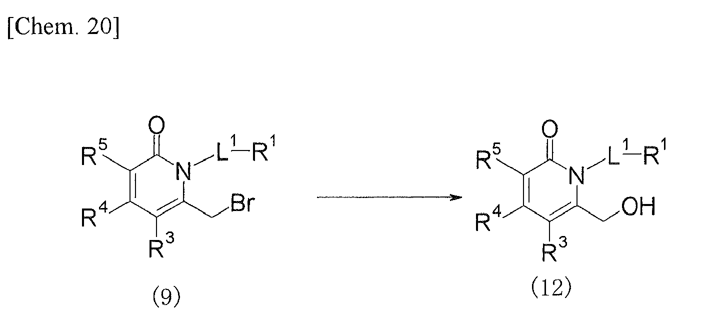

The compound (12) can also be obtained by hydrolyzing the compound (9).
The reaction can be carried out using the compound (9) in a mixed solvent of ethers and water in the presence of Celite from at room temperature to under heating under reflux.

### (Starting Material Synthesis 4)

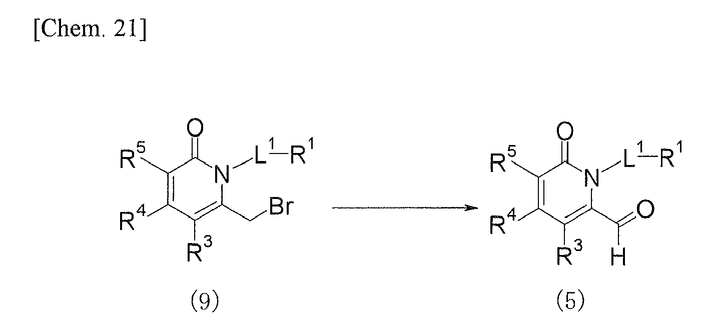

The compound (5) can also be obtained by reacting the compound (9) and a tertiary aminoxide such as trimethylamineoxide and the like.
The reaction can be carried out using the compound (9) and a tertiary aminoxide such as trimethylamineoxide and the like in a solvent such halogenated hydrocarbons, aromatic hydrocarbons, and the like from at room temperature to under heating under reflux.

The compound of the formula (I) is isolated and purified as its free compound, pharmaceutically acceptable salts, hydrates, solvates, or polymorphic crystal substances thereof. The pharmaceutically acceptable salt of the compound of the formula (I) can also be prepared in accordance with a conventional method for a salt formation reaction.
Isolation and purification are carried out by employing general chemical operations such as extraction, fractional crystallization, various types of fractional chromatography, and the like.
Various isomers can be separated by selecting an appropriate starting compound or by making use of the difference in the physicochemical properties between isomers. For example, the optical isomer can be lead into a stereochemically pure isomer by means of general optical resolution methods (for example, fractional crystallization for inducing diastereomer salts with optically active bases or acids, chromatography using a chiral column, etc., and the like). In addition, the isomers can also be prepared from an appropriate optically active starting compound.

The pharmacological activity of the compound of the formula (I) was confirmed by the following test.

### Test Example 1 Evaluation Test on EP4 Receptor Affinity in Rat

### (1) Cell culture and transfection

A rat EP4 receptor cDNA was subcloned into an expression vector (pCDNA3.1-V5-His-topo, manufactured by Invitrogen Corporation) to prepare a rat EP4 expression vector. HEK293 cells were cultured in a collagen type 1-treated 15 cm dish (manufactured by Asahi Techno Glass Co,. Ltd.) to a confluence of 70%. An Opti-MEM culture medium at 1.2 mL/dish and a transfection reagent (Lipofectamine 2000, manufactured by Invitrogen Corporation) at 60 µL/dish were mixed, followed by being left to stand at room temperature for 5 minutes. Then, the rat EP4 receptor expression vector at 15 µg/dish was added thereto, followed by being left to stand at room temperature for 30 minutes. The liquid mixture of the transfection reagent was added into the dish, followed by culturing for 20 to 24 hours. The cell culture was carried out in a CO₂ incubator (37°C, 5% CO₂).

### (2) Preparation of Membrane Fraction

The culture medium was removed by suction, 10 mL of cooled PBS was added thereto per 15 cm dish, and the cells were scraped using a cell scraper. After washed with cooled PBS (1,200 rpm, 4°C, 5 minutes), suspended in 6 mL/dish of cooled 20 mM Tris-HCl (pH 7.4; manufactured by Nacalai Tesque Inc., 5 mM EDTA included) and homogenized using a Polytron, the homogenate was centrifuged (26,000 rpm, 20 minutes, 4°C). The obtained precipitate was resuspended in cooled 20 mM Tris-HCl and homogenized again using a Polytron, and the homogenate was centrifuged (26,000 rpm, 20 minutes, 4°C). The obtained precipitate was resuspended in 50 mM HEPES (pH 7.5; manufactured by Dojindo Laboratories) at 1 mL per dish, homogenized using a Polytron, and freeze-stored at -80°C as a membrane fraction. At this time, a part thereof was used for the measurement of the protein concentration. Measurement of the protein concentration was carried out using a Protein assay stain (manufactured by Bio-Rad Laboratories) in accordance with a standard Protocol as appended in duplicate.

### (3) Receptor Binding Test

[³H]PGE2 50 µL (final concentration 0.3 nM; manufactured by Perkin Elmer Co., Ltd.), 100 µL (20 µg/well) of the membrane fraction prepared from the rat EP4 expression cell, and 50 µL of a test compound were mixed in a 96-well microplate (manufactured by Sumitomo Bakelite Co., Ltd.), incubated at room temperature for 1 hour, then filtered by suction on a microplate (UniFilter-96 GF/B, manufactured by Perkin Elmer Co., Ltd.) using a cell harvester (FilterMate Harvester, manufactured by Perkin Elmer Co., Ltd.), and washed three times with 300 µL/well of a cooled assay buffer (50 mM HEPES, 10 mM MgCl₂). Dilution of [³H]PGE2 and the membrane fraction was carried out using the assay buffer, and dilution of the test compound and the unlabeled PGE2 was carried out using dimethyl sulfoxide and the assay buffer. The UniFilter-96 GF/B was treated by preliminarily washing twice with 200 µL/well of the cooled assay buffer. The UniFilter-96 GF/B after filtration was dried in a dryer overnight, 50 µL/well of a liquid scintillation cocktail (MicroScint20, manufactured by Perkin Elmer Co., Ltd.) was added thereto, and the radioactivity was then measured using a liquid scintillation counter for a microplate (TopCount, manufactured by Perkin Elmer Co., Ltd.). For measurement of the non-specific binding, an unlabeled PGE2 (final concentration 1 µM; manufactured by Cayman Chemical Company) was added. All of the measurements were carried out in duplicate, and the specific binding amount was determined by subtracting the non-specific binding amount from the total binding amount.
According to the test method as described above, the rat EP4 receptor affinity (Ki) of the compound of the present invention was measured. The Ki values of the representative Example Compounds of the present invention are shown below. In addition, Ex means Example Compound No.

**[Table 1]**

| EX | Ki(nM) |
|---|---|
| 1 | 2.8 |
| 2 | 61 |
| 3 | 6.9 |
| 4 | 7.1 |
| 15 | 35 |
| 74 | 10 |
| 90 | 6.2 |
| 91 | 9.4 |
| 99 | 1.7 |
| 106 | 30 |
| 120 | 7.9 |
| 122 | 12 |
| 124 | 4.4 |
| 125 | 7.5 |
| 126 | 4.9 |
| 139 | 8.2 |
| 171 | 1.1 |
| 350 | 19 |

Further, Example compound 4-(2-{3-bromo-5-chloro-2-[(3-methoxybenzyl)oxy]phenyl}ethyl)benzoic acid as described at page 24 of Patent Document 1 was used in the same manner, and evaluated using the method of Test Example 1, and as a result, its Ki value was 27 nM.

### Test Example 2 EP4 Receptor Agonistic Action in Rat

The agonistic activity was evaluated by the cAMP increasing action in the rat EP4 receptor expression cells. The rat EP4 receptor expression vector was introduced into CHO-K1 cells (American Type Culture Collection (ATCC)) to prepare a rat EP4 receptor stable expression cell line. These cells were seeded onto a 96-well microplate at 2×10⁴ cell/well, and used for the experiment the next day. The culture medium of each well was removed by suction, 100 µL/well of an assay culture medium (2 µM indomethacin, 0.1% bovine serum albumin-containing α-MEM) was added thereto, and incubated at 37°C for 1 hour. The culture medium was removed again by suction, and replaced with 100 µL/well of an assay culture medium comprising a test compound and 1 mM IBMX (3-isobutyl-1-methylxanthine). After incubating at 37°C for 30 minutes, the culture medium was removed by suction, 100 µL/well of a cell lysate (0.2% Triton-X100-containing phosphate buffer physiological saline) was put thereinto, and the plate was shaken for 10 minutes. Using a cAMP femto 2 kit (manufactured by Cis Bio International), the concentration of cAMP in the cell lysate was measured.
As a result of the measurement above, when the cAMP increasing action by 1 µM PGE₂ was taken at 100%, the test compounds Ex2 and Ex4 showed a cAMP increasing action of 30% or more at 10 µM.
As shown above, it was confirmed that these compounds had a rat EP4 receptor agonistic action.

### Test Example 3 Evaluation on Selectivity: Rat Prostaglandin EP Receptor Agonistic Action/Antagonistic Action

### (1) Rat EP1 and Rat EP3 Receptor Agonistic Action/Antagonistic Action

Using rat EP1 or rat EP3β receptor stable expression cells, the intracellular Ca²⁺ concentration was measured using a fluorescent imaging plate reader (FLIPR manufactured by Molecular Devices Corporation). The agonistic activity was evaluated by the intracellular Ca²⁺ increasing action of the test compound, and the antagonistic activity was evaluated by the inhibiting action of the test compound on the intracellular Ca²⁺ increasing action by PGE2.
The cDNA of the rat EP1 or EP3β receptor was subcloned into an expression vector (pCDNA3.1-V5-His-topo, manufactured by Invitrogen Corporation). This expression vector was introduced into HEK293 cells (American Type Culture Collection (ATCC)) to prepare a rat EP1 or EP3β receptor stable expression cell line. These cells were seeded onto a 96-well poly-D-lysin treated black wall clear bottom plate (manufactured by Becton, Dickinson and Company) at 2 to 3×10⁴ cell/well, and used for the experiment the next day. The measurement of the intracellular Ca²⁺ concentration was carried out by an FLIPR calcium 3 assay kit (manufactured by Molecular Devices Corporation). The culture medium of each well was removed by suction and replaced with a loading buffer (Hank's balanced salt solution containing 20 mM HEPES-NaOH (pH 7.4), 2.5 mM Probenecid, 0.1% bovine serum albumin, and a color), followed by incubating at room temperature for 3 hours and loading a color. For the evaluation of the agonistic action, the change in the intracellular Ca²⁺ concentration was determined from the difference in the maximum value of the intracellular Ca²⁺ concentration after the addition of the test compound and the value before the addition of the test compound.
For the evaluation of the antagonistic action, after incubating the test compound for 5 minutes, PGE2 was added thereto to determine the change in the intracellular Ca²⁺ concentration by PGE2.

### (2) Rat EP2 Receptor Agonistic Action/Antagonistic Action

For a rat EP2 receptor, stable expression cells were used to carry out a cAMP assay. The agonistic activity was evaluated by the cAMP increasing action by the test compound, and the antagonistic activity was evaluated by the inhibiting action of the test compound on the cAMP increasing action by PGE2.
The rat EP2 receptor cDNA was subcloned into an expression vector (pCDNA3.1-V5-His-topo, manufactured by Invitrogen Corporation). This expression vector was introduced into CHO-K1 cells (American Type Culture Collection (ATCC)) to prepare a rat EP2 receptor stable expression cell line. These cells were seeded onto a 96-well microplate at 0.5×10⁴ cell/well, and used for the experiment the next day. The culture medium of each well was removed by suction, 100 µL/well of an assay culture medium (α-MEM containing 2 µM indomethacin and 0.1% bovine serum albumin) was added thereto, and incubated at 37°C for 1 hour. The culture medium was removed again by suction, and replaced with 100 µL/well of an assay culture medium comprising a test compound and 1 mM IBMX. After incubating at 37°C for 30 minutes, the culture medium was removed by suction, 100 µL/well of a cell lysate (0.2% Triton-X100-containing phosphate buffer physiological saline) was added thereto, and the plate was shaken for 10 minutes. Using a cAMP femto 2 kit (manufactured by Cis Bio International), the cAMP concentration in the cell lysate was measured.

### Test Example 4 LPS Induced TNF-α Production Inhibiting action in RAW264.7 Cells

A mouse macrophage cell line RAW264.7 was seeded onto a 96-well microplate at 5×10⁴ cell/well, and used for the experiment the next day. The culture medium of each well was removed by suction, replaced with 90 µL/well of an assay culture medium (10 µM rolipram-containing D-MEM). After incubation at 37°C for 1 hour, 10 µL/well of an assay culture medium comprising the test compound was added thereto, followed by incubation at 37°C for 30 minutes. Further, 10 µL/well of an assay culture medium comprising 100 ng/mL of LPS was added thereto, the TNF-α concentration in the assay culture medium of each well after 1.5 hours was measured. The measurement was carried out using a BD OptEIA mouse TNF ELISA set (manufactured by Becton, Dickinson and Company) according to the attached method.

### Test Example 5 In Vivo TNF-α Production Inhibiting Action in Rat

LPS (10 µg/kg) was administered to caudal veins of SD male rats, and after 90 minutes from the administration, the heparin blood was collected from the abdominal vena cava to prepare a plasma. The test compound was orally administered 1 hour before the administration of LPS. The amount of TNF-α in the plasma was measured using a BD OptEIA rat TNF ELISA set (manufactured by Becton, Dickinson and Company) according to the attached method. The inhibitory rate by the test compound was determined from the amount of TNF-α in plasma in a control group (administered with a solvent).
As a result of the evaluation of the several compounds of the present invention in Test Example 4 and Test Example 5 above, it was confirmed that since these compounds have a TNF-α production inhibition action, and they have an anti-inflammatory action.

### Test Example 6 Hindlimb Blood Flow Increasing Action in Anesthetized Rat

Wistar male rats were used. The test compound was orally administered, and after 2 hours, the hindlimb blood was measured using a laser blood flow imaging apparatus (PIM II, manufactured by Integral Corporation). At 20 minutes before the measurement, 60 mg/kg of pentobarbital was intraperitoneally administered to conduct anesthesia.
As a result of evaluation of the several compounds of the formula (I), it was confirmed that these compounds exhibit a blood flow increasing action. For example, it was confirmed that the compounds of Examples 5, 161, 194, and 200 exhibit a blood flow increasing action by 120% or more when orally administered at 1 mg/kg. Further, as a result of the evaluation of Example compound 4-(2-{3-bromo-5-chloro-2-[(3-methoxybenzyl)oxy]phenyl}ethyl)benzoic acid as described at page 24 of Patent Document 1 according to the method as shown in Test Example 6 above, the lowest effective dose causing a blood flow increasing action of 120% or more was found to be 3 mg/kg.

As a result of each of the tests above, it was confirmed that the compound of the formula (I) has an EP4 receptor agonistic action, and exhibits an anti-inflammatory action and a blood flow increasing action. Based on this, the compound can be used as an agent for treating peripheral arterial occlusive disease such as arteriosclerosis obliterans, thromboangiitis obliterans, and the like, various symptoms based on peripheral circulatory disorders (intermittent claudication/numbness in lower extremities due to lumbar spinal stenosis, Raynaud's syndrome, erectile dysfunction, and the like), inflammatory diseases such as ulcerative colitis, Crohn's disease, and the like, renal diseases such as nephritis, renal failure, and the like, bone diseases such as osteoporosis and the like, and eye diseases such as glaucoma, ocular hypertension, and the like.

A preparation comprising one or two or more kinds of the compound of the formula (I) or a pharmaceutically acceptable salt thereof as an active ingredient can be prepared in accordance with a generally used method, using a pharmaceutical carrier, excipient, or the like, that is usually used in the art.
The administration can be carried out in any mode of oral administration via tablets, pills, capsules, granules, powders, liquid preparations, or the like, or parenteral administration via injections such as intraarticular, intravenous, intramuscular, or others, suppositories, eye drops, eye ointments, percutaneous liquid preparations, ointments, percutaneous patches, transmucosal liquid preparations, transmucosal patches, inhalations, and the like.

Regarding the solid composition for oral administration according to the present invention, tablets, powders, granules, or the like are used. In such a solid composition, one or two or more kinds of active ingredients are mixed with at least one inert excipient, for example, lactose, mannitol, glucose, hydroxypropylcellulose, microcrystalline cellulose, starch, polyvinyl pyrrolidone, and/or magnesium aluminometasilicate, or the like. According to a conventional method, the composition may contain inert additives for example, a lubricant such as magnesium stearate, a disintegrator such as carboxymethylstarch sodium, a stabilizing agent, and a solubilizing aid. As occasion demands, the tablets or the pills may be coated with a sugar coating, or a film of a gastric or enteric coating agent.
The liquid composition for oral administration includes pharmaceutically acceptable emulsions, soluble liquid preparations, suspensions, syrups, elixirs, or the like, and contains a generally used inert diluent such as purified water or ethanol. In addition to the inert diluent, this liquid composition may contain an adjuvant such as a solubilizing agent, a moistening agent, and a suspending agent, a sweetener, a flavor, an aroma, and an antiseptic.

Injections for parenteral administration contain sterile aqueous or non-aqueous soluble liquid preparations, suspensions and emulsions. The aqueous solvent includes, for example, distilled water for injection or physiological saline. Examples of the non-aqueous solvent include propylene glycol, polyethylene glycol, plant oils such as olive oil, alcohols such as ethanol, Polysorbate 80 (Japanese Pharmacopeia), and the like. Such a composition may further contain a tonicity agent, an antiseptic, a moistening agent, an emulsifying agent, a dispersing agent, a stabilizing agent, or a solubilizing agent These are sterilized, for example, by filtration through a bacteria retaining filter, blending of a bactericide, or irradiation. In addition, these can also be used by preparing a sterile solid composition, and dissolving or suspending it in sterile water or a sterile solvent for injection prior to its use.

The agent for external use includes ointments, plasters, creams, jellies, cataplasms, sprays, lotions, eye drops, eye ointments, and the like. The agents contain generally used ointment bases, lotion bases, aqueous or non-aqueous liquid preparations, suspensions, emulsions, and the like. Examples of the ointment bases or the lotion bases include polyethylene glycol, propylene glycol, white vaseline, bleached bee wax, polyoxyethylene hydrogenated castor oil, glyceryl monostearate, stearyl alcohol, cetyl alcohol, lauromacrogol, sorbitan sesquioleate, and the like.
Regarding the transmucosal agents such as an inhalation, a transnasal agent, and the like, those in the form of a solid, liquid, or semi-solid state are used, and can be prepared in accordance with a conventionally known method. For example, a known excipient, and also a pH adjusting agent, an antiseptic, a surfactant, a lubricant, a stabilizing agent, a thickening agent, or the like may be appropriately added thereto.
For their administration, an appropriate device for inhalation or blowing can be used.
For example, a compound may be administered alone or as a powder of formulated mixture, or as a solution or suspension in combination with a pharmaceutically acceptable carrier, using a conventionally known device or sprayer, such as a measured administration inhalation device, and the like. The dry powder inhaler or the like may be for single or multiple administration use, and a dry powder or a powder-containing capsule may be used. Alternatively, this may be in a form such as a pressurized aerosol spray which uses an appropriate propellant, for example, a suitable gas such as chlorofluoroalkane, hydrofluoroalkane, carbon dioxide, and the like, or other forms.

Generally, in the case of oral administration, the daily dose is from about 0.001 to 100 mg/kg, preferably from 0.1 to 30 mg/kg, and more preferably 0.1 to 10 mg/kg, per body weight, administered in one portion or in 2 to 4 divided portions. In the case of intravenous administration, the daily dose is suitably administered from about 0.0001 to 10 mg/kg per body weight, once a day or two or more times a day. In addition, a transmucosal agent is administered at a dose from about 0.001 to 100 mg/kg per body weight, once a day or two or more times a day. The dose is appropriately decided in response to the individual case by taking the symptoms, the age, the gender, and the like into consideration.

The compound of the formula (I) can be used in combination with various agents for treating or preventing the diseases for which the compound of the formula (I) is considered to be effective. The combined preparation may be administered simultaneously, or separately and continuously or at a desired time interval. The preparations to be co-administered may be a combination drug, or may be prepared individually.

### Examples

Hereinbelow, the production processes for the compound of the formula (I) are described with reference to Examples in more detail. The compounds of the formula (I) are not limited to the compounds as described in Examples below. In addition, the production processes for the starting compounds are shown in Production Examples. Further, the production processes for the compound of the formula (I) are not limited to the production methods of specific Examples as shown below, but the compound of the formula (I) can be prepared by the combination of these production processes therefor or the methods apparent to a skilled person in the art.

### Production Example 1

To a solution of 2.76 g of (3-methoxyphenyl)methanol in 20 ml of DMF was added 1.13 g of 55% sodium hydride (oily) under ice-cooling, followed by stirring for 10 minutes, and then a solution of 2.96 g of 2,6-dichloropyridine in 10 ml ofDMF was added thereto at the same temperature, followed by slowly warming to room temperature and stirring for 2 hours. To the reaction liquid were added water and diethyl ether to carry out a liquid separation operation. The organic layer was washed with saturated aqueous sodium chloride solution and then dried over anhydrous sodium sulfate, and the solvent was evaporated under reduced pressure. The residue was purified by silica gel column chromatography to obtain 4.06 g of 2-chloro-6-[(3-methoxybenzyl)oxy]pyridine.

### Production Example 2

To a solution of 2.24 g of (4-methoxyphenyl)methanol in 20 ml of DMF was added 849 mg of 55% sodium hydride (oily) under ice-cooling, followed by stirring for 20 minutes. A solution of 4.05 g of 2-chloro-6-[(3-methoxybenzyl)oxy]pyridine in 10 ml of DMF was added thereto at the same temperature, followed by slowly warming to room temperature and stirring for 1 hour, and then stirring at 60°C for 14 hours and at 80°C for 1 hour. A saturated aqueous ammonium chloride solution and ethyl acetate were added thereto under ice-cooling to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 4.23 g of a crude product comprising 2-[(3-methoxybenzyl)oxy]-6-[(4-methoxybenzyl)oxy]pyridine.
To a solution of 4.23 g of the obtained crude product comprising 2-[(3-methoxybenzyl)oxy]-6-[(4-methoxybenzyl)oxy]pyridine in 40 ml of DCM was added 2.78 ml of trifluoroacetic acid under ice-cooling, followed by stirring for 1 hour. A saturated aqueous sodium hydrogen carbonate solution and chloroform were added thereto to carry out a liquid separation operation. The organic layer was dried over anhydrous sodium sulfate, and the solvent was evaporated under reduced pressure. The residue was purified by silica gel column chromatography to obtain 1.4 g of 6-[(3-methoxybenzyl)oxy]pyridin-2(1H)-one.

### Production Example 3

To a mixed solution of 500 mg of 3,5-dichloro-6-methylpyridin-2(1H)-one in 3 ml ofDME and 3 ml of DMF was added 147 mg of 55% sodium hydride (oily) at room temperature, followed by stirring for 10 minutes, and then 488 mg of lithium bromide was added thereto, followed by stirring for 5 minutes. 1.25 g of methyl 4-(2-iodoethyl)benzoate was added thereto at the same temperature, followed by stirring at 65°C over two nights. A saturated aqueous ammonium chloride solution and ethyl acetate were added thereto under ice-cooling to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 88 mg of a white solid of methyl 4-[2-(3,5-dichloro-6-methyl-2-oxopyridin-1(2H)-yl)ethyl]benzoate.

### Production Example 4

To a solution of 88 mg of methyl 4-[2-(3,5-dichloro-6-methyl-2-oxopyridin-1(2H)-yl)ethyl]benzoate in 5 ml of carbon tetrachloride were added 51 mg ofNBS, and 5 mg of AIBN, followed by heating under reflux for 30 minutes. After cooling, chloroform and a saturated aqueous sodium hydrogen carbonate solution were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 50 mg of pale yellow oily methyl 4-{2-[6-(bromomethyl)-3,5-dichloro-2-oxopyridin-1(2H)-yl]ethyl}benzoate.

### Production Example 5

To a solution of 2.0 g of methyl 4-(2-bromomethyl)benzoate in 50 ml of acetone was added 2.6 g of sodium iodide at room temperature, followed by stirring overnight. After removing the precipitated salt by filtration, the solvent was evaporated under reduced pressure, and water and ethyl acetate were added thereto to carry out a liquid separation operation. The organic layer was washed with a saturated aqueous sodium chloride solution and then dried over anhydrous sodium sulfate, and the solvent was evaporated under reduced pressure to obtain 2.4 g of a pale yellow solid of methyl 4-(2-iodomethyl)benzoate.

### Production Example 6

To a mixed solution of 373 mg of ethyl 7-[3,5-dibromo-6-(bromomethyl)-2-oxopyridin-1(2H)-yl]heptanoate in 5.0 ml of 1,4-dioxane and 2.5 ml of water was added 500 mg of Celite, followed by stirring at 100°C for 4 days. After cooling, Celite was removed, and water and ethyl acetate were added thereto to carry out a liquid separation operation. The organic layer was washed with a saturated aqueous sodium chloride solution and then dried over anhydrous sodium sulfate, and the solvent was evaporated under reduced pressure to obtain 282 mg of pale yellow oily 7-[3,5-dibromo-6-(hydroxymethyl)-2-oxopyridin-1(2H)-yl]heptanoic acid.

### Production Example 7

To a solution of 280 mg of 7-[3,5-dibromo-6-(hydroxymethyl)-2-oxopyridin-1(2H)-yl]heptanoic acid in 10 ml of ethanol was slowly added 1.0 ml of concentrated sulfuric acid at room temperature, followed by heating under reflux for 2 hours. The reaction system was neutralized by the addition of a saturated aqueous sodium hydrogen carbonate solution under ice-cooling, and ethyl acetate was added thereto to carry out a liquid separation operation. The organic layer was washed with a saturated aqueous sodium chloride solution and dried over anhydrous sodium sulfate, and the solvent was then evaporated under reduced pressure. The residue was purified by silica gel column chromatography to obtain 238 mg of a pale yellow solid of ethyl 7-[3,5-dibromo-6-(hydroxymethyl)-2-oxopyridin-1 (2H)-yl]heptanoate.

### Production Example 8

To a solution of 410 mg of 3,5-dichloro-6-methylpyridin-2(1H)-one in 10 ml of DME was added 319 mg of potassium carbonate at room temperature, followed by stirring at 60°C for 30 minutes, and then 692 mg oftert-butyl 4-{2-[2-(methylsulfonyl)oxy]ethyl}benzoate was added thereto, followed by heating under reflux over two nights. After leaving to be cooled at room temperature, a saturated aqueous ammonium chloride solution and ethyl acetate were added thereto to carry out a liquid separation operation. The organic layer was washed with saturated sodium chloride and then dried over anhydrous sodium sulfate, and the solvent was evaporated under reduced pressure. The residue was purified by silica gel column chromatography to obtain 321 mg of a colorless solid of tert-butyl 4-[2-(3,5- dichloro-6-methyl-2-oxopyridin-1 (2H)yl)ethyl]benzoate.

### Production Example 9

To a solution of 444 mg oftert-butyl 4-[2-(3,5-dichloro-6-methyl-2-oxopyridin-1(2H)-yl)ethyl]benzoate in 10 ml of carbon tetrachloride were added 210 mg ofNBS and 19 mg of AIBN, followed by heating under reflux for 1 hour. After leaving to be cooled at room temperature, chloroform and a saturated aqueous sodium hydrogen carbonate solution were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 393 mg of pale yellow amorphous tert-butyl 4-{2-[6-(bromomethyl)-3,5-dichloro-2-oxopyridin-1(2H)-yl]ethyl}benzoate.

### Production Example 10

To a solution of 390 mg of tert-butyl 4-{2-[6-(bromomethyl)-3,5-dichloro-2-oxopyridin-1(2H)-yl]ethyl}benzoate in 10 ml of acetone was added 347 mg of sodium acetate, followed by stirring at 70°C for 6 hours. After cooling, a saturated aqueous ammonium chloride solution and ethyl acetate were added thereto to carry out a liquid separation operation. The organic layer was washed with a saturated aqueous sodium chloride solution and then dried over anhydrous sodium sulfate, and the solvent was evaporated under reduced pressure to obtain 369 mg of pale yellow amorphous tert-butyl 4-{2-[6-(acetoxymethyl)-3,5-dichloro-2-oxopyridin-1(2H)-yl]ethyl}benzoate.

### Production Example 11

To a solution of 365 mg of tert-butyl 4-{2-[6-(acetoxymethyl)-3,5-dichloro-2-oxopyridin-1(2H)-yl]ethyl}benzoate in 10 ml of methanol was added 343 mg of potassium carbonate at room temperature, followed by stirring for 30 minutes. Ethyl acetate and a saturated aqueous ammonium chloride solution were added thereto to carry out a liquid separation operation. The organic layer was washed with a saturated aqueous sodium chloride solution and then dried over anhydrous sodium sulfate, and the solvent was evaporated under reduced pressure. The obtained pale yellow solid was washed with a mixed solvent of ethyl acetate/hexane to obtain 251 mg of a white solid of tert-butyl 4-{2-[3,5-dichloro-6-(hydroxymethyl)-2-oxopyridin-1(2H)-yl]ethyl}benzoate. On the other hand, the mother liquid was concentrated under reduced pressure, and the residue was then purified by silica gel column chromatography to obtain 54 mg of tert-butyl 4-{2-[3,5-dichloro-6-(hydroxymethyl)-2-oxopyridin-1(2H)-yl]ethyl}benzoate.

### Production Example 12

To a solution of 32 mg of methyl 4-{2-[6-(acetoxymethyl)-3,5-dibromo-2-oxopyridin-1(2H)-yl]ethyl}benzoate in 2.7 ml of methanol was slowly added 0.27 ml of concentrated sulfuric acid at room temperature, followed by heating under reflux for 4 hours. A saturated aqueous sodium hydrogen carbonate solution and ethyl acetate were added thereto under ice-cooling to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 27 mg of a colorless solid of methyl 4-{2-[3,5-dibromo-6-(hydroxymethyl)-2-oxopyridin-1(2H)-yl]ethyl}benzoate.

### Production Example 13

To a solution of 301 mg of tert-butyl 4-{2-[3,5-dichloro-6-(hydroxymethyl)-2-oxopyridin-1(2H)-yl]ethyl}benzoate in 8 ml of chloroform was added 1.3 g of manganese dioxide at room temperature, followed by stirring for 3 days. The solution was filtered using Celite, and the solvent was evaporated under reduced pressure. The residue was purified by silica gel column chromatography to obtain 240 mg of a pale yellow solid of tert-butyl 4-[2-(3,5-dichloro-6-formyl-2-oxopyridin-1(2H)-yl)ethyl]benzoate.

### Production Example 14

To a solution of 5.67 g of 5-bromo-6-methylpyridin-2(1H)-one in 170 ml of DCM was added 4.02 g of N-chlorosuccinimide at room temperature, followed by heating under reflux for 3 hours. After cooling, a saturated aqueous sodium hydrogen carbonate solution was added thereto, and the precipitated solid was collected by filtration to obtain 5.59 g of 5-bromo-3-chloro-6-methylpyridin-2(1H)-one.

### Production Example 15

To a mixed solution of 3.0 g of 5-bromo-3-chloro-6-methylpyridin-2(1H)-one in 30 ml of toluene and 30 ml of water were added 1.35 g of potassium hydrogen carbonate, 458 mg of tetrabutylammonium hydrosulfate, and 3.91 g of methyl 4-(2-iodoethyl)benzoate in this order, followed by heating under reflux overnight. After leaving to be cooled to room temperature, the reaction liquid was neutralized by the addition of citric acid, and ethyl acetate was added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 853 mg of a pale yellow solid of methyl 4-[2-(5-bromo-3-chloro-6-methyl-2-oxopyridin-1(2H)-yl)ethyl]benzoate.

### Production Example 16

To a solution of 690 mg of methyl 4-{2-[5-bromo-6-(bromomethyl)-3-chloro-2-oxopyridin-1(2H)-yl]ethyl}benzoate in 6.9 ml of chloroform was added 224 mg of trimethylamine N-oxide at room temperature, followed by stirring overnight. The solvent was evaporated under reduced pressure and the residue was then purified by silica gel column chromatography to obtain 353 mg of a colorless solid of methyl 4-[2-(5-bromo-3-chloro-6-formyl-2-oxopyridin-1(2H)-yl)ethyl]benzoate.

### Production Example 32

To a solution of 2.93 g of methyl 4-{2-[6-(bromomethyl)-3,5-dichloro-2-oxopyridin-1(2H)-yl]ethyl}benzoate in 58 ml of DMF were slowly added 2.22 g of sodium carbonate and 1.55 g of trimethylamine N-oxide at room temperature, and the reaction system was then subject to pressure reduction using an aspirator, followed by stirring at 40°C for 15 minutes. To the reaction liquid were added ethyl acetate and water to carry out a liquid separation operation. The organic layer was washed with water and a saturated aqueous sodium chloride solution in this order and then dried over anhydrous sodium sulfate, and the solvent was evaporated under reduced pressure. The residue was purified by silica gel column chromatography to obtain 1.65 g of a pale yellow solid of methyl 4-[2-(3,5-dichloro-6-formyl-2-oxopyridin-1(2H)-yl)ethyl]benzoate.

### Production Example 33

To a solution of 100 mg of methyl 4-[2-(3,5-dichloro-6-formyl-2-oxopyridin-1 (2H)-yl)ethyl]benzoate in 3.0 ml of THF was added 52 µl of trimethylsilyl chloride under ice-cooling, followed by stirring for 5 minutes. Then, 113 µl of a 3.0 M methylmagnesium bromide diethyl ether solution was added thereto at the same temperature, followed by further stirring for 20 minutes. A saturated aqueous ammonium chloride solution and ethyl acetate were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 92 mg of pale yellow oily methyl 4-{2-[3,5-dichloro-6-(1-hydroxyethyl)-2-oxopyridin-1(2H)-yl]ethyl}benzoate.

### Production Example 34

To a solution of 1.0 g of [4-(ethoxycarbonyl)cyclohexyl]acetic acid in 10 ml of 1,4-dioxane were added 333 µl of thionyl chloride and one droplet of DMF in this order at room temperature, followed by stirring at the same temperature for 1 hour. The reaction liquid was concentrated under reduced pressure, and to a solution of the residue in 10 ml of THF was added 176 mg of sodium borohydride under ice-cooling, followed by stirring at room temperature overnight. To the reaction liquid were added water and ethyl acetate to carry out a liquid separation operation, and the organic layer was washed with a saturated aqueous sodium chloride solution and then dried over anhydrous sodium sulfate. The solvent was evaporated under reduced pressure to obtain 780 mg of colorless oily ethyl 4-(2-hydroxyethyl)cyclohexanecarboxylate.

### Production Example 35

To a solution of 2.0 g of [3-ethoxy-4-(ethoxycarbonyl)phenyl]acetic acid in 40 ml of THF was added 1.54 g of CDI at room temperature, followed by stirring overnight. 360 mg of sodium borohydride and 20 ml of water were added thereto under ice-cooling, followed by further stirring at room temperature overnight. To the reaction liquid were added water and ethyl acetate to carry out a liquid separation operation, and the organic layer was washed with a saturated aqueous sodium chloride solution and then dried over anhydrous sodium sulfate. The solvent was evaporated under reduced pressure to obtain 2.0 g of colorless oily ethyl 2-ethoxy-4-(2-hydroxyethyl)benzoate.

### Production Example 36

To a solution of 390 mg of ethyl 4-(2-hydroxyethyl)cyclohexanecarboxylate in 3.9 ml of DCM were added 678 µl of triethylamine and 301 µl of methanesulfonyl chloride in this order under ice-cooling, followed by stirring at the same temperature for 2 hours. A saturated aqueous sodium hydrogen carbonate solution and chloroform were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 430 mg of colorless oily ethyl 4-{2-[(methylsulfonyl)oxy]ethyl}cyclohexanecarboxylate.

### Production Example 37

To a mixed solution of 19.6 g of 4-[2-(3,5-dichloro-6-methyl-2-oxopyridin-1 (2H)-yl)ethyl]benzoic acid in 150 ml of THF and 150 ml of butyl alcohol were slowly added, 16 g of di-tert-butyl dicarbonate and 3.7 g of N,N-dimethylpyridine-4-amine, followed by stirring at 60°C overnight. After leaving to be cooled to room temperature, 16 g of di-tert-butyl dicarbonate and 3.7 g of N,N-dimethylpyridin-4-amine were further added thereto, followed by stirring at 60°C overnight. After leaving to be cooled to room temperature again, 7.0 g of di-tert-butyl dicarbonate was added thereto, followed by further stirring at 60°C overnight. After leaving to be cooled to room temperature, water and ethyl acetate were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 18.8 g of a white solid of tert-butyl 4-[2-(3,5-dichloro-6-methyl-2-oxopyridin-1(2H)-yl)ethyl]benzoate.

### Production Example 38

To a solution of 300 mg of methyl 4-(3-hydroxypropyl)benzoate in 3.0 ml of DCM were added 0.28 ml of triethylamine and 0.14 ml of methanesulfonyl chloride in this order under ice-cooling, followed by stirring at the same temperature for 2 hours. To the reaction liquid were added a saturated aqueous sodium hydrogen carbonate solution and chloroform to carry out a liquid separation operation. The organic layer was washed with a saturated aqueous sodium chloride solution and then dried over anhydrous sodium sulfate, and the solvent was evaporated under reduced pressure to obtain methyl 4-{3-[(methylsulfonyl)oxy]propyl}benzoate.
To a solution of 265 mg of 3,5-dichloro-6-methylpyridin-2(1H)-one in 8.4 ml of DME was added 207 mg of potassium carbonate at room temperature, followed by stirring at 60°C for 30 minutes, and then 420 mg of the previously obtained methyl 4-{3-[(methylsulfonyl)oxy]propyl}benzoate was added thereto, followed by heating under reflux overnight. After leaving to be cooled to room temperature, to the reaction liquid were added a saturated aqueous ammonium chloride solution, and ethyl acetate to carry out a liquid separation operation, and the organic layer was dried over saturated sodium chloride and dried over anhydrous sodium sulfate, and the solvent was evaporated under reduced pressure. The residue was purified by silica gel column chromatography to obtain 200 mg of methyl 4-[3-(3,5-dichloro-6-methyl-2-oxopyridin-1(2H)-yl)propyl]benzoate.

### Production Example 39

To a suspension of 62.7 g of 4-hydroxy-6-methyl-2H-pyran-2-one in 40 ml of water was added 497 ml of a 1 M aqueous sodium hydroxide solution at room temperature, followed by stirring at the same temperature for 15 minutes. 50 g of 4-(2-aminoethyl)benzoic acid hydrochloride was added thereto at the same temperature, followed by stirring at 80°C for 24 hours. After leaving to be cooled to room temperature, the reaction liquid was neutralized by the addition of 249 ml of 1 M hydrochloric acid, and 200 ml of methanol was then added thereto, followed by stirring for 30 minutes. The precipitated solid was collected by filtration to obtain 64.6 g of a pale brown solid of 4-[2-(4-hydroxy-6-methyl-2-oxopyridin-1(2H)-yl)ethylbenzoic acid.

### Production Example 40

To a suspension of 67.8 g of 4-[2-(4-hydroxy-6-methyl-2-oxopyridin-1(2H)-yl)ethylbenzoic acid in 500 ml of methanol was slowly added 50 ml of concentrated sulfuric acid at room temperature, followed by heating under reflux for 4 hours. After leaving to be cooled to room temperature, 1500 ml of water was added thereto, and the precipitated solid was collected by filtration to obtain 69.2 g of a pale yellow solid of methyl 4-[2-(4-hydroxy-6-methyl-2-oxopyridin-1(2H)-yl]benzoate.

### Production Example 41

To a suspension of 69.2 g of methyl 4-[2-(4-hydroxy-6-methyl-2-oxopyridin-1(2H)-yl]benzoate in 500 ml of pyridine was added 60 ml of trifluoromethanesulfonic anhydride at 5°C over about 1 hour, followed by stirring at the same temperature for 2 hours. To the reaction liquid were added 500 ml of 1 M hydrochloric acid and 500 ml of water, and the precipitated solid was collected by filtration to obtain a pale brown solid. To the obtained solid were added ethyl acetate and water to carry out a liquid separation operation. The organic layer was washed with a saturated aqueous sodium chloride solution and then dried over anhydrous sodium sulfate, and the solvent was evaporated under reduced pressure to obtain 84.0 g of methyl 4-{2-[6-methyl-2-oxo-4-{[(trifluoromethyl)sulfonyl]oxy}pyridin-1(2H)-yl]ethyl}benzoate. On the other hand, to the above-mentioned filtrate were added ethyl acetate and water to carry out a liquid separation operation, and the same operation was carried out to obtain 20.2 g of methyl 4-{2-[6-methyl-2-oxo-4-{[(trifluoromethyl)sulfonyl]oxy}pyridin-1(2H)-yl]ethyl}benzoate.

### Production Example 42

To a solution of 56 g of methyl 4-{2-[6-methyl-2-oxo-4-{[(trifluoromethyl)sulfonyl]oxy}pyridin-1(2H)-yl]ethyl}benzoate in 300 ml of ethyl acetate were added 28 ml of DIPEA and 2.8 g of 10% palladium-carbon (hydrate product), followed by stirring at room temperature under a hydrogen atmosphere for 4 hours. The reaction liquid was filtered through Celite, and to the mother liquid were added with water and ethyl acetate to carry out a liquid separation operation. The organic layer was washed with a saturated aqueous sodium chloride solution and then dried over anhydrous sodium sulfate, and the solvent was evaporated under reduced pressure. The obtained brown solid was washed with a mixed solvent of ethyl acetate and hexane under heating to obtain 21.3 g of a white solid of methyl 4-[2-(6-methyl-2-oxopyridin-1(2H)-yl)ethyl]benzoate. On the other hand, the filtrate was evaporated under reduced pressure, and the residue was then purified by silica gel column chromatography to obtain 12.3 g of a pale brown solid of methyl 4-[2-(6-methyl-2-oxopyridin-1(2H)-yl)ethyl]benzoate.

### Production Example 43

To a solution of 348 mg oftert-butyl [4-(2-{3,5-dichloro-6-[(E)-2-(3-methoxyphenyl)vinyl]-2-oxopyridin-1(2H)-yl}ethyl)phenyl]carbamate in 3.0 ml of ethyl acetate was added 3.0 ml of a 4 M hydrogen chloride-ethyl acetate solution, followed by stirring at room temperature for 3 hours. The precipitated solid was collected by filtration to obtain 300 mg of 1-[2-(4-aminophenyl)ethyl]-3,5-dichloro-6-[(E)-2-(3-methoxyphenyl)vinyl]pyridin-1(2H)-one hydrochloride.

### Production Example 44

To a solution of 11.8 g of 1-(chloromethyl)-3-isopropylbenzene in 400 ml of toluene was added 37 g of triphenylphosphine, followed by heating under reflux for 3 days. After leaving to be cooled to room temperature, the precipitated solid was collected by filtration to obtain 26.5 g of a pale white solid of (3-isopropylbenzyl)(triphenyl)phosphonium chloride.

### Production Example 45

To a mixed solution of 350 mg of methyl 4-[2-(5-chloro-6-methyl-2-oxopyridin-1 (2H)-yl)ethyl]benzoate in 5.0 ml of acetic acid and 3.0 ml of water was added 210 mg of NBS at room temperature, followed by stirring for 30 minutes. To the reaction liquid was added water, and the precipitated solid was collected by filtration to obtain 430 mg of a pale yellow solid of methyl 4-[2-(3-bromo-5-chloro-6-methyl-2-oxopyridin-1(2H)-yl)ethyl]benzoate.

### Production Example 46

To a solution of 3.3 g of methyl 4-[2-(6-methyl-2-oxopyridin-1(2H)-yl)ethyl]benzoate in 100 ml of acetic acid was added 1.63 g ofN-chlorosuccinimide at room temperature, followed by stirring at 80°C overnight. After leaving to be cooled to room temperature, water was added thereto, and the precipitated solid was collected by filtration. The obtained pale brown solid was purified by silica gel column chromatography to obtain 2.8 g of a pale yellow solid of methyl 4-[2-(5-chloro-6-methyl-2-oxopyridin-1(2H)-yl)ethyl]benzoate.

### Production Example 47

{0073] To a solution of 3.0 g of methyl 4-[2-(6-methyl-2-oxopyridin-1(2H)-yl)ethyl]benzoate in 60 ml of acetic acid was added 2.0 g ofNBS, followed by stirring at room temperature for 3 hours. To the reaction liquid were added water and ethyl acetate to carry out a liquid separation operation, and the organic layer was washed with a saturated aqueous sodium chloride solution and then dried over anhydrous sodium sulfate, and the solvent was evaporated under reduced pressure. The residue was purified by silica gel column chromatography to obtain 2.2 g of a pale yellow solid of methyl 4-[2-(5-bromo-6-methyl-2-oxopyridin-1(2H)-yl)ethyl]benzoate and 556 mg of methyl 4-[2-(3-bromo-6-methyl-2-oxopyridin-1(2H)-yl)ethyl]benzoate, respectively.

### Production Example 48

To a mixed solution of 1.55 g of methyl 4-[2-(5-cyclopropyl-6-methyl-2-oxopyridin-1(2H)-yl)ethyl]benzoate in 25 ml of acetic acid and 25 ml of water was added 997 mg of N-chlorosuccinimide at room temperature, followed by stirring at 70°C overnight. After leaving to be cooled to room temperature, to the reaction liquid were added water and ethyl acetate to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 948 mg of a white solid of methyl 4-[2-(3-chloro-5-cyclopropyl-6-methyl-2-oxopyridin-1(2H)-yl)ethyl]benzoate.

### Production Example 49

To a mixture of 2.2 g of methyl 4-[2-(5-bromo-6-methyl-2-oxopyridin-1(2H)-yl)ethyl]benzoate in 50 ml of toluene and 2.5 ml of water were added 1.08 g of cyclopropylboric acid, 5.34 g of tripotassium phosphate, 141 mg of palladium acetate, and 352 mg of tricyclohexylphosphine in this order, followed by heating under reflux overnight. After leaving to be cooled to room temperature, to the reaction liquid were added ethyl acetate and water, and the insoluble materials were removed by filtration using Celite. To the filtrate were added ethyl acetate and 1 M hydrochloric acid to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 1.59 g of pale yellow amorphous methyl 4-[2-(5-cyclopropyl-6-methyl-2-oxopyridin-1(2H)-yl)ethyl]benzoate.

### Production Example 50

To a solution of 1.0 g of 4-{2-[6-methyl-2-oxo-4-{[(trifluoromethyl)sulfonyl]oxy}pyridin-1(2H)-yl]ethyl}benzoic acid in 20 ml of 1,4-dioxane were added 225 mg of cyclopropylboric acid, 362 mg of potassium carbonate, and 137 mg of tetrakis(triphenylphosphine)palladium (0) at room temperature, followed by heating under reflux for 2 days. After leaving to be cooled to room temperature, the insoluble materials were removed by filtration using Celite, and the filtrate was concentrated under reduced pressure. The residue was purified by silica gel column chromatography to obtain 310 mg of methyl 4-[2-(4-cyclopropyl-6-methyl-2-oxopyridin-1 (2H)-yl)ethyl]benzoate.

### Production Example 51

To a solution of 300 mg of methyl 4-[2-(5-bromo-3-chloro-6-formyl-2-oxopyridin-1(2H)-yl)ethyl]benzoate in 3.0 ml of 1,4-dioxane were added 50 mg of methylboric acid, 313 mg of potassium carbonate, and 87 mg of tetrakis(triphenylphosphine)palladium (0) at room temperature, followed by heating under reflux for 3 days. After leaving to be cooled to room temperature, to the reaction liquid were added water and ethyl acetate, the insoluble materials were removed by filtration using Celite, and a liquid separation operation was carried out. The organic layer was washed with a saturated aqueous sodium chloride solution and then dried over anhydrous sodium sulfate, and the solvent was evaporated under reduced pressure. The residue was purified by silica gel column chromatography to obtain 62 mg of a yellow solid of methyl 4-[2-(3-chloro-6-formyl-5-methyl-2-oxopyridin-1(2H)-yl)ethyl]benzoate.

### Production Example 52

To a suspension of 5.0 g of 3,5-dichloro-6-methylpyridin-2(1H)-one in 100 ml of chloroform were added 5.0 g of a 2,4,6-trivinylcyclotriboroxane/pyridine complex, 6.25 g of copper(II) acetate, 3.18 ml of pyridine, and 5.0 g of 4 Å Molecular Sieves, followed by stirring at room temperature overnight under an oxygen atmosphere. Then, 1 M hydrochloric acid was added thereto, the reaction liquid was filtered through Celite, and then to the filtrate were added water and chloroform to carry out a liquid separation operation. The organic layer was washed with a saturated aqueous sodium chloride solution and then dried over anhydrous sodium sulfate, and the solvent was evaporated under reduced pressure to obtain 5.2 g of 3,5-dichloro-6-methyl-1-vinylpyridin-2(1H)-one.

### Production Example 53

To a mixture of 200 mg of 3,5-dichloro-6-methyl-1-vinylpyridin-2(1H)-one and 5.0 ml of triethylamine were added 513 mg of methyl 4-iodobenzoate, 20 mg of palladium acetate, and 30 mg oftris(2-methylphenyl)phosphine, followed by stirring at 80°C for 3 hours. After leaving to be cooled to room temperature, the precipitated solid was collected by filtration to obtain 168 mg of a pale brown solid of methyl 4-[(E)-2-(3, 5-dichloro-6-methyl-2-oxopyridin-1 (2H)-yl)vinyl]benzoate.

### Production Example 54

To a mixed solvent of 21.5 g of methyl 4-[2-(3,5-dichloro-6-methyl-2-oxopyridin-1(2H)-yl)ethyl]benzoate in 130 ml of THF and 70 ml of methanol was added 76 ml of a 1 M aqueous sodium hydroxide solution, followed by stirring at 70°C for 4 hours. After leaving to be cooled at room temperature, the reaction liquid was neutralized by the addition of 1 M hydrochloric acid, and the precipitated solid was collected by filtration to obtain 19.6 g of a pale brown solid of 4-[2-(3,5-dichloro-6-methyl-2-oxopyridin-1(2H)-yl)ethyl]benzoic acid.

### Production Example 55

To a solution of 330 mg of 4-[2-(3,5-dichloro-6-methyl-2-oxopyridin-1(2H)-yl)ethyl]benzoic acid in 6.6 ml of DMF was added 246 mg of CDI, followed by stirring at the same temperature overnight. The reaction liquid was poured into aqueous ammonia, and the precipitated solid was collected by filtration to obtain 300 mg of 4-[2-(3,5-dichloro-6-methyl-2-oxopyridin-1(2H)-yl)ethyl]benzamide.

### Production Example 56

A mixture of 306 mg of 4-[2-(3,5-dichloro-6-methyl-2-oxopyridin-1(2H)-yl)ethyl]benzamide, 6.0 ml ofDMF, and 0.15 ml ofthionyl chloride was stirred at 80°C overnight. After leaving to be cooled to room temperature, water was added thereto, and the precipitated solid was collected by filtration to obtain 270 mg of 4-[2-(3,5-dichloro-6-methyl-2-oxopyridin-1(2H)-yl)ethyl]benzonitrile.

### Production Example 57

To a suspension of 239 mg of 4-(2-{3,5-dichloro-6-[(E)-2-(3-methoxyphenyl)vinyl]-2-oxopyridin-1(2H)-yl}ethyl)benzonitrile and 117 mg of hydroxylamine hydrochloride in 2.5 ml of ethanol was added 235 µl of triethylamine, followed by stirring at 80°C for 5 hours. After leaving to be cooled to room temperature, water and ethyl acetate were added thereto to carry out a liquid separation operation. The organic layer was washed with a saturated aqueous sodium chloride solution and then dried over anhydrous sodium sulfate, and the solvent was evaporated under reduced pressure to obtain 243 mg of a yellow solid of 4-(2-{3,5-dichloro-6-[(E)-2-(3-methoxyphenyl)vinyl]-2-oxopyridin-1(2H)-yl}ethyl)-N'-hydroxybenzenecarboxyimidamide.

### Production Example 58

To a solution of 239 mg of 4-(2-{3,5-dichloro-6-[(E)-2-(3-methoxyphenyl)vinyl]-2-oxopyridin-1(2H)-yl}ethyl)-N'-hydroxybenzenecarboxyimidamide and 63 µl of pyridine in 2.5 ml of DMF was slowly added 101 µl of2-ethylhexyl chlorocarbonate under ice-cooling, followed by stirring at the same temperature for 1 hour. Then, water and ethyl acetate were added thereto to carry out a liquid separation operation. The organic layer was washed with a saturated aqueous sodium chloride solution and then dried over anhydrous sodium sulfate, and the solvent was evaporated under reduced pressure to obtain 300 mg of a white solid of 4-(2-{3,5-dichloro-6-[(E)-2-(3-methoxyphenyl)vinyl]-2-oxopyridin-1(2H)-yl}ethyl)-N'-({[(2-ethylhexyl)oxy]carbonyl}oxy)benzenecarboxyimidamide.

### Production Example 59

To a solution of 189 mg of 4-(2-{3,5-dichloro-6-[(3-ethylphenoxy)methyl]-2-oxopyridin-1(2H)-yl}ethyl)benzonitrile and 93 mg of hydroxylamine hydrochloride in 2.0 ml of DMSO was added 185 µl of triethylamine, followed by stirring at 80°C for 5 hours. After leaving to be cooled to room temperature, water and ethyl acetate were added thereto to carry out a liquid separation operation. The organic layer was washed with a saturated aqueous sodium chloride solution and then dried over anhydrous sodium sulfate, and the solvent was evaporated under reduced pressure to obtain 203 mg of a yellow solid of 4-(2-{3,5-dichloro-6-[(3-ethylphenoxy)methyl]-2-oxopyridin-1(2H)-yl}ethyl)-N'-hydroxybenzenecarboxyimidamide.
To a solution of 120 mg of 4-(2-{3,5-dichloro-6-[(3-ethylphenoxy)methyl]-2-oxopyridin-1(2H)-yl}ethyl)-N'-hydroxybenzenecarboxyimidamide and 32 µl of pyridine in 3.0 ml of DMF was slowly added 56 µl of 2-ethylhexyl chlorocarbonate under ice-cooling, followed by stirring at the same temperature for 1 hour. Water and ethyl acetate were added thereto to carry out a liquid separation operation. The organic layer was washed with a saturated aqueous sodium chloride solution and then dried over anhydrous sodium sulfate, and the solvent was evaporated under reduced pressure to obtain 150 mg of a white solid of 4-(2-{3,5-dichloro-6-[(3-ethylphenoxy)methyl]-2-oxopyridin-1(2H)-yl}ethyl)-N'-({[(2-ethylhexyl)oxy]carbonyl}oxy)benzenecarboxyimidamide.

### Production Example 60

To a solution of 300 mg of 4-(2-{3,5-dichloro-6-[(3-ethylphenoxy)methyl]-2-oxopyridin-1(2H)-yl}ethyl)benzoic acid in DMF 6.0 ml was added 163 mg of CDI, followed by stirring at room temperature overnight. Then, water and ethyl acetate were added thereto to carry out a liquid separation operation. The organic layer was washed with a saturated aqueous sodium chloride solution and then dried over anhydrous sodium sulfate, and the solvent was evaporated under reduced pressure to obtain 350 mg of 3,5-dichloro-6-[(3-ethylphenoxy)methyl]-1-{2-[4-(1H-imidazol-1-ylcarbonyl)phenyl]ethyl}pyridin-2(1H)-one.

### Production Example 61

To a mixed solution of 200 mg of 3,5-dichloro-6-[(3-ethylphenoxy)methyl]-1-{2-[4-(1H-imidazol-1-ylcarbonyl)phenyl]ethyl}pyridin-2(1H)-one in 4.0 ml of THF and 2.0 ml of water was added 30 mg of sodium borohydride under ice-cooling, followed by stirring at room temperature for 3 hours. Then, water and ethyl acetate were added thereto to carry out a liquid separation operation. The organic layer was washed with 1 M hydrochloric acid and a saturated aqueous sodium chloride solution in this order and then dried over anhydrous sodium sulfate, and the solvent was evaporated under reduced pressure to obtain 150 mg of 3,5-dichloro-6-[(3-ethylphenoxy)methyl]-1-{2-[4-(hydroxymethyl)phenyl]ethyl}pyridin-2(1H)-one.

### Production Example 62

To a solution of 160 mg of 4-(2-{3,5-dichloro-6-[(3-ethylphenoxy)methyl]-2-oxopyridin-1(2H)-yl}ethyl)benzoic acid in 3.2 ml of DMF were added 78 mg of WSC hydrochloride, 73 mg ofHOBt, and 23 mg of hydrazine hydrochloride, followed by stirring at room temperature overnight. Then, water and ethyl acetate were added thereto to carry out a liquid separation operation. The organic layer was washed with a saturated aqueous sodium chloride solution and then dried over anhydrous sodium sulfate, and the solvent was evaporated under reduced pressure to obtain 180 mg of 4-(2-{3,5-dichloro-6-[(3-ethylphenoxy)methyl]-2-oxopyridin-1(2H)-yl}ethyl)benzohydrazine.

### Production Example 63

To a suspension of 300 mg of 4-(2-{3,5-dichloro-6-[(E)-2-(3-methoxyphenyl)vinyl]-2-oxopyridin-1(2H)-yl}ethyl)benzoic acid in 3.0 ml of tert-butyl alcohol were added 175 µl of diphenylphosphoryl azide and 125 µl of triethylamine, followed by stirring at 100°C overnight. After leaving to be cooled to room temperature, water was added thereto, and the precipitated solid was collected by filtration to obtain 348 mg oftert-butyl [4-(2-{3,5-dichloro-6-[(E)-2-(3-methoxyphenyl)vinyl]-2-oxopyridin-1(2H)-yl}ethyl)phenyl]carbamate.

The Production Example Compounds 17 to 30, and 64 to 123 were prepared in the same manner as the methods of Production Examples 1 to 16 and 32 to 63 above using each of the corresponding starting materials. The structures, production processes, and physicochemical data of Production Example Compounds are shown in Tables 2 to 5 and Tables 12 to 25.

### Example 1

To a mixed solvent of 175 mg of methyl 4-(2-{3,5-dichloro-6-[(3-isopropylphenoxy)methyl]-2-oxopyridin-1(2H)-yl}ethyl)benzoate in 4.0 ml of THF and 1.0 ml of methanol was added 550 µl of a 1 M aqueous sodium hydroxide solution, followed by stirring at 70°C for 5 hours. The reaction liquid was acidified by the addition of 1 M hydrochloric acid under ice-cooling, and ethyl acetate and water were added thereto to carry out a liquid separation operation. The organic layer was washed with a saturated aqueous sodium chloride solution and then dried over anhydrous sodium sulfate, and the solvent was evaporated under reduced pressure. The residue was solidified by a mixed solvent of methanol and water to obtain 163 mg of a pale gray solid of 4-(2-{3,5-dichloro-6-[(3-isopropylphenoxy)methyl]-2-oxopyridin-1(2H)-yl}ethyl)benzoic acid.

### Example 2

To a solution of 260 mg of methyl 4-(2-{3,5-dibromo-6-[(3-methoxyphenoxy)methyl]-2-oxopyridin-1(2H)-yl}ethyl)benzoate in 4 ml of 1,4-dioxane was added 4 ml of 6 M hydrochloric acid, followed by stirring at 90°C for 6 hours. After leaving to be cooled to room temperature, the reaction liquid was then neutralized by the addition of a 1 M aqueous sodium hydroxide solution under ice-cooling, and ethyl acetate was added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography, and the obtained solid was then solidified by a mixed solvent of ethyl acetate-hexane to obtain 212 mg of 4-(2-{3,5-dibromo-6-[(3-methoxyphenoxy)methyl]-2-oxopyridin-1(2H)-yl}ethyl)benzoic acid.

### Example 3

To a solution of 280 mg of methyl 4-(2-{5-bromo-3-chloro-6-[(3-methoxyphenoxy)methyl]-2-oxopyridin-1(2H)-yl}ethyl)benzoate in 2.8 ml of methanol was added 0.56 ml of a 1 M aqueous sodium hydroxide solution at room temperature, followed by heating under reflux for 8 hours. After leaving to be cooled to room temperature, the reaction liquid was neutralized by the addition of 1 M hydrochloric acid, and the precipitated solid was collected by filtration. The obtained solid was purified by ODS column chromatography and washed with diethyl ether to obtain 60 mg of a pale yellow solid of 4-(2-{5-bromo-3-chloro-6-[(3-methoxyphenoxy)methyl]-2-oxopyridin-1(2H)-yl}ethyl)benzoic acid.

### Example 4

To a solution of 24 mg of methyl 4-(2-{3,5-dibromo-6-[(E)-2-(3-methoxyphenyl)vinyl]-2-oxopyridin-1(2H)-yl}ethyl)benzoate in 4 ml of 1,4-dioxane was added 4 ml of 6 M hydrochloric acid, followed by stirring at 90°C for 8 hours. After leaving to be cooled to room temperature, the reaction liquid was neutralized by the addition of a 1 M aqueous sodium hydroxide solution under ice-cooling. The solvent was evaporated under reduced pressure, and diethyl ether and a 1 M aqueous sodium hydroxide solution were added thereto to carry out a liquid separation operation. The aqueous layer was acidified with 1 M hydrochloric acid, and the precipitated solid was collected by filtration to obtain 12 mg of a yellow solid of 4-(2-{3,5-dibromo-6-[(E)-2-(3-methoxyphenyl)vinyl]-2-oxopyridin-1(2H)-yl}ethyl)benzoic acid.

### Example 5

To a solution of 230 mg of tert-butyl 4-(2-{3,5-dichloro-6-[(E)-2-(3-methoxyphenyl)vinyl]-2-oxopyridin-1(2H)-yl}ethyl)benzoate in 10 ml of chloroform was added 5 ml of trifluoroacetic acid under ice-cooling, followed by stirring at room temperature for 3 hours. The solvent was evaporated under reduced pressure, and the residue was made into powders using ethyl acetate-hexane to obtain 176 mg of a pale yellow solid of 4-(2-{3,5-dichloro-6-[(E)-2-(3-methoxyphenyl)vinyl]-2-oxopyridin-1(2H)-yl}ethyl)benzoic acid.

### Example 6

To a solution of 49 mg of methyl 4-{2-[6-(bromo methyl)-3,5-dichloro-2-oxopyridin-1(2H)-yl]ethyl}benzoate in 8 ml of acetone were added 73 mg of 3-methoxyphenol and 81 mg of potassium carbonate, followed by heating under reflux for 1 hour. After leaving to be cooled to room temperature, a saturated aqueous ammonium chloride solution and ethyl acetate were added thereto to carry out a liquid separation operation. The organic layer was washed with a 0.5 M aqueous sodium hydroxide solution and a saturated aqueous sodium chloride solution in this order and then dried over anhydrous sodium sulfate, and the solvent was evaporated under reduced pressure. The residue was purified by silica gel column chromatography to obtain 43 mg of a white solid of methyl 4-(2-{3,5-dichloro-6-[(3-methoxyphenoxy)methyl]-2-oxopyridin-1(2H)-yl}ethyl)benzoate.

### Example 7

To a solution of 835 mg of (3-methoxybenzyl)(triphenyl)phosphonium bromide in 8 ml of THF was added 202 mg of potassium tert-butoxide under ice-cooling, followed by stirring for 15 minutes. A solution of 238 mg oftert-butyl 4-[2-(3,5-dichloro-6-formyl-2-oxopyridin-1(2H)-yl)ethyl]benzoate in 8 ml of THF was added thereto at the same temperature, followed by stirring for 1 hour. A saturated aqueous ammonium chloride solution and ethyl acetate were added thereto under ice-cooling to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 283 mg of a pale yellow solid of tert-butyl 4-(2-{3,5-dichloro-6-[(E)-2-(3-methoxyphenyl)vinyl]-2-oxopyridin-1(2H)-yl}ethyl)benzoate.

### Example 27

To a mixed solvent of 220 mg of methyl 4-(2-{3,5-dichloro-2-oxo-6-[(E)-2-(3-propylphenyl)vinyl]pyridin-1(2H)-yl}ethyl)benzoate in 2.2 ml of acetone and 2.2 ml of water were added 24 mg of osmium oxide (VIII) and 164 mg of 4-methylmorpholine N-oxide, followed by stirring at 50°C overnight. Then, 2-propanol was added thereto, followed by stirring for 15 minutes and then leaving to be cooled to room temperature. Water and ethyl acetate were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 27 mg of methyl 4-(2-{3,5-dichloro-6-[1,2-dihydroxy-2-(3-propylphenyl)ethyl]-2-oxopyridin-1(2H)-yl}ethyl)benzoate.

### Example 28

To a mixed liquid of 220 mg of methyl 4-(2-{3,5-dichloro-2-oxo-6-[(E)-2-(3-propylphenyl)vinyl]pyridin-1(2H)-yl}ethyl)benzoate in 2.2 ml of acetone and 2.2 ml of water were added 24 mg of osmium oxide (VIII) and 164 mg of 4-methylmorpholine N-oxide at room temperature, followed by stirring at 50°C overnight. 2-Propanol was added thereto, followed by stirring for 15 minutes and then leaving to be cooled to room temperature. Water and ethyl acetate were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 62 mg of methyl 4-(2-{3,5-dichloro-2-oxo-6-[oxo(3-propyl phenyl)acetyl]pyridin-(2H)-yl}ethyl)benzoate.
To a solution of 25 mg of the obtained methyl 4-(2-{3,5-dichloro-2-oxo-6-[oxo(3-propylphenyl)acetyl]pyridin-1(2H)-yl}ethyl)benzoate in 2.0 ml of DCM were added 329 mg of bis(2-methoxyethyl)aminosulfur trifluoride and one droplet of ethanol in this order, followed by stirring at 60°C overnight. After leaving to be cooled to room temperature, a saturated aqueous sodium hydrogen carbonate solution and ethyl acetate were added thereto under ice-cooling to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 22 mg of colorless oily methyl 4-(2-{3,5-dichloro-6-[difluoro(3-propylphenyl)acetyl]-2-oxopyridin-1(2H)-yl}ethyl)benzoate.

### Example 29

To a mixed liquid of 220 mg of methyl 4-(2-{3,5-dichloro-2-oxo-6-[(E)-2-(3-propylphenyl)vinyl]pyridin-1(2H)-yl}ethyl)benzoate, 2.2 ml of acetone and 2.2 ml of water were added 24 mg of osmium oxide (VIII) and 164 mg of 4-methylmorpholine N-oxide, followed by stirring at 50°C overnight. 2-Propanol was added thereto, followed by stirring for 15 minutes and then cooling. Water and ethyl acetate were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 62 mg of methyl 4-(2-{3,5-dichloro-2-oxo-6-[oxo(3-propylphenyl)acetyl]pyridin-1(2H)-yl}ethyl)benzoate.
To a solution of 6.2 mg of the obtained methyl 4-(2-{3,5-dichloro-2-oxo-6-[oxo(3-propyl phenyl)acetyl]pyridin-1(2H)-yl}ethyl)benzoate in 1.0 ml of 1,4-dioxane was added 1.0 ml of 6 M hydrochloric acid, followed by heating under reflux overnight. After leaving to be cooled to room temperature, water and ethyl acetate were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography and then solidified by a mixed solvent of ethyl acetate/hexane to obtain 6.0 mg of 4-(2-{3-chloro-5-cyclopropyl-6-[(3-ethylphenoxy)methyl]-2-oxopyridin-1(2H)-yl}ethyl)benzoic acid.

### Example 30

To a solution of 100 mg of tert-butyl 4-{2-[6-(bromomethyl)-3,5-dichloro-2-oxopyridin-1(2H)-yl]ethyl}benzoate in 2.0 ml of THF was added 434 µl of a 0.1 M lithium copper (II) tetrachloride-THF solution under ice-cooling. Separately, to a suspension of 16 mg of magnesium in 2.0 ml of diethyl ether was slowly added 113 µl of 2-methoxyphenethyl bromide, followed by heating, to prepare a Grignard reagent. The Grignard reagent was added to the previous reaction system under ice-cooling, followed by stirring at the same temperature for 30 minutes. Then, a saturated aqueous ammonium chloride solution and ethyl acetate were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 57 mg of yellow oily tert-butyl 4-(2-{3,5-dichloro-6-[3-(2-methoxyphenyl)propyl]-2-oxypyridin-1(2H)-yl}ethyl)benzoate.

### Example 31

To a solution of 200 mg of tert-butyl 4-{2-[6-(bromomethyl)-3,5-dichloro-2-oxopyridin-1(2H)-yl]ethyl}benzoate in 2.2 ml of THF was added 867 µl of a 0.1 M lithium copper (II) tetrachloride -THF solution under ice-cooling. Separately, to a suspension of 158 mg of magnesium in 6.0 ml of diethyl ether was slowly added 1.13 ml of 3-methoxyphenethyl bromide, followed by heating, to prepare a Grignard reagent.
The Grignard reagent was added to the previous reaction system under ice-cooling, followed by stirring at the same temperature for 30 minutes. Then, a saturated aqueous ammonium chloride solution and ethyl acetate were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 89 mg of colorless oily tert-butyl 4-(2-{3,5-dichloro-6-[3-(3-methoxyphenyl)propyl]-2-oxypyridin-1(2H)-yl}ethyl)benzoate.
To a solution of 89 mg of the obtained tert-butyl 4-(2-{3,5-dichloro-6-[3-(3-methoxyphenyl)propyl]-2-oxypyridin-1(2H)-yl}ethyl)benzoate in 2.0 ml of chloroform was added 1.0 ml of trifluoroacetic acid, followed by stirring at room temperature for 3 hours. The solvent was evaporated under reduced pressure, and the residue was then purified by silica gel column chromatography and solidified by a mixed solvent of diisopropyl ether, ethyl acetate, and hexane to obtain 62 mg of 4-(2-{3,5-dichloro-6-[3-(3-methoxyphenyl)propyl]-2-oxypyridine-1(2H)-yl}ethyl)benzoic acid.

### Example 32

To a suspension of 160 mg of 4-(2-{3,5-dichloro-6-[(3-ethylphenoxy)methyl]-2-oxopyridin-1(2H)-yl}ethyl)benzonitrile in 3.0 ml of toluene were added 49 mg of sodium azide and 129 mg of triethylamine hydrochloride, followed by heating under reflux for 24 hours. After leaving to be cooled to room temperature, 97 mg of sodium azide and 258 mg of triethylamine hydrochloride were added thereto, followed by further heating under reflux for 24 hours. After leaving to be cooled to room temperature, water and ethyl acetate were added thereto to carry out a liquid separation operation. The organic layer was dried over anhydrous sodium sulfate, and the solvent was evaporated under reduced pressure. The residue was solidified by a mixed solvent of ethyl acetate and hexane to obtain 91 mg of 3,5-dichloro-6-[(3-ethylphenoxy)methyl]-1-{2-[4-(1H-tetrazol-5-yl)phenyl]ethyl}pyridin-2(1H)-one.

### Example 33

A mixture of 150 ml of 4-(2-{3,5-dichloro-6-[(3-ethylphenoxy)methyl]-2-oxopyridin-1(2H)-yl}ethyl)-N'-{[(2-ethylhexyl)oxy]carbonyl}oxybenzenecarboxyimidamide and 3.0 ml of xylene was stirred at 140°C for 5 hours. After leaving to be cooled to room temperature, the precipitated solid was collected by filtration and washed with diethyl ether to obtain 80 mg of a yellow solid of 3,5-dichloro-6-[(3-ethylphenoxy)methyl]-1-{2-[4-(5-oxo-4,5-dihyrdo-1,2,4-oxazol-3-yl)phenyl]ethyl}pyridin-2(1H)-one.

### Example 34

To a solution of 60 mg of 4-(2-{3,5-dichloro-6-[(3-ethylphenoxy)methyl]-2-oxopyridin-1(2H)-yl}ethyl)-N'-hydroxybenzene carboxyimidamide in 1.0 ml of 1,4-dioxane were added 51 mg of 1,1'-thiocarbonyldiimidazole and 44 mg of DBU in this order, followed by stirring at room temperature for 5 minutes. Then, a saturated aqueous sodium hydrogen carbonate solution and ethyl acetate were added thereto to carry out a liquid separation operation. The organic layer was washed with a saturated aqueous sodium chloride solution and then dried over anhydrous sodium sulfate, and the solvent was evaporated under reduced pressure. The residue was solidified by a mixed solvent of ethyl acetate and hexane to obtain 36 mg of 3,5-dichloro-6-[(3-ethylphenoxy)methyl]-1-{2-[4-(5-thioxo-4,5-dihydro-1,2,4-oxazol-3-yl)phenyl]ethyl}pyridin-2(1H)-one.

### Example 35

To a solution of 60 mg of 4-(2-{3,5-dichloro-6-[(3-ethylphenoxy)methyl]-2-oxopyridin-1(2H)-yl}ethyl)-N'-hydroxybenzenecarboxyimidamide in 1.0 ml of DCM were added 21 µl of pyridine and 11 µl of thionyl chloride at -20°C, followed by stirring at the same temperature for 5 minutes. Then, water and ethyl acetate were added thereto to carry out a liquid separation operation. The organic layer was washed with a saturated aqueous sodium chloride solution and then dried over anhydrous sodium sulfate, and the solvent was evaporated under reduced pressure. The residue was solidified by a mixed solvent of ethyl acetate and hexane to obtain 56 mg of 3,5-dichloro-6-[(3-ethylphenoxy)methyl]-1-{2-[4-(2-oxide-3H-1,2,3,5-oxathiazol-4-yl)phenyl]ethyl}pyridin-2(1H)-one.

### Example 36

To a solution of 103 mg of 3,5-dichloro-6-[(3-ethylphenoxy)methyl]-1-{2-[4-(1H-imidazol-1-ylcarbonyl)phenyl]ethyl}pyridin-2(1H)-one in 2.0 ml of 1,4-dioxane were added 40 mg of DBU and 44 mg of 3-(aminosulfonyl)propyl acetate, followed by stirring at room temperature overnight. 1 M hydrochloric acid and water were added thereto, and the precipitated solid was collected by filtration to obtain 100 mg of 3-({[4-(2-{3,5-dichloro-6-[(3-ethoxyphenoxy)methyl]-2-oxopyridin-1(2H)-yl}ethyl)benzoyl]amino}sulfonyl)propyl acetate.

### Example 37

To a solution of 111 mg of 3,5-dichloro-6-[(3-ethylphenoxy)methyl]-1-{2-[4-(1H-imidazol-1-ylcarbonyl)phenyl]ethyl}pyridin-2(1H)-one in 2.2 ml of 1,4-dioxane were added 51 mg of DBU and 31 mg of methanesulfonamide, followed by stirring at 90°C for 1 hour. 1 M hydrochloric acid and chloroform were added thereto under ice-cooling to carry out a liquid separation operation. The organic layer was washed with a saturated aqueous sodium chloride solution and then dried over anhydrous sodium sulfate, and the solvent was evaporated under reduced pressure. The residue was solidified by a mixed solvent of ethyl acetate and hexane to obtain 70 mg of 4-(2-{3,5-dichloro-6-[(3-ethylphenoxy)methyl]-2-oxopyridin-1(2H)-yl}ethyl)-N-(methylsulfonyl)benzamide.

### Example 38

A solution of 150 mg of 3,5-dichloro-6-[(3-ethylphenoxy)methyl]-1-{2-[4-(1H-imidazol-1-ylcarbonyl)phenyl]ethyl}pyridin-2(1H)-one in 2.0 ml of THF was added to 5.0 ml of a 28% aqueous ammonia solution, followed by stirring at room temperature for 2 hours. The precipitated solid was collected by filtration to obtain 95 mg of 4-(2-{3,5-dichloro-6-[(3-ethylphenoxy)methyl]-2-oxopyridin-1(2H)-yl}ethyl)benzamide.

### Example 39

To a solution of 220 mg of 4-(2-{3,5-dichloro-6-[(E)-2-(3-methoxyphenyl)vinyl]-2-oxopyridin-1(2H)-yl}ethyl)benzoic acid in 4.4 ml of 1,4-dioxane was added 99 mg of CDI, followed by stirring at room temperature overnight. Then, water and ethyl acetate were added thereto to carry out a liquid separation operation. The organic layer was washed with a saturated aqueous sodium chloride solution and then dried over anhydrous sodium sulfate, and the solvent was evaporated under reduced pressure to obtain 3,5-dichloro-6-[(E)-2-(3-methoxyphenyl)vinyl]-1-{2-[4-(1H-imidazol-1-ylcarbonyl)phenyl]ethyl}pyridin-2(1H)-one.
To a solution of 3,5-dichloro-6-[(E)-2-(3-methoxyphenyl)vinyl]-1-{2-[4-(1H-imidazol-1-ylcarbonyl)phenyl]ethyl}pyridin-2(1H)-one in 4.4 ml of 1,4-dioxane were added 113 mg of DBU and 135 mg of 3-(aminosulfonyl)propyl acetate, followed by stirring at 50°C overnight. After leaving to be cooled to room temperature, 1 M hydrochloric acid and chloroform were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 300 mg of 3-({[4-(2-{3,5-dichloro-6-[(E)-2-(3-methoxyphenyl)vinyl]-2-oxopyridin-1(2H)-yl}ethyl)benzoyl]amino}sulfonyl)propyl acetate.

### Example 40

To a mixed liquid of 100 mg of 3-({[4-(2-{3,5-dichloro-6-[(3-ethoxyphenoxy)methyl]-2-oxopyridin-1(2H)-yl}ethyl)benzoyl]amino}sulfonyl)propyl acetate, 1.0 ml of THF, and 1.0 ml of methanol was added 0.33 ml of a 1 M aqueous sodium hydroxide solution, followed by stirring at room temperature overnight. Then, under ice-cooling, the reaction liquid was neutralized by the addition of 1 M hydrochloric acid, and the precipitated solid was collected by filtration to obtain 41 mg of 4-(2-{3,5-dichloro-6-[(3-ethylphenoxy)methyl]-2-oxopyridin-1(2H)-yl}ethyl)-N-[(3-hydroxy propyl)sulfonyl]benzamide.

### Example 41

To a solution of 4.35 g of 2-hydroxyethanesulfonamide and 2.6 g of imidazole in 50 ml of DMF was added 5.76 g of tert-butyl(chloro)dimethylsilane under ice-cooling, followed by stirring at room temperature for 4 hours. Then, a saturated aqueous ammonium chloride solution and ethyl acetate were added thereto to carry out a liquid separation operation. The organic layer was washed with a saturated aqueous sodium chloride solution and then dried over anhydrous sodium sulfate, and the solvent was evaporated under reduced pressure. The residue was washed with hexane to obtain 7.27 g of a white solid of 2-{[tert-butyl(dimethyl)silyl]oxy}ethanesulfonamide.
To a solution of 3,5-dichloro-6-[(3-ethoxyphenoxy)methyl]-1-{2-[4-(1H-imidazol-1-ylcarbonyl)phenyl]ethyl}pyridin-2(1H)-one in 2.0 ml of 1,4-dioxane were added 6 mg of DBU and 72 mg of 2-{[tert-butyl(dimethyl)silyl]oxy}ethanesulfonamide as synthesized above, followed by stirring at 60°C overnight. After leaving to be cooled to room temperature, water and chloroform were added thereto to carry out a liquid separation operation. The organic layer was washed with a saturated aqueous sodium chloride solution and then dried over anhydrous sodium sulfate, and the solvent was evaporated under reduced pressure. To a solution of the obtained residue in 2.0 ml of 1,4-dioxane was added 0.5 ml of concentrated hydrochloric acid under ice-cooling, followed by stirring at room temperature for 1 hour. Then, water was added thereto, and the precipitated solid was collected by filtration to obtain 33 mg of 4-(2-{3,5-dichloro-6-[(3-ethylphenoxy)methyl]-2-oxopyridin-1 (2H)-yl} ethyl)-N-[(2-hydroxyethyl)sulfonyl]benzamide.

### Example 42

To a solution of 200 mg of 4-(2-{3,5-dichloro-6-[(3-ethylphenoxy)methyl]-2-oxopyridin-1(2H)-yl}ethyl)benzoic acid in 4.0 ml of DMF were added 97 mg of WSC hydrochloride, 90 mg of HOBt, 75 mg of glycine ethyl ester hydrochloride, and 81 µl of triethylamine, followed by stirring at room temperature overnight. Then, water was added thereto, and the precipitated solid was collected by filtration to obtain 90 mg of ethyl N-[4-(2-{3,5-dichloro-6-[(3-ethylphenoxy)methyl]-2-oxopyridin-1(2H)-yl}ethyl)benzoyl]glycinate.

### Example 43

To a mixed liquid of 90 mg of ethyl N-[4-(2-{3,5-dichloro-6-[(3-ethylphenoxy)methyl]-2-oxopyridin-1(2H)-yl}ethyl)benzoyl]glycinate, 1.8 ml of THF, and 0.9 ml of methanol was added 0.34 ml of a 1 M aqueous sodium hydroxide solution, followed by stirring at room temperature overnight. Then, the reaction liquid was neutralized by the addition of 1 M hydrochloric acid under ice-cooling, and the precipitated solid was then collected by filtration to obtain 27 mg of N-[4-(2-{3,5-dichloro-6-[(3-ethylphenoxy)methyl]-2-oxopyridin-1(2H)-yl}ethyl)benzoyl]glycine.

### Example 44

To a solution of 30 mg of 4-(2-{3,5-dichloro-6-[(3-ethylphenoxy)methyl]-oxopyridin-1(2H)-yl}ethyl)benzaldehyde in 0.6 ml of DMF were added 30 mg of potassium carbonate and 54 mg of tert-butyl diethylphosphoacetate, followed by stirring at 80°C overnight. After leaving to be cooled to room temperature, water was added thereto, and the precipitated solid was collected by filtration.
To a solution of 10 mg of the obtained solid in 0.5 ml of DCM was added 0.2 ml of trifluoroacetic acid under ice-cooling, followed by stirring at room temperature for 3 hours. The solvent was evaporated under reduced pressure, and the obtained residue was dissolved in a 1 M aqueous sodium hydroxide solution and water. The solution was neutralized with 1 M hydrochloric acid under ice-cooling, and the precipitated solid was then collected by filtration to obtain 1.0 mg of (2E)-3-[4-(2-{3,5-dichloro-6-[(3-ethylphenoxy)methyl]-2-oxopyridin-1(2H)-yl}ethyl)phenyl]acrylic acid.

### Example 45

To a solution of 180 mg of 4-(2-{3,5-dichloro-6-[(3-ethylphenoxy)methyl]-2-oxopyridin-1(2H)-yl}ethyl)benzohydrazine in 4.0 ml of ethanol were added 30 mg of potassium hydroxide and 70.5 µl of carbon disulfide, followed by stirring at room temperature for 5 hours and then stirring at 60°C overnight. After leaving to be cooled to room temperature, water was added thereto, and the precipitated solid was collected by filtration to obtain 97 mg of 3,5-dichloro-6-[(3-ethylphenoxy)methyl]-1-{2-[4-(5-thioxo-4,5-dihydro-1,3,4-oxazol-2-yl)phenyl]ethyl}pyridin-2(1H)-one,

### Example 46

To a suspension of 100 mg of 1-[2-(4-aminophenyl)ethyl]-3,5-dichloro-6-[(E)-2-(3-methoxyphenyl)vinyl]pyridin-2(1H)-one hydrochloride in 2.0 ml of toluene were added 45 µl of DIPEA and 45 mg of ethyl(methylsulfonyl)carbamate, followed by stirring at 100°C overnight. After leaving to be cooled to room temperature, water and ethyl acetate were added thereto to carry out a liquid separation operation. The organic layer was washed with a saturated aqueous sodium chloride solution and then dried over anhydrous sodium sulfate, and the solvent was evaporated under reduced pressure. The residue was solidified by a mixed solvent of ethyl acetate and hexane to obtain 87 mg of N-{[4-(2-{3,5-dichloro-6-[(E)-2-(3-methoxyphenyl)vinyl]-2-oxopyridin-1(2H)-yl}ethyl)phenyl]carbamoyl}methanesulfonamide.

### Example 47

To a solution of 99 mg of 2-isopropylphenol in 1.0 ml of DMSO was added 29 mg of 55% sodium hydride (oily) under ice-cooling, followed by stirring at room temperature for 10 minutes, and then a solution of 100 mg of tert-butyl 4-{2-[6-(bromomethyl)-3,5-dichloro-2-oxopyridin-1(2H)-yl]ethyl}benzoate in 1.0 ml of DMF was added thereto, followed by further stirring at the same temperature overnight. A saturated aqueous ammonium chloride solution and ethyl acetate were added thereto under ice-cooling to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 98 mg of a yellow solid of tert-butyl 4-(2-{3,5-dichloro-6-[(2-isopropoxyphenoxy)methyl]-2-oxopyridin-1(2H)-yl}ethyl)benzoate.

### Example 48

To a solution of 556 mg of 3-(trifluoromethoxy)thiophenol in 15 ml of DMF was added 115 mg of 55% sodium hydride (oily) under ice-cooling, followed by stirring at the same temperature for 30 minutes, and then 600 mg of methyl 4-{2-[6-(bromomethyl)-3,5-dichloro-2-oxopyridin-1(2H)-yl]ethyl}benzoate was added thereto, followed by stirring at room temperature for 2 hours. Then, a saturated aqueous ammonium chloride solution and ethyl acetate were added thereto under ice-cooling to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 578 mg of a white solid of methyl 4-{2-[3,5-dichloro-2-oxo-6-({[3-(trifluoromethoxy)phenyl]sulfanyl}methyl)pyridin-1(2H)-yl]ethyl}benzoate.

### Example 49

To a solution of 47 mg of methyl 4-{2-[3,5-dichloro-6-(1-hydroxyethyl)-2-oxopyridin-1(2H)-yl]ethyl}benzoate in 1.0 ml of THF were added 23 mg of 3-ethylphenol, 50 mg of triphenylphosphine, and 87 µl of a 2.2 M diethyl azodicarboxylate (DEAD)/toluene solution in this order, followed by stirring at 50°C for 3 hours. After leaving to be cooled to room temperature, water and ethyl acetate were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 31 mg of colorless oily 4-(2-{3,5-dichloro-6-[1-(3-ethylphenoxy)ethyl]-2-oxopyridin-1(2H)-yl}ethyl)benzoic acid.

### Example 50

To a solution of 100 mg of tert-butyl 4-(2-{3,5-dichloro-6-[(3-hydroxyphenoxy)methyl]-2-oxopyridin-1(2H)-yl}ethyl)benzoate in 2.0 ml of DCM were added 37 mg of 2-ethoxyethanol, 107 mg of triphenylphosphine, and 0.18 ml of a 2.2 M DEAD/toluene solution in this order, followed by stirring at room temperature overnight. The solvent was evaporated under reduced pressure, and the residue was purified by silica gel column chromatography to obtain 50 mg oftert-butyl 4-{2-[3,5-dichloro-6-{[3-(2-ethoxyethoxy)phenoxy]methyl}-2-oxopyridin-1(2H)-yl]ethyl}benzoate.

### Example 51

To a solution of 100 mg oftert-butyl 4-(2-{3,5-dichloro-6-[(3-hydroxyphenoxy)methyl]-2-oxopyridin-1(2H)-yl}ethyl)benzoate in 2.0 ml of DCM were added 50 µl of 2-(tetrahydro-2H-pyran-2-yloxy)ethanol, 100 mg of triphenylphosphine, and 0.18 ml of a 2.2 M DEAD/toluene solution in this order, followed by stirring at room temperature overnight. The solvent was evaporated under reduced pressure, and the residue was purified by silica gel column chromatography to obtain 120 mg of tert-butyl 4-{2-[3,5-dichloro-2-oxo-6-({3-[2-(tetrahydro-2H-pyran-2-yloxy)ethoxy]phenoxyl}methyl)pyridin-1(2H)-yl]ethyl}benzoate.
To a solution of 110 mg of tert-butyl 4-{2-[3,5-dichloro-2-oxo-6-({3-[2-(tetrahydro-2H-pyran-2-yloxy)ethoxy]phenoxy}methyl)pyridin-1(2H)-yl]ethyl}benzoate in 2.2 ml of THF was added 0.2 ml of 6 M hydrochloric acid, followed by heating under reflux overnight. After leaving to be cooled to room temperature, water and chloroform were added thereto to carry out a liquid separation operation. The organic layer was washed with a saturated aqueous sodium chloride solution and then dried over anhydrous sodium sulfate, and the solvent was evaporated under reduced pressure. The residue was dissolved in a 1 M aqueous sodium hydroxide solution, and the aqueous solution was neutralized by the addition of a 1 M aqueous hydrochloric acid solution. In addition, water was added thereto, and the precipitated solid was collected by filtration to obtain 9.0 mg of 4-{2-[3,5-dichloro-6-{[3-(2-hydroxyethoxy)phenoxy]methyl}-2-oxopyridin-1(2H)-yl]ethyl}benzoic acid.

### Example 52

To a solution of 200 mg of methyl 4-(2-{3,5-dibromo-6-[(3-ethylphenoxy)methyl]-2-oxopyridin-1(2H)-yl}ethyl)benzoate in 4.0 ml of DMF were added 55 mg of methylboric acid, 100 mg of sodium carbonate, and 30 mg of tetrakis(triphenylphosphine)palladium (0) at room temperature, followed by stirring at 120°C for 3 days. After leaving to be cooled to room temperature, the insoluble materials were removed by filtration through Celite, and the filtrate was concentrated under reduced pressure. The residue was purified by silica gel column chromatography to obtain 50 mg of methyl 4-(2-{5-bromo-6-[(3-ethylphenoxy)methyl]-3-methyl-2-oxopyridin-1(2H)-yl}ethyl)benzoate.

### Example 53

To a solution of 100 mg of methyl 4-(2-{3-bromo-5-chloro-6-[(3-ethylphenoxy)methyl]-2-oxopyridin-1(2H)-yl}ethyl)benzoate in 2.0 ml of 1,4-dioxane were added 13 mg of methylboric acid, 77 mg of cesium carbonate, and 11 mg of tetrakis(triphenyl phosphine)palladium (0) at room temperature, followed by heating under reflux for 3 days. After leaving to be cooled to room temperature, the insoluble materials were removed by filtration through Celite, and the filtrate was concentrated under reduced pressure. The residue was purified by silica gel column chromatography to obtain 17 mg of methyl 4-(2-{5-chloro-6-[(3-ethylphenoxy)methyl]-3-methyl-2-oxopyridin-1(2H)-yl}ethyl)benzoate.

### Example 54

To a solution of 130 mg of methyl 4-{2-[3-bromo-5-chloro-2-oxo-6-{[3-(trifluoromethoxy)phenoxy]methyl}pyridin-1(2H)-yl]ethyl}benzoate in 3.0 ml of 1,4-dioxane were added 40 mg of cyclopropylboric acid, 110 mg of tripotassium phosphate, and 50 mg of tetrakis(triphenylphosphine)palladium (0), followed by stirring at 90°C for 3 days. After leaving to be cooled to room temperature, the insoluble materials were removed by filtration through Celite, and the filtrate was concentrated under reduced pressure. The residue was purified by silica gel column chromatography to obtain 75 mg of methyl 4-{2-[5-chloro-3-cyclopropyl-2-oxo-6-{[3-(trifluoromethoxy)phenoxy]methyl}pyridin-1(2H)-yl]ethyl}benzoate.

### Example 55

To a solution of 109 mg of methyl 4-{2-[5-bromo-3-chloro-2-oxo-6-{[3-(trifluoromethoxy)phenoxy]methyl}pyridin-1(2H)-yl]ethyl}benzoate in 1.0 ml ofDMF were added 123 mg oftri-n-butylvinyltin, 59 mg of cesium fluoride, 4 mg of copper (I) iodide, and 11 mg of tetrakis(triphenylphosphine)palladium (0) in this order, followed by stirring at 90°C overnight. After leaving to be cooled to room temperature, a saturated aqueous ammonium chloride solution and ethyl acetate were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 12 mg of colorless oily methyl 4-{2-3-chloro-2-oxo-6-{[3-(trifluoromethoxy)phenoxy]methyl}-5-vinylpyridin-1(2H)-yl]ethyl}benzoate.

### Example 56

To a solution of 52 mg of methyl 4-{2-[5-bromo-3-chloro-2-oxo-6-{[3-(trifluoromethoxy)phenoxy]methyl}pyridin-1(2H)-yl]ethyl}benzoate in 2.0 ml of N-methyl-2-pyrrolidone were added 47 mg of sodium methanesulfinate and 88 mg of copper (I) iodide, followed by heating under reflux overnight. After leaving to be cooled to room temperature, water and ethyl acetate were added thereto to carry out a liquid separation operation. The organic layer was washed with a saturated aqueous sodium chloride solution and then dried over anhydrous sodium sulfate, and the solvent was concentrated under reduced pressure. The residue was purified by silica gel column chromatography to obtain 21 mg of a white solid of methyl 4-{2-[3-chloro-5-(methylsulfonyl)-2-oxo-6-{[3-(trifluoromethoxy)phenoxy]methyl}-pyridin-1(2H)-yl]ethyl}benzoate.

### Example 57

To a mixture of 180 mg of methyl 4-(2-{5-bromo-3-chloro-6-[(3-ethylphenoxy)methyl]-2-oxopyridin-1(2H)-yl}ethyl)benzoate, 1.8 ml of toluene and 90 µl of water were added 61 mg of cyclopropylboric acid, 303 mg of tripotassium phosphate, 20 mg of tricyclohexylphosphine, and 8 mg of palladium acetate, followed by heating under reflux overnight. After leaving to be cooled to room temperature, water and ethyl acetate were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 68 mg of a white solid of methyl 4-(2-{3-chloro-5-cyclopropyl-6-[(3-ethylphenoxy)methyl]-2-oxopyridin-1(2H)-yl}ethyl)benzoate.

### Example 58

To a mixture of 405 mg of methyl 4-(2-{3,5-dibromo-6-[(3-ethylphenoxy)methyl]-2-oxopyridin-1(2H)-yl}ethyl)benzoate in 3.4 ml of toluene and 170 µl of water were added 176 mg of methylboric acid, 782 mg of tripotassium phosphate, 62 mg of tricyclohexylphosphine, and 25 mg of palladium acetate, followed by heating under reflux overnight. After leaving to be cooled to room temperature, water and ethyl acetate were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 250 mg of a white solid of compound.
To a mixed solvent of 184 mg of a white solid of the compound in 2.0 ml of THF and 2.0 ml of methanol was added 2.0 ml of a 1 M aqueous sodium hydroxide solution, followed by heating under reflux for 30 minutes. The reaction liquid was acidified by the addition of a 1 M aqueous hydrochloric acid solution under ice-cooling, and ethyl acetate was added thereto to carry out a liquid separation operation. The organic layer was washed with a saturated aqueous sodium chloride solution and then dried over anhydrous sodium sulfate, and the solvent was evaporated under reduced pressure. The residue was solidified by a mixed solvent of ethyl acetate and n-hexane to obtain 159 mg of a white solid of 4-(2-{6-[(3-ethylphenoxy)methyl]-3,5-dimethyl-2-oxopyridin-1(2H)-yl}ethyl)benzoic acid.

### Example 59

To a solution of 95 mg of methyl 4-{2-[3,5-dichloro-6-{[2-(2-hydroxyethyl)phenoxy]methyl}-2-oxopyridin-1(2H)-yl]ethyl}benzoate in 1.5 ml of DCM were added 0.4 ml of a 12 M aqueous sodium hydroxide solution, 0.38 ml of dimethyl sulfate, and 5.0 mg of benzyltriethylammonium chloride, followed by stirring at room temperature overnight. After leaving to be cooled to room temperature, ethyl acetate and a saturated aqueous ammonium chloride solution were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 87 mg of colorless oily methyl 4-{2-[3,5-dichloro-6-{[2-(2-methoxyethyl)phenoxy]methyl}-2-oxopyridin-1(2H)-yl]ethyl}benzoate.

### Example 60

To a suspension of 1.36 g of 1,3-dihydro-1H-inden-1-yl(triphenyl)phosphonium bromide and 157 mg of 1,4,7,10,13,16-hexaoxacyclooctadecane in 3.7 ml of DCM was added 410 mg of potassium carbonate, followed by stirring at room temperature for 20 minutes. Then, 210 mg of methyl 4-[2-(3,5-dichloro-6-formyl-2-oxopyridin-1(2H)-yl)ethyl]benzoate was added thereto, followed by further stirring for 2 hours. A saturated aqueous ammonium chloride solution and ethyl acetate were added thereto under ice-cooling to carry out a liquid separation operation, and the organic layer was washed with a saturated aqueous sodium chloride solution and then dried over anhydrous sodium sulfate, and the solvent was evaporated under reduced pressure. The residue was purified by silica gel column chromatography to obtain 222 mg of a yellow solid of methyl 4-{2-[3,5-dichloro-6-(2,3-dihydro-1H-inden-1-yridenemethyl)-2-oxopyridin-1(2H)-yl]ethyl}benzoate.

### Example 61

To a suspension of 587 mg of (3-isopropylbenzyl)(triphenyl)phosphonium bromide in 3.5 ml of THF was added 138 mg of potassium tert-butoxide under ice-cooling, followed by stirring for 20 minutes. Then, 150 mg of methyl 4-[2-(5-bromo-6-formyl-2-oxopyridin-1(2H)-yl)ethyl]benzoate was added thereto at the same temperature, followed by further stirring for 2 hours. A saturated aqueous ammonium chloride solution and ethyl acetate were added thereto under ice-cooling to carry out a liquid separation operation, and the organic layer was washed with a saturated aqueous sodium chloride solution and then dried over anhydrous sodium sulfate, and the solvent was concentrated under reduced pressure. The residue was purified by silica gel column chromatography to obtain 124 mg of a yellow solid of methyl 4-(2-{5-bromo-6-[(E)-2-(3-methoxyphenyl)vinyl]-2-oxopyridin-1(2H)-yl}ethyl)benzoate.
To a mixed solvent of 124 mg of methyl 4-(2-{5-bromo-6-[(E)-2-(3-methoxyphenyl)vinyl]-2-oxopyridin-1(2H)-yl}ethyl)benzoate in 1.2 ml of THF and 1.2 ml of methanol was added 1.2 ml of a 1 M aqueous sodium hydroxide solution, followed by heating under reflux for 30 minutes. Then, the reaction liquid was acidified by the addition of 1 M hydrochloric acid under ice-cooling, and the precipitated solid was collected by filtration and solidified by methanol-water to obtain 85 mg of 4-(2-{5-bromo-6-[(E)-2-(3-isopropylphenyl)vinyl]-2-oxopyridin-1(2H)-yl}ethyl)benzoic acid.

### Example 62

To a solution of 60 mg of methyl 4-{3-[3,5-dichloro-6-(hydroxymethyl)-2-oxopyridin-1(2H)-yl]propyl}benzoate in 1.2 ml of DCM was added 14 mg of manganese dioxide, followed by stirring at room temperature overnight. The insoluble materials were removed by filtration through Celite, and the filtrate was concentrated under reduced pressure. The residue was solidified by hexane to obtain 55 mg of methyl 4-[3-(3,5-dichloro-6-formyl-2-oxopyridin-1(2H)-yl)propyl]benzoate.
To a suspension of 237 mg of (3-methoxybenzyl)(triphenyl)phosphonium bromide in 1.0 ml of THF was added 57 mg of potassium tert-butoxide under ice-cooling, followed by stirring for 30 minutes, and 27 mg of the obtained methyl 4-[3-(3,5-dichloro-6-formyl-2-oxopyridin-1(2H)-yl)propyl]benzoate above was added thereto, followed by stirring for 1 hour. A saturated aqueous ammonium chloride solution and ethyl acetate were added thereto under ice-cooling to carry out a liquid separation operation, and the organic layer was washed with a saturated aqueous sodium chloride solution and then dried over anhydrous sodium sulfate, and the solvent was evaporated under reduced pressure. The residue was purified by silica gel column chromatography to obtain 34 mg of methyl 4-(3-{3,5-dichloro-6-[(E)-2-(3-methoxyphenyl)vinyl]-2-oxopyridin-1(2H)-yl}propyl)benzoate.

### Example 63

To a solution of 150 mg of tert-butyl 4-(2-{6-[(E)-2-(3-aminophenyl)vinyl]-3,5-dichloro-2-oxopyridin-1(2H)-yl}ethyl)benzoate in 3.0 ml of DMF were added 100 mg of potassium carbonate and 42 µl of methyl iodide, followed by stirring at room temperature overnight. A saturated aqueous ammonium chloride solution and ethyl acetate were added thereto under ice-cooling to carry out a liquid separation operation, and the organic layer was washed with a saturated aqueous sodium chloride solution and dried over anhydrous sodium sulfate, and the solvent was evaporated under reduced pressure. The residue was purified by silica gel column chromatography to obtain 20 mg of tert-butyl 4-{2-[3,5-dichloro-6-{(E)-2-[3-(dimethylamino)phenyl]vinyl}-2-oxopyridin-1(2H)-yl]ethyl}benzoate.

### Example 64

To a solution of 1.3 g of methyl 4-(2-{3,5-dichloro-6-[(E)-2-(2-ethoxyphenyl)vinyl]-2-oxopyridin-1(2H)-yl}ethyl)benzoate in 50 ml of ethyl acetate was added 65 mg of 10% palladium-carbon (50% hydrate product), followed by stirring at room temperature for 5 hours under a hydrogen atmosphere. The insoluble materials were removed by filtration through Celite, and the filtrate was concentrated under reduced pressure. The residue was purified by silica gel column chromatography to obtain 1.2 g of white amorphous methyl 4-(2-{3,5-dichloro-6-[2-(2-ethoxyphenyl)ethyl]-2-oxopyridin-1(2H)-yl}ethyl)benzoate.

### Example 65

To a solution of 150 mg of methyl 4-{2-[3,5-dichloro-6-{(E)-2-[2-fluoro-5-(trifluoromethyl)phenyl]vinyl}-2-oxopyridin-1(2H)-yl]ethyl}benzoate in 1.5 ml of ethyl acetate was added 150 mg of 10% palladium-carbon, followed by stirring at room temperature for 2 days under a hydrogen atmosphere. The insoluble materials were removed by filtration through Celite, and the solvent was evaporated under reduced pressure. The residue was purified by silica gel column chromatography to obtain a colorless solid.
To a solution of the obtained colorless solid in 1.5 ml of THF was added 100 µl of a 1 M aqueous sodium hydroxide solution, followed by heating under reflux overnight. After leaving to be cooled to room temperature, the reaction liquid was concentrated under reduced pressure and neutralized by the addition of 1 M hydrochloric acid under ice-cooling, water was then added thereto, and the precipitated solid was collected by filtration to obtain 23 mg of 4-{2-[3,5-dichloro-6-{2-[2-fluoro-5-(trifluoromethyl)phenyl]ethyl}-2-oxopyridin-1(2H)-yl]ethyl}benzoic acid.

### Example 66

To a solution of 320 mg of tert-butyl 4-(2-{6-[(3-acetoxyphenoxy)methyl]-3,5-dichloro-2-oxopyridin-1(2H)-yl}ethyl)benzoate in 6.4 ml of methanol was added 100 mg of potassium carbonate, followed by stirring at room temperature overnight. To the reaction liquid were added ethyl acetate and a saturated aqueous ammonium chloride solution to carry out a liquid separation operation, and the organic layer was washed with a saturated aqueous sodium chloride solution and dried over anhydrous sodium sulfate, and the solvent was evaporated under reduced pressure to obtain 250 mg of a pale yellow solid of tert-butyl 4-(2-{3,5-dichloro-6-[(3-hydroxyphenoxy)methyl]-2-oxopyridin-1(2H)-yl}ethyl)benzoate.

### Example 67

To a mixed solution of 600 mg of tert-butyl 4-(2-{3,5-dichloro-6-[(E)-2-(3-nitrophenyl)vinyl]-2-oxopyridin-1(2H)-yl}ethyl)benzoate in 12 ml of ethanol and 6.0 ml of water were added 325 mg of iron and 62 mg of ammonium chloride, followed by stirring at room temperature overnight. The insoluble materials were removed by filtration through Celite, and the filtrate was then concentrated under reduced pressure. The residue was purified by silica gel column chromatography to obtain 500 mg of tert-butyl 4-(2-{6-[(E)-2-(3-aminophenyl)vinyl]-3,5-dichloro-2-oxopyridin-1(2H)-yl}ethyl)benzoate.

### Example 68

To a solution of 120 mg of methyl 4-{2-[6-(bromomethyl)-3,5-dichloro-2-oxopyridin-1(2H)-yl]ethyl}benzoate in 3.6 ml of acetone were added 119 mg of potassium carbonate and 143 mg of 2-isobutoxyphenol, followed by heating under reflux overnight. After leaving to be cooled to room temperature, ethyl acetate and a saturated aqueous ammonium chloride solution were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 144 mg of a colorless oily compound.
To a solution of 144 mg of the obtained colorless oily compound in 3.0 ml of 1,4-dioxane was added 3.0 ml of 6 M hydrochloric acid, followed by heating under reflux overnight. After leaving to be cooled to room temperature, water and ethyl acetate were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography and solidified by a mixed solvent of ethyl acetate and hexane to obtain 29 mg of 4-(2-{3,5-dichloro-6-[(2-isobutoxyphenoxy)methyl]-2-oxopyridin-1(2H)-yl}ethyl)benzoic acid.

### Example 69

To a solution of 100 mg of methyl 4-{2-[5-bromo-6-(bromomethyl)-2-oxopyridin-1(2H)-yl]ethyl}benzoate in 2.0 ml of DMF were added 48 mg of potassium carbonate and 48 mg of 2-ethoxyphenol, followed by stirring at 60°C overnight. After leaving to be cooled to room temperature, ethyl acetate and a saturated aqueous ammonium chloride solution were added thereto to carry out a liquid separation operation. The organic layer was washed with a saturated aqueous sodium chloride solution and then dried over anhydrous sodium sulfate, and the solvent was evaporated under reduced pressure. The residue was purified by silica gel column chromatography to obtain 104 mg of a white solid of the compound.
To a mixed solvent of 100 mg of the obtained white solid of the compound in 1.0 ml of THF and 1.0 ml of methanol was added 1.0 ml of a 1 M aqueous sodium hydroxide solution, followed by heating under reflux for 30 minutes. The reaction liquid was acidified by the addition of 1 M hydrochloric acid under ice-cooling, and the precipitated solid was collected by filtration and the obtained solid was washed with methanol-water to obtain 61 mg of 4-(2-{5-bromo-6-[(2-ethoxyphenoxy)methyl]-2-oxopyridin-1(2H)-yl}ethyl)benzoic acid.

### Example 70

To a solution of 220 mg of tert-butyl 4-{(E)-2-[6-(bromomethyl)-3,5-dichloro-2-oxopyridin-1(2H)-yl]vinyl}benzoate in 4.4 ml of acetone were added 90 mg of potassium carbonate and 82 mg of 3-ethylphenol at room temperature, followed by heating under reflux overnight. After leaving to be cooled to room temperature, ethyl acetate and a saturated aqueous ammonium chloride solution were added thereto to carry out a liquid separation operation. The organic layer was washed with a saturated aqueous sodium chloride solution and then dried over anhydrous sodium sulfate, and the solvent was evaporated under reduced pressure. To a solution of 40 mg of the obtained residue in 2.0 ml of DCM was added 500 µl of trifluoroacetic acid under ice-cooling, followed by stirring at the same temperature for 2 hours. The solvent was evaporated under reduced pressure and the residue was then solidified with a mixed solvent of ethyl acetate and hexane to obtain 32 mg of 4-[(E)-2-{3,5-dichloro-6-[(3-ethylphenoxy)methyl]-2-oxopyridin-1(2H)-yl}vinyl]benzoic acid.

In the same manner as in the methods of Examples 1 to 7 and Examples 27 to 70, and the Production Examples above, the compounds of Examples 8 to 26 and Examples 71 to 358 as shown in Tables below were prepared using each of the corresponding starting materials. The structures of the compounds of Examples are respectively shown in Tables 6 to 9 and Tables 26 to 59, and the physicochemical data and the production methods are shown in Tables 10 to 11 and Tables 60 to 71.

In addition, the following abbreviations are used in Examples, Production Examples, and Tables below.
PEx: Production Example, Ex: Example, Data: Physicochemical data (NMR: δ (ppm) in 1H NMR in DMSO-d₆, FAB+:FAB-MS (representing (M+H)⁺ unless otherwise specifically explained), FAB-: FAB-MS (representing (M-H)⁻ unless otherwise specifically explained), ESI+: ESI-MS (representing (M+H)⁺ unless otherwise specifically explained), ESI-:ESI-MS (representing (M-H)⁻ unless otherwise specifically explained), EI: EI-MS (representing (M)⁺ unless otherwise specifically explained), CI+: CI-MS (representing (M+H)⁺ unless otherwise specifically explained), APCI+: APCI-MS (representing (M+H)⁺ unless otherwise specifically explained), APCI/ESI+: meaning the simultaneous measurement of APCI and ESI, and representing (M+H)⁺ unless otherwise specifically explained), Structure: Structural Formula, Syn: Production Method (the number shows that it was prepared using a corresponding starting material in the same manner as the compound having the number as the Example No. When P is attached before the number, it is shown that it was prepared using a corresponding starting material in the same manner as the compound having the number as the Production Example No.). Me: Methyl, Et: Ethyl, tBu: tert-Butyl, iPr: Isopropyl, Ph: Phenyl, TFA: Trifluoroacetic acid, and in addition, as in the compound of Example 110, the crossed double bond shows that it is a mixture of an (E)-form and a (Z)-form, or that whether it is an (E)-form or a (Z)-form is not specified.

**[Table 2]**

| PEx | Syn | Structure | Data |
|---|---|---|---|
| 1 | P1 | 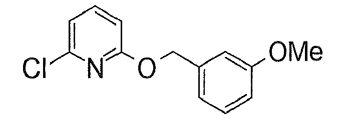 | EI:249 |
| 2 | P2 | 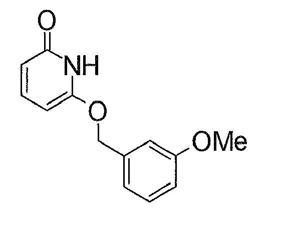 | ESI+:232 |
| 19 | P3 | 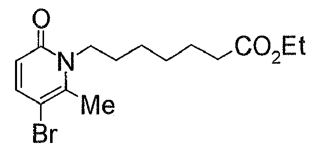 | ESI+: 344, 346 |
| 20 | P4 | 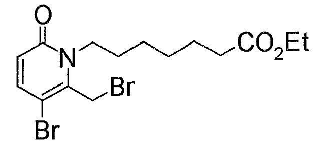 | ESI+: 422, 424,426 |
| 21 | P3 | 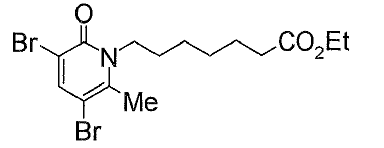 | ESI+: 422, 424,426 |
| 22 | P4 | 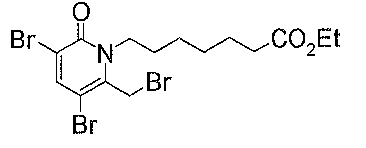 | ESI+: 500, 502, 504, 506 |

**[Table 3]**

| | | | |
|---|---|---|---|
| 23 | P3 | 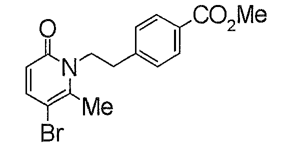 | FAB+: 350, 352 |
| 24 | P4 | 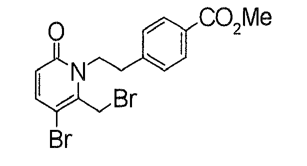 | FAB+: 428, 430,432 |
| 25 | P3 | 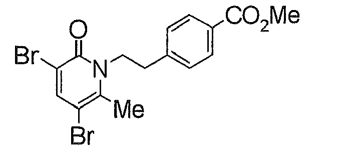 | ESI+: 428, 430,432 |
| 26 | P4 | 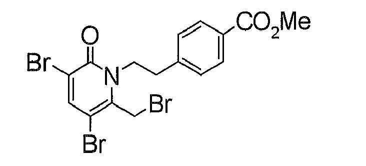 | ESI+: 506, 508, 510, 512 |
| 3 | P3 | 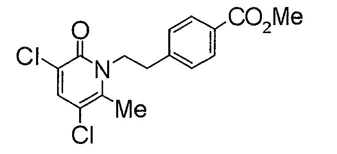 | ESI+: 340, 342, 344 |
| 4 | P4 | 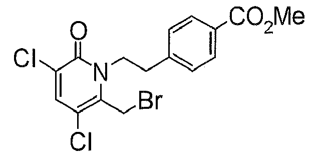 | ESI+: 418, 420,422,424 |
| 5 | P5 | 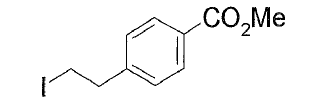 | CI+:291 |
| 6 | P6 | 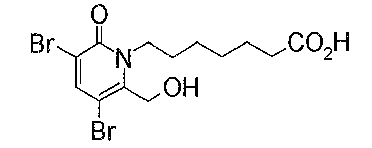 | FAB+: 410, 412,414 |

**[Table 4]**

| | | | |
|---|---|---|---|
| 7 | P7 | 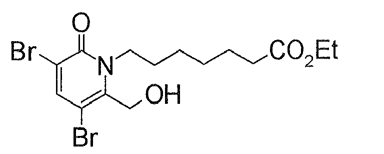 | ESI+: 438, 440,442 |
| 27 | P13 | 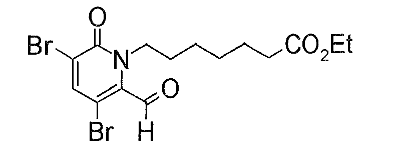 | FAB+: 436, 438,440 |
| 28 | P10 | 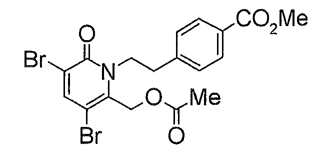 | ESI+: 486, 488,490 |
| 12 | P12 | 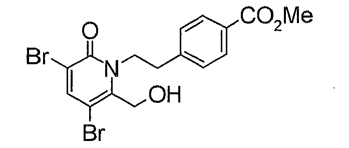 | ESI+: 444, 446,448 |
| 29 | P13 | 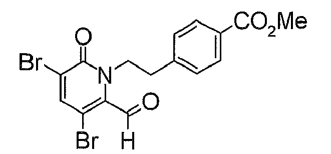 | FAB+: 442, 444,446 |
| 14 | P14 | 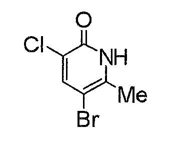 | ESI+: 222 224, 226 |
| 15 | 15 | 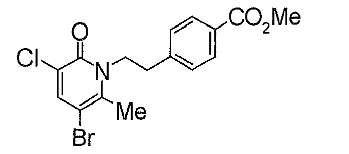 | CI+: 384 386, 388 |
| 30 | P9 | 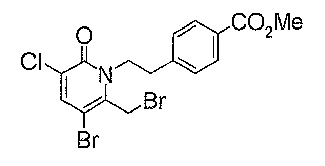 | ESI+: 462,464,466,4 68 |

**[Table 5]**

| | | | |
|---|---|---|---|
| 16 | P16 | 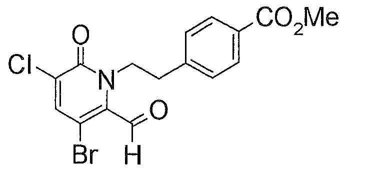 | CI+: 398, 400, 402 |
| 8 | P8 | 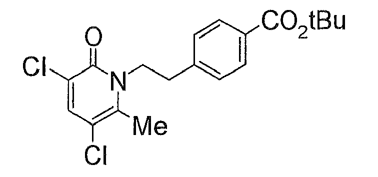 | FAB+: 382, 384, 386 |
| 9 | P9 | 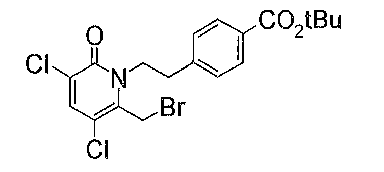 | FAB+: 460, 462,464,466 |
| 10 | P10 | 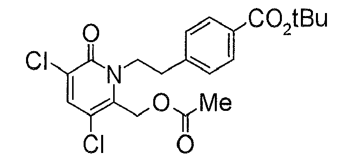 | FAB+: 440, 442,444 |
| 11 | P11 | 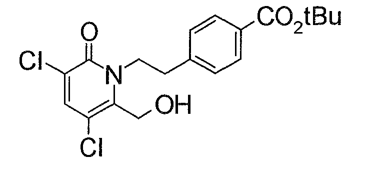 | FAB+: 398, 400,402 |
| 13 | P13 | 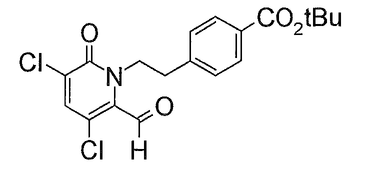 | FAB+: 396, 398,340 |

**[Table 6]**

| Ex | Structure |
|---|---|
| 9 | 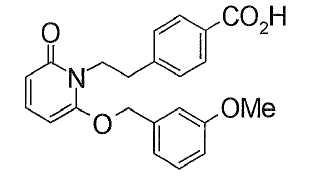 |
| 11 | 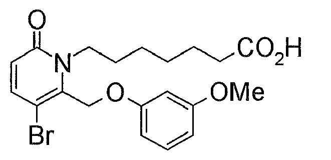 |
| 12 | 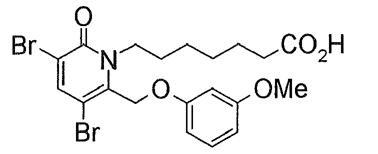 |
| 13 | 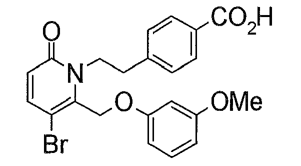 |
| 2 | 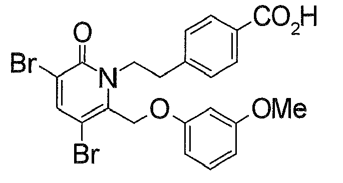 |

**[Table 7]**

| | |
|---|---|
| 1 | 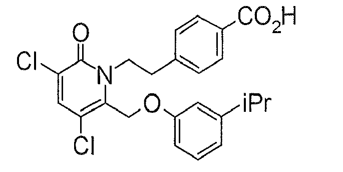 |
| 14 | 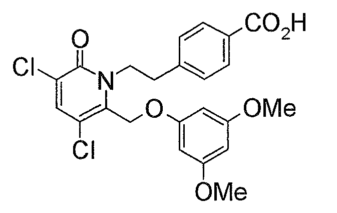 |
| 15 | 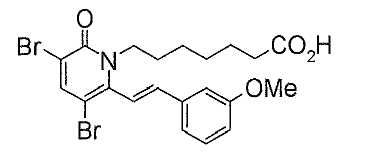 |
| 4 | 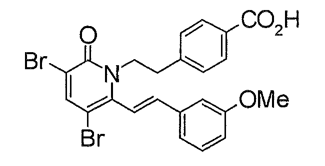 |
| 3 | 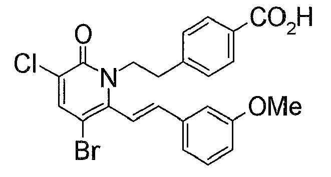 |
| 5 | 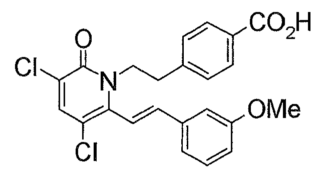 |

**[Table 8]**

| | |
|---|---|
| 17 | 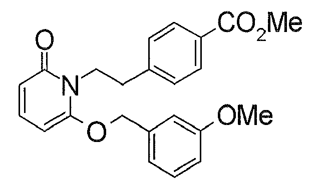 |
| 19 | 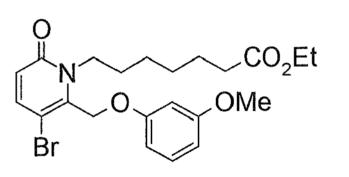 |
| 20 | 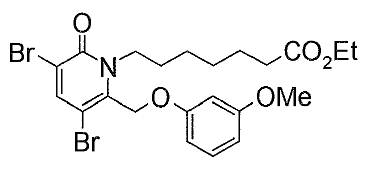 |
| 21 | 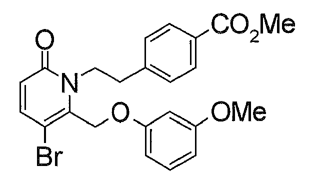 |
| 22 | 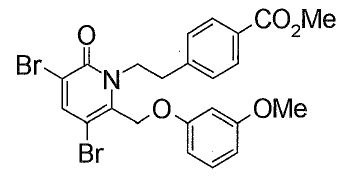 |
| 6 | 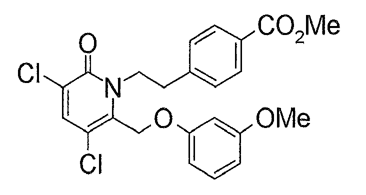 |

**[Table 9]**

| | |
|---|---|
| 23 | 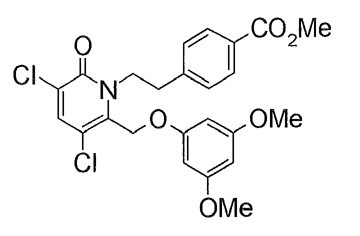 |
| 24 | 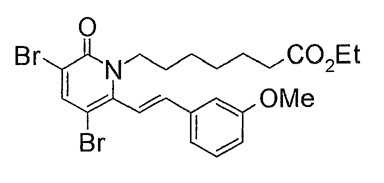 |
| 25 | 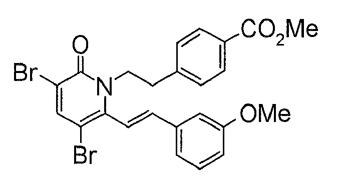 |
| 26 | 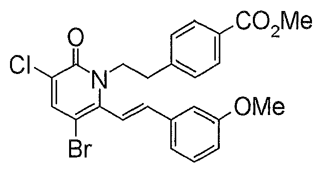 |
| 7 | 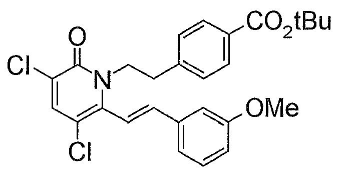 |

**[Table 10]**

| Ex | Syn | Data |
|---|---|---|
| 9 | 1 | ESI+:380 |
| 11 | 1 | ESI+; 438, 440 |
| 12 | 1 | ESI+: 516, 518, 520 NMR 1.18-1.27 (4H, m), 1.38-1.48 (2H, m), 1.60-1.70 (2H, m), 2.12 (2H, t, J = 8.0 Hz), 3.75 (3H, s), 3.98-4.06 (2H, m), 5.20 (2H, s), 6.58-6.68 (3H, m), 7.22-7.28 (1H, m), 8.26 (1H, s), 11.96 (1H, s) |
| 13 | 1 | ESI+: 458, 460 |
| 2 | 2 | ESI+: 536, 538, 540 NMR: 3.01-3.11 (2H, m),3.75 (3H, s), 4.22-4.31 (2H, m), 5.23 (2H, s), 6.60-6.70 (3H, m), 7.22-7.30 (3H, m), 7.81 (2H, d, J = 8.4 Hz), 8.31 (1H, s), 12.88 (1H, s) |
| 1 | 1 | FAB+: 460, 462, 464 NMR: 1.19 (6H, d, J = 6.8Hz), 2.87 (1H, septet, J = 6.8Hz), 3.02-3.12 (2H, m), 4.21-4.28 (2H, m), 5.21 (2H, s), 6.87-6.96 (3H, m), 7.21-7.30 (3H, m), 7.81 (2H, d, J = 8.0 Hz), 8.10 (1H, s), 12.87 (1H, s) |
| 14 | 2 | ESI+; 478, 480, 482 NMR: 3.02-3.11 (2H, m),3.72 (6H, s), 4.20-4.29 (2H, m), 5.19 (2H, s), 6.20 (1H, dd, J = 2.0, 2.0 Hz), 6.25-6.28 (2H, m), 7.29 (2H, d, J = 8.4 Hz), 7.84 (2H, d, J = 8.4 Hz), 8.10 (1H, s), 12.89 (1H, s) |
| 15 | 3 | ESI+: 512, 514, 516 NMR: 1.16-1.28 (4H, m), 1.34-1.48 (2H, m), 1.52-1.64 (2H, m), 2.09 (2H, t, J = 8.0 Hz), 3.80 (3H, s), 4.01-4.12 (2H, m), 6.93-7.01 (2H, m), 7.06 (1H, d, J = 16.8 Hz), 7.20-7.26 (2H, m), 7.35 (1H, dd, J = 8.0, 8.0 Hz), 8.22 (1H, s), 11.93 (1H, s) |
| 4 | 4 | ESI+: 532, 534, 536 NMR: 2.97-3.04 (2H, m), 3.82 (3H, s), 4.27-4.34 (2H, m), 6.80 (1H, d, J = 16.4 Hz), 6.89 (1H, d, J = 16.4 Hz), 6.94-6.99 (1H, m), 7.14-7.19 (2H, m), 7.23 (2H, d, J = 8.4 Hz), 7.35 (1H, dd, J = 8.0, 8.0 Hz), 7.81 (2H, d, J = 8.4 Hz), 8.27 (1H, s), 12.88 (1H, s) |

**[Table 11]**

| | | |
|---|---|---|
| 3 | 3 | ESI+: 488, 490, 492 NMR: 2.95-3.05 (2H, m), 3.82 (3H, s), 4.25-4.36 (2H, m), 6.79 (1H, d, J = 16.4 Hz), 6.90 (1H, d, J = 16.4 Hz), 6.95-7.00 (1H, m), 7.14-7.18 (2H, m), 7.22 (2H, d, J = 8.0 Hz), 7.35 (1H, dd, J = 8.0, 8.0 Hz), 7.81 (2H, d, J = 8.0 Hz), 8.13 (1H, s), 12.89 (1H, s) |
| 5 | 5 | ESI+: 444, 446, 448 NMR: 2.98.-3.05 (2H, m), 3.82 (3H, s), 4.26-4.35 (2H, m), 6.83 (1H, d, J = 16.4 Hz), 6.90 (1H, d, J = 16.4 Hz), 6.95-7.00 (1H, m), 7.13-7.19 (2H, m), 7.24 (2H, d, J = 8.0 Hz), 7.35 (1H, dd, J = 8.0, 8.0 Hz), 7.81 (2H, d, J = 8.0 Hz), 8.06 (1H, s), 12.88 (1H, s) |
| 17 | P3 | ESI+:394 |
| 19 | 6 | ESI+: 466, 468 |
| 20 | 6 | ESI+: 544, 546, 548 |
| 21 | 6 | ESI+: 472, 474 |
| 22 | 6 | ESI+: 550, 552, 554 |
| 6 | 6 | ESI+: 462, 464, 466 |
| 23 | 6 | FAB+: 492, 494, 496 |
| 24 | 7 | FAB+: 540, 542, 544 |
| 25 | 7 | ESI+: 546, 548, 550 |
| 26 | 7 | ESI+: 502, 504, 506 |
| 7 | 7 | ESI+: 500, 502, 504 |

**[Table 12]**

| PEx | Syn | Structure | Data |
|---|---|---|---|
| 32 | P32 | 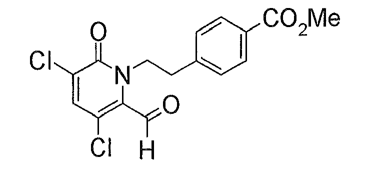 | EI: 353, 355, 357 |
| 33 | P33 | 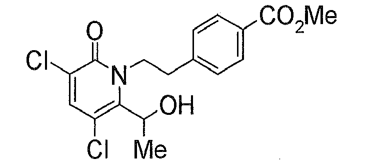 | ESI+; 370, 372 |
| 34 | P34 | 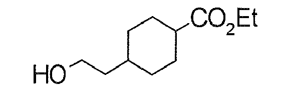 | ESI+; 201 |
| 35 | P35 | 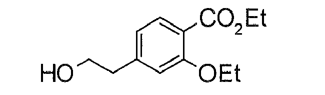 | EI: 238, 239 |
| 36 | P36 | 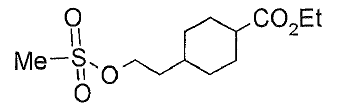 | ESI+: 279 |
| 37 | P37 | 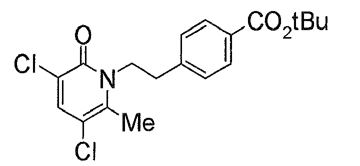 | ESI+: 382, 384, 386 |
| 38 | P38 | 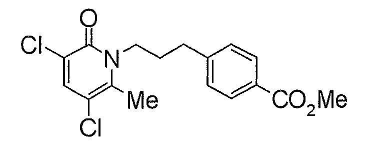 | ESI+: 354 |
| 39 | P39 | 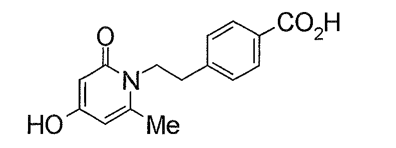 | ESI+: 274 |
| 40 | P40 | 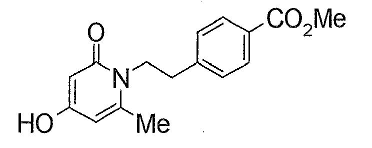 | ESI+: 288 |

**[Table 13]**

| | | | |
|---|---|---|---|
| 41 | P41 | 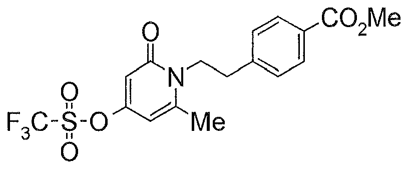 | EI: 419 |
| 42 | P42 | 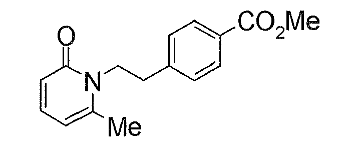 | ESI+: 272 |
| 43 | P43 | 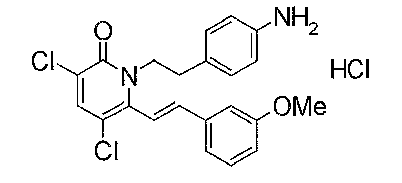 | ESI+: 415 |
| 44 | P44 | 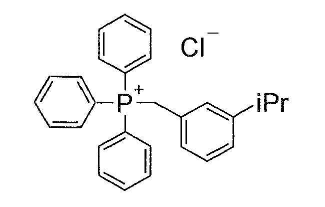 | ESI+: 395 |
| 45 | P45 | 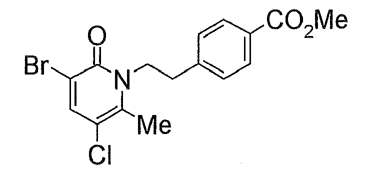 | ESI+: 384, 386, 388 |
| 46 | P46 | 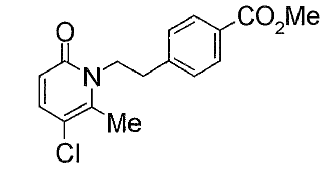 | EI: 305, 307 |
| 47 | P47 | 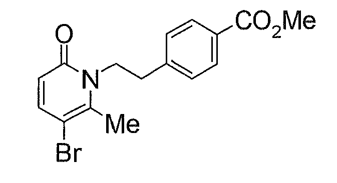 | EI: 349, 351 |

**[Tabe 14]**

| | | | |
|---|---|---|---|
| 48 | P48 | 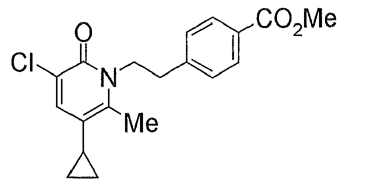 | ESI+: 346, 348 |
| 49 | P49 | 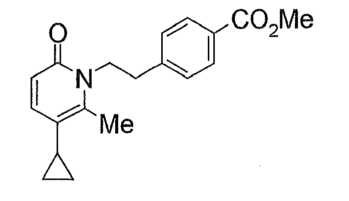 | ESI+: 312 |
| 50 | P50 | 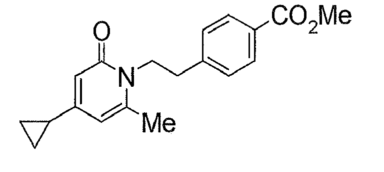 | FAB+: 312 |
| 51 | P51 | 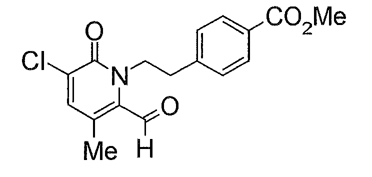 | CI+: 334, 336 |
| 52 | P52 | 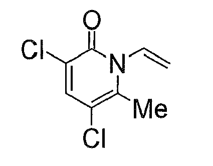 | ESI+: 204, 206 |
| 53 | P53 | 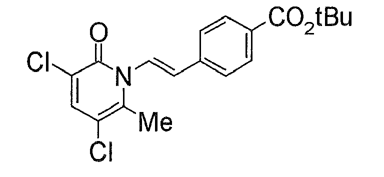 | FAB+: 380 |
| 54 | P54 | 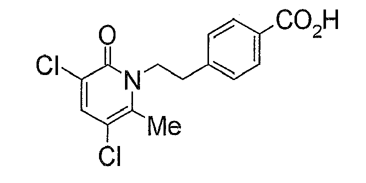 | ESI+: 326, 328, 330 |

**[Table 15]**

| | | | |
|---|---|---|---|
| 55 | P55 | 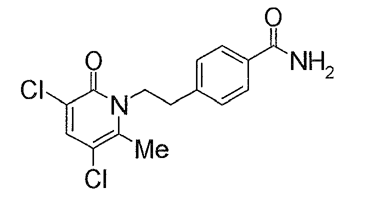 | FAB+: 325 |
| 56 | P56 | 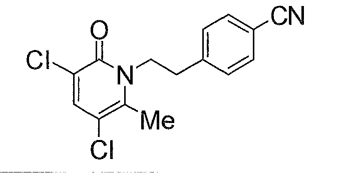 | ESI+: 307, 309, 311 |
| 57 | P57 | 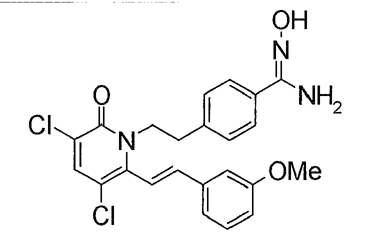 | ESI+: 458 |
| 58 | P58 | 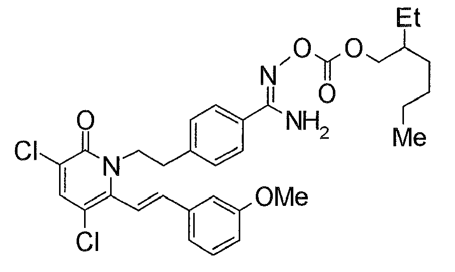 | ESI+: 614 |
| 59 | P59 | 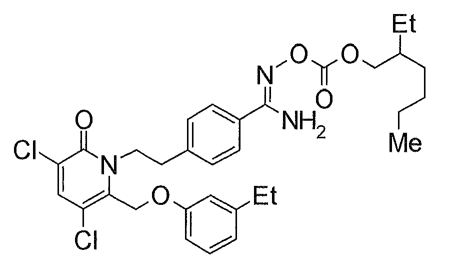 | ESI+: 616, 618 |

**[Table 16]**

| | | | |
|---|---|---|---|
| 60 | P60 | 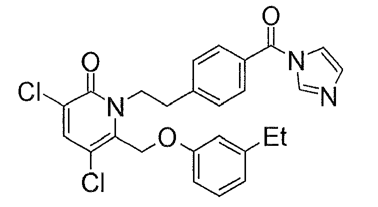 | FAB+: 496 |
| 61 | P61 | 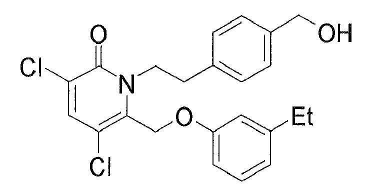 | FAB+: 432 |
| 62 | P62 | 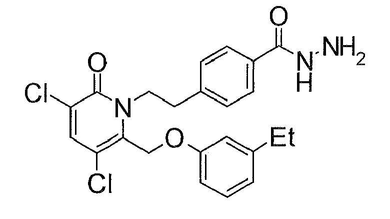 | ESI+; 460, 462, 463 |
| 63 | P63 | 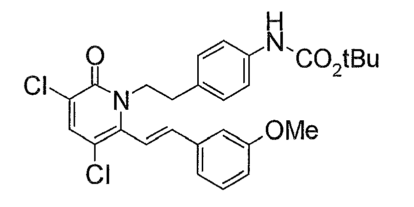 | FAB+: 515 |
| 64 | P36 | 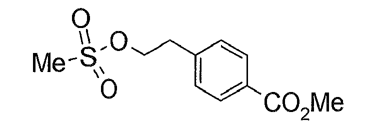 | CI+; 259 |
| 65 | P3 | 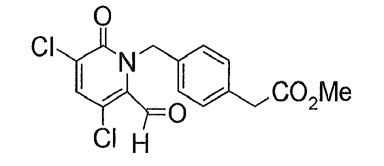 | ESI-: 353 355 |
| 66 | P3 | 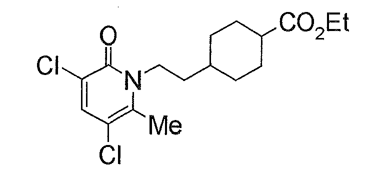 | ESI+: 360, 362, 364 |

**[Table 17]**

| | | | |
|---|---|---|---|
| 67 | P4 | 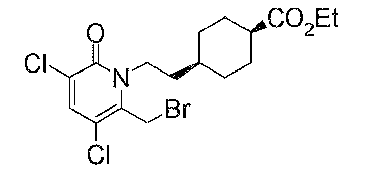 | ESI+: 438, 440, 442 |
| 68 | P4 | 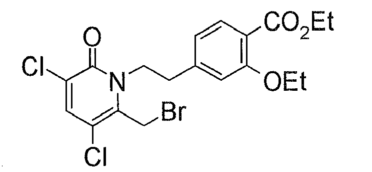 | FAB+: 478 |
| 69 | P4 | 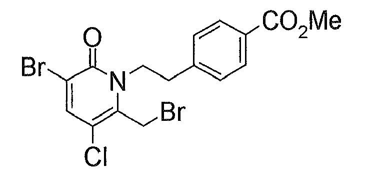 | ESI+: 462, 464, 466 |
| 70 | P4 | 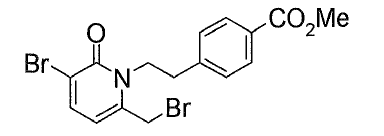 | ESI+: 428, 430, 432 |
| 71 | P4 | 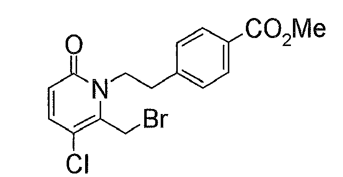 | ESI+: 384, 386, 388 |
| 72 | P4 | 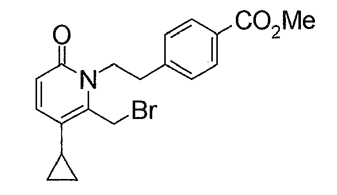 | ESI+: 390, 392 |
| 73 | P4 | 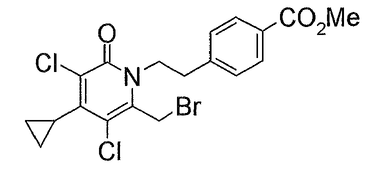 | ESI+: 458, 460, 462, 463 |

**[Table 18]**

| | | | |
|---|---|---|---|
| 74 | P4 | 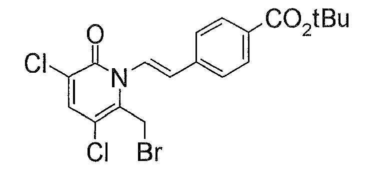 | ESI+: 458, 460 |
| 75 | P4 | 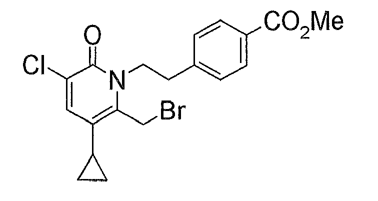 | ESI+: 424, 426, 428 |
| 76 | P4 | 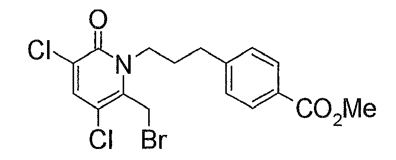 | ESI+: 432, 434 |
| 77 | P4 | 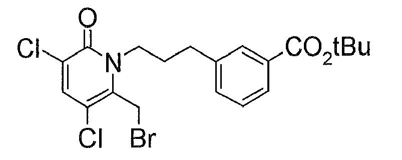 | ESI+: 474 |
| 78 | P4 | 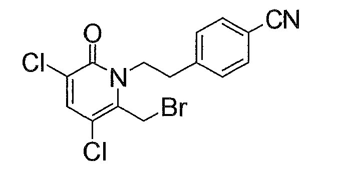 | ESI+: 385, 387 |
| 79 | P8 | 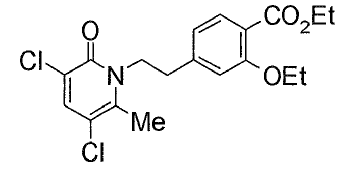 | ESI+: 398 |
| 80 | P8 | 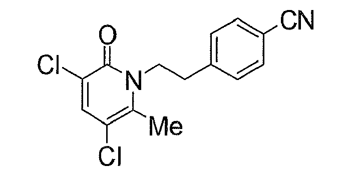 | ESI+: 307, 309, 311 |

**[Table 19]**

| | | | |
|---|---|---|---|
| 81 | P10 | 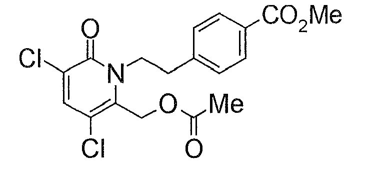 | ESI+: 398, 400, 402 |
| 82 | P10 | 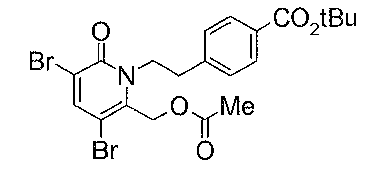 | FAB+: 528, 530, 532 |
| 83 | P10 | 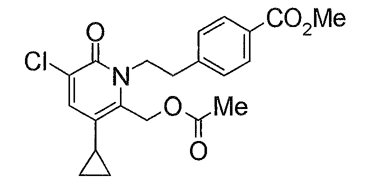 | ESI+: 404, 406 |
| 84 | P10 | 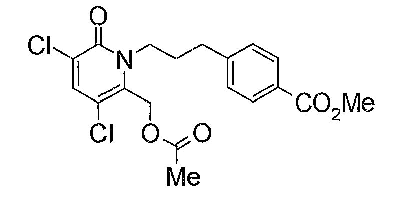 | ESI+: 412 |
| 85 | P10 | 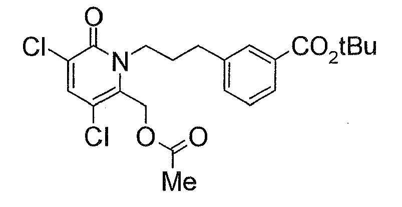 | FAB+: 454 |
| 86 | P10 | 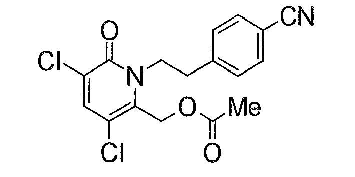 | ESI+: 365, 367, 369 |
| 87 | P11 | 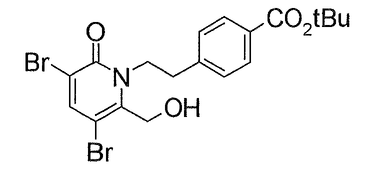 | FAB+: 486, 488, 490 |

**[Table 20]**

| | | | |
|---|---|---|---|
| 88 | P11 | 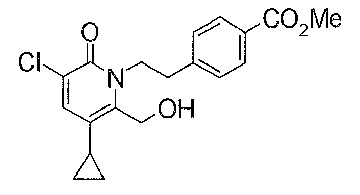 | ESI+: 362, 364 |
| 89 | P11 | 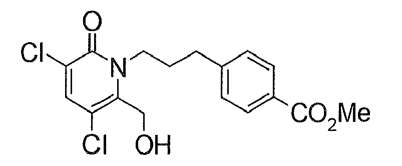 | ESI+: 371 |
| 90 | P11 | 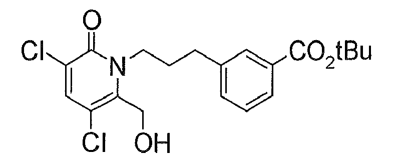 | ESI+: 412 |
| 91 | P11 | 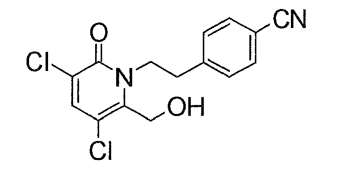 | ESI+: 323, 325, 327 |
| 92 | P12 | 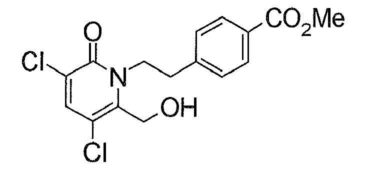 | ESI+: 356, 358, 360 |
| 93 | P13 | 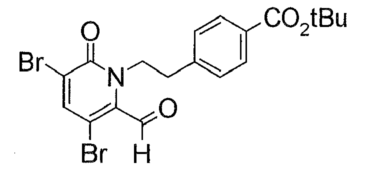 | FAB+: 484, 486, 488 |
| 94 | P13 | 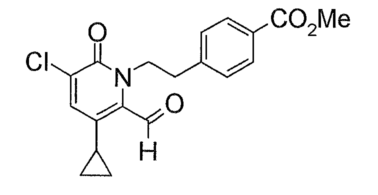 | FAB+: 360, 362 |

**[Table 21]**

| | | | |
|---|---|---|---|
| 95 | P13 | 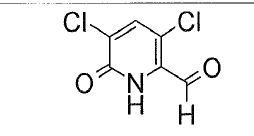 | ESI-: 190, 192, 194 |
| 96 | P47 | 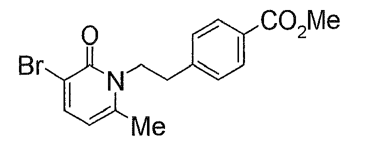 | EI: 349, 351 |
| 97 | P4 | 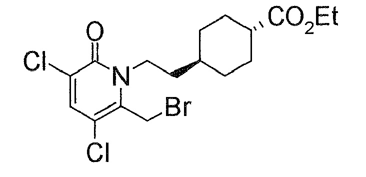 | ESI+: 438, 440, 442 |
| 98 | P13 | 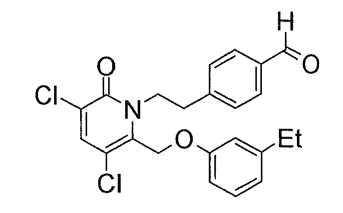 | ESI+: 430, 432, 433 |
| 99 | P14 | 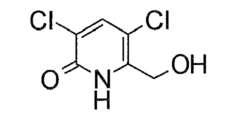 | FAB+: 194, 196, 198 |
| 100 | P16 | 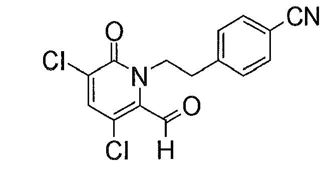 | CI+: 321 |
| 101 | P32 | 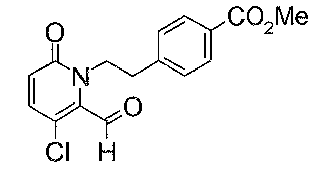 | FAB+: 320, 322 |
| 102 | P32 | 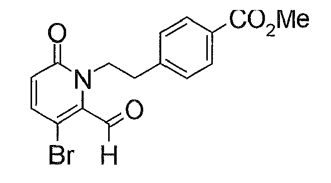 | FAB+: 364, 366 |

**[Table 22]**

| | | | |
|---|---|---|---|
| 103 | P36 | 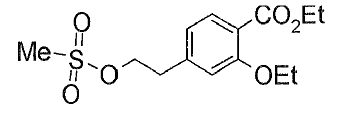 | EI: 316, 317 |
| 105 | P40 | 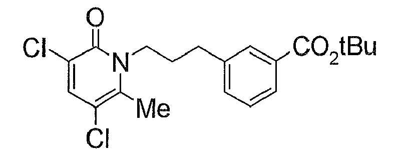 | FAB+: 396 |
| 107 | P44 | 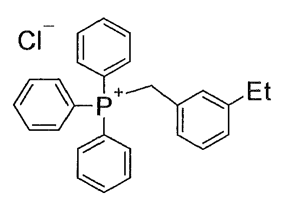 | ESI+: 381 |
| 108 | P44 | 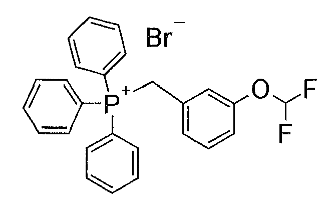 | FAB+: 419 |
| 109 | P44 | 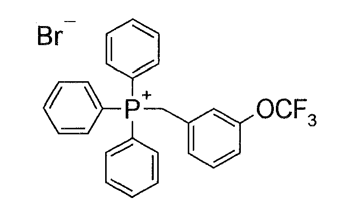 | FAB+: 437 |

**[Table 23]**

| | | | |
|---|---|---|---|
| 110 | P44 | 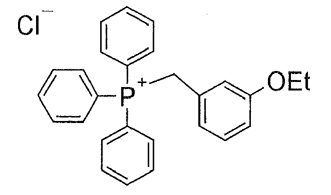 | FAB+: 397 |
| 111 | P44 | 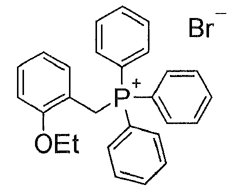 | FAB+: 397 |
| 112 | P44 | 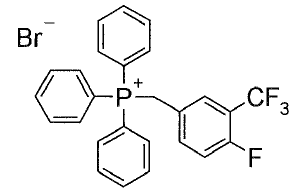 | ESI+: 439 |
| 113 | P44 | 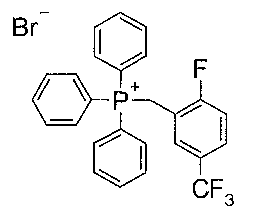 | ESI+: 439 |
| 114 | P44 | 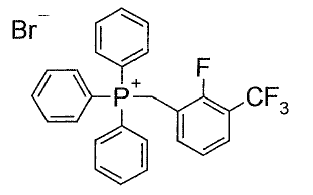 | ESI+: 439 |
| 115 | P44 | 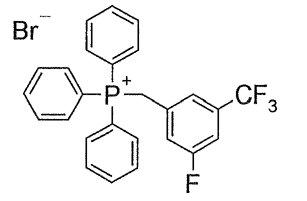 | ESI+: 439 |

**[Table 24]**

| | | | |
|---|---|---|---|
| 116 | P44 | 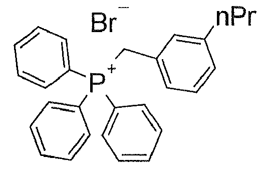 | ESI+: 395 |
| 117 | P44 | 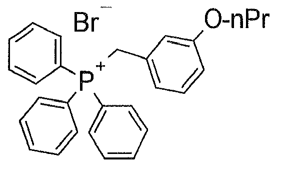 | ESI+: 411 |
| 118 | P44 | 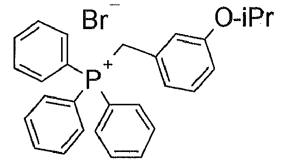 | APCI+: 411 |
| 119 | P44 | 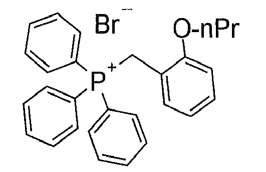 | ESI+: 411 |
| 120 | P44 | 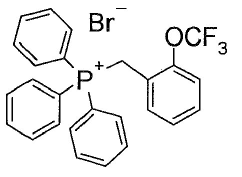 | ESI+: 437 |
| 121 | P44 | 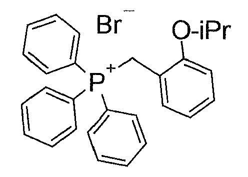 | ESI+: 411 |
| 122 | P44 | 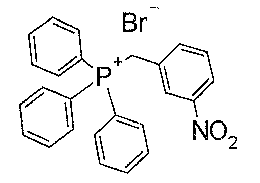 | FAB+: 398 |

**[Table 25]**

| | | | |
|---|---|---|---|
| 123 | P48 | 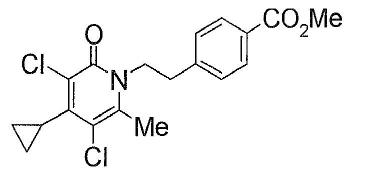 | ESI+: 380, 382, 383 |

**[Table 26]**

| Ex | Structure | | Ex | Structure |
|---|---|---|---|---|
| 27 | 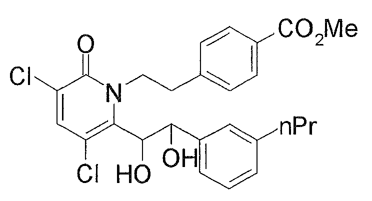 | | 28 | 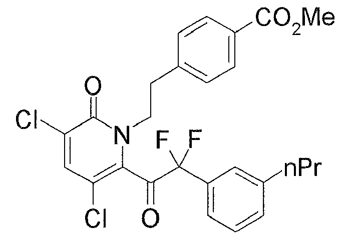 |
| 29 | 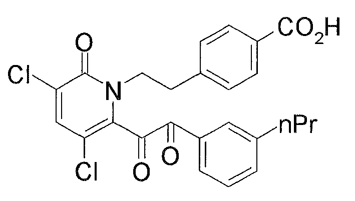 | | 30 | 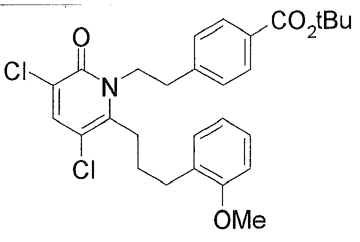 |
| 31 | 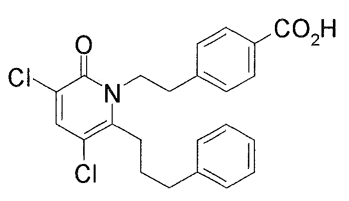 | | 32 | 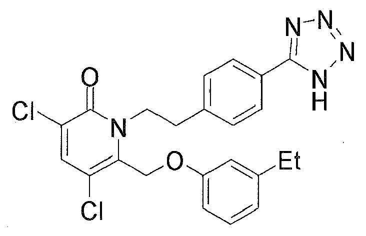 |
| 33 | 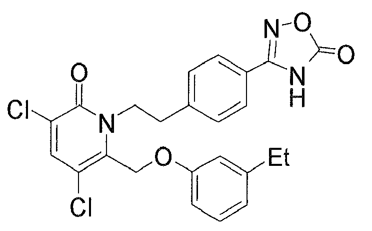 | | 34 | 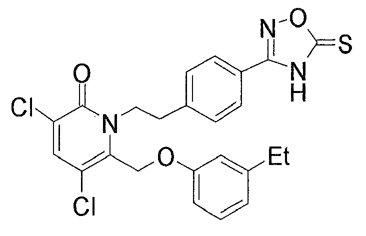 |

**[Table 27]**

| | | | | |
|---|---|---|---|---|
| 35 | 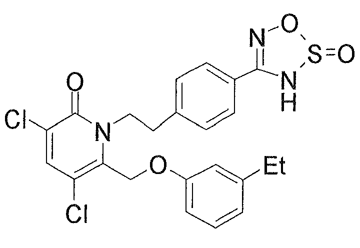 | | 36 | 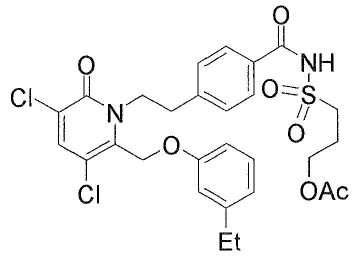 |
| 37 | 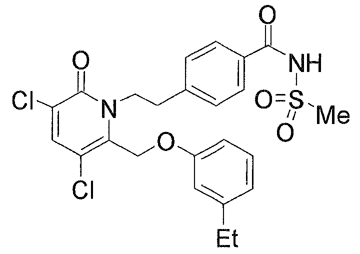 | | 38 | 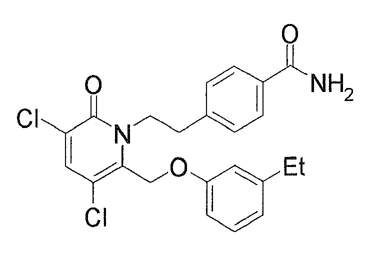 |
| 39 | 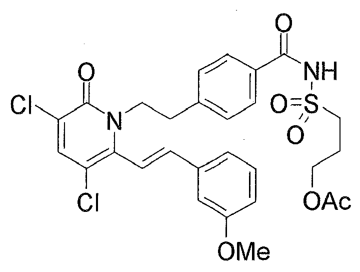 | | 40 | 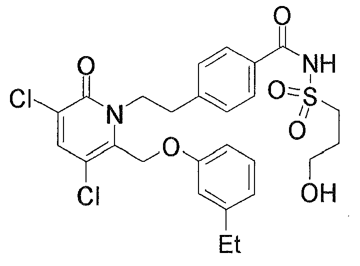 |
| 41 | 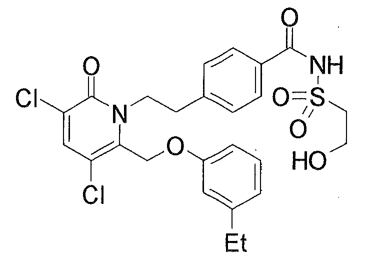 | | 42 | 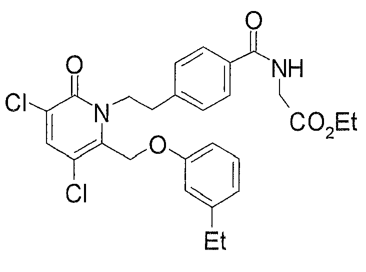 |

**[Table 28]**

| | | | | |
|---|---|---|---|---|
| 43 | 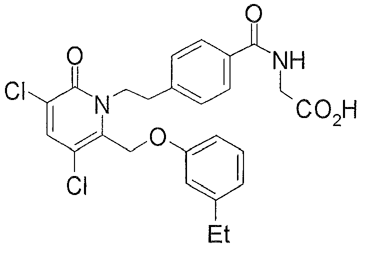 | | 44 | 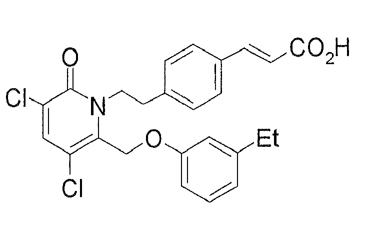 |
| 45 | 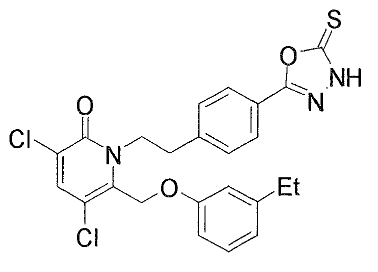 | | 46 | 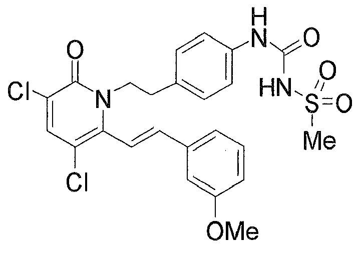 |
| 47 | 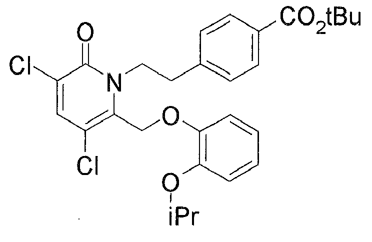 | | 48 | 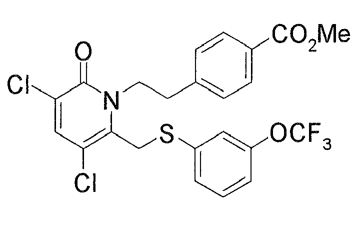 |
| 49 | 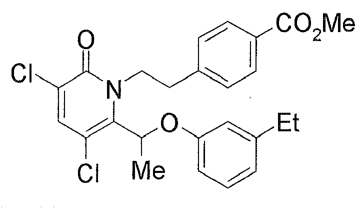 | | 50 | 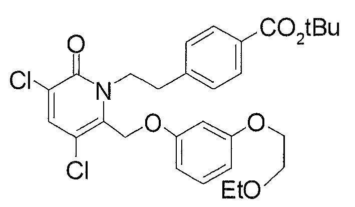 |
| 51 | 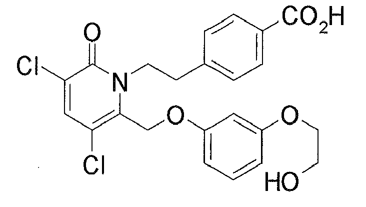 | | 52 | 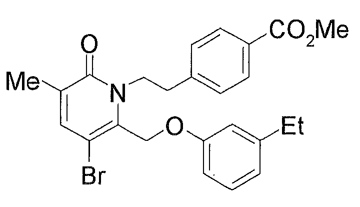 |

**[Table 29]**

| | | | | |
|---|---|---|---|---|
| 53 | 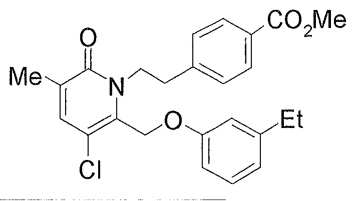 | | 54 | 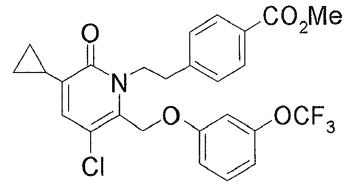 |
| 55 | 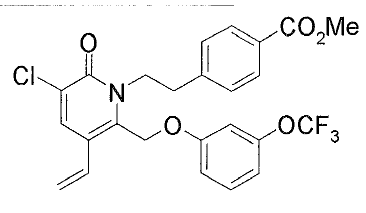 | | 56 | 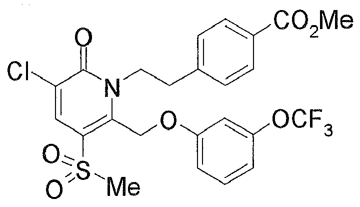 |
| 57 | 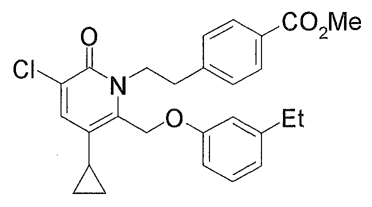 | | 58 | 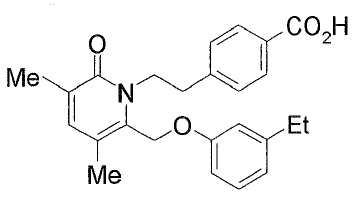 |
| 59 | 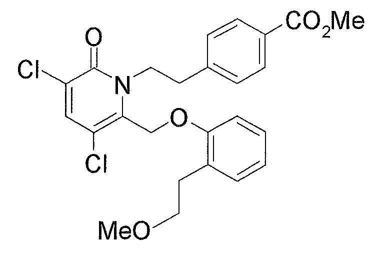 | | 60 | 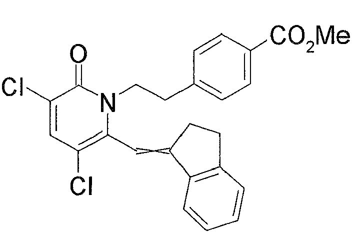 |
| 61 | 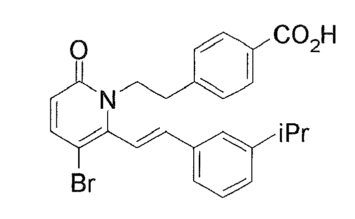 | | 62 | 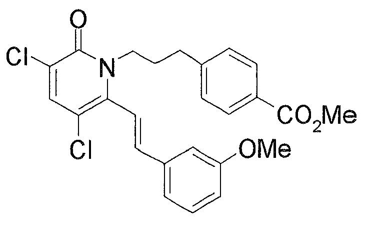 |

**[Table 30]**

| | | | | |
|---|---|---|---|---|
| 63 | 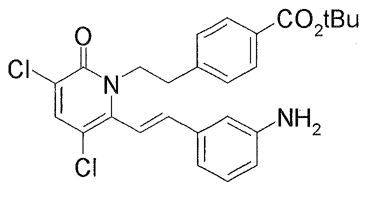 | | 64 | 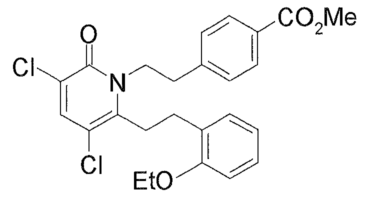 |
| 65 | 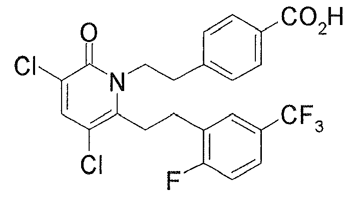 | | 66 | 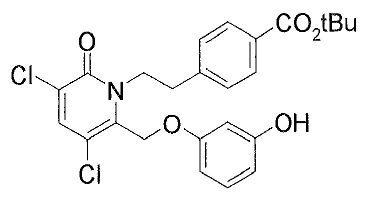 |
| 67 | 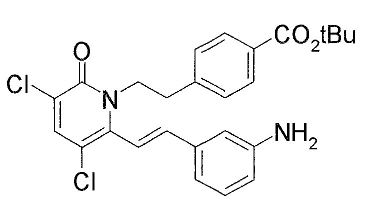 | | 68 | 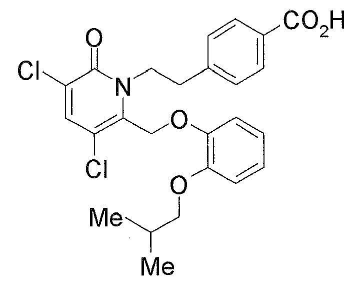 |
| 69 | 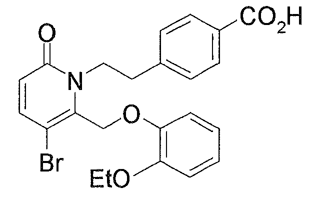 | | 70 | 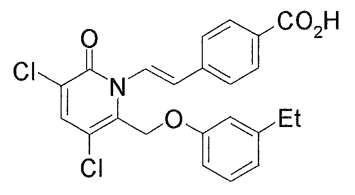 |
| 71 | 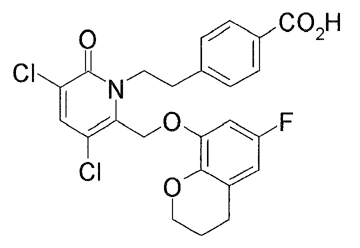 | | 72 | 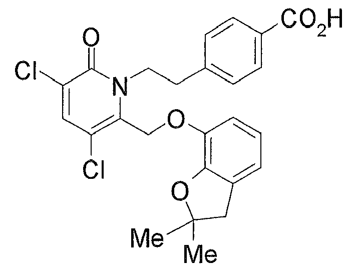 |

**[Table 31]**

| | | | | |
|---|---|---|---|---|
| 73 | 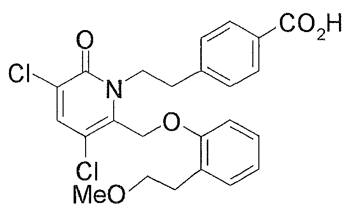 | | 74 | 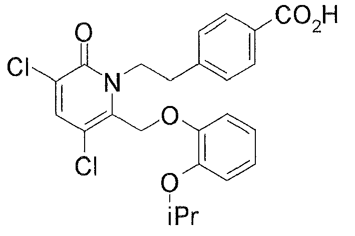 |
| 75 | 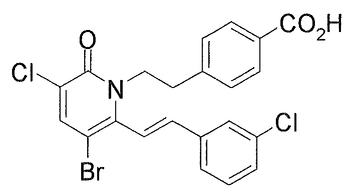 | | 76 | 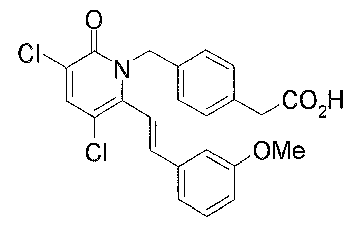 |
| 77 | 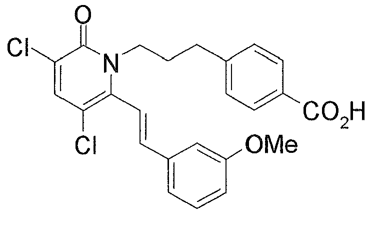 | | 78 | 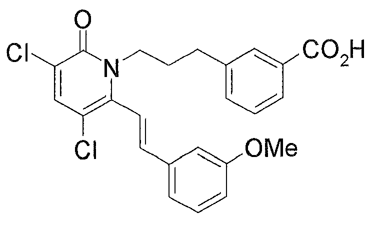 |
| 79 | 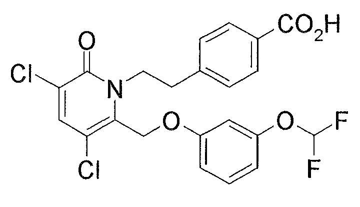 | | 80 | 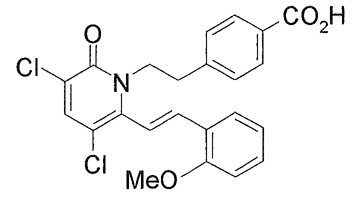 |
| 81 | 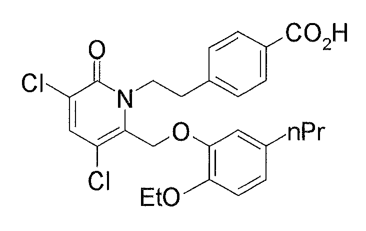 | | 82 | 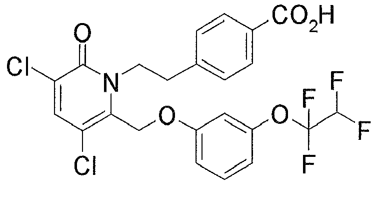 |

**[Table 32]**

| | | | | |
|---|---|---|---|---|
| 83 | 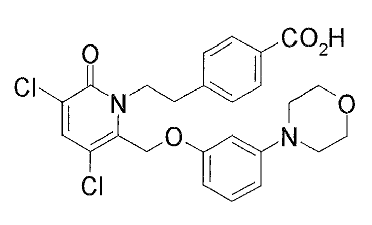 | | 84 | 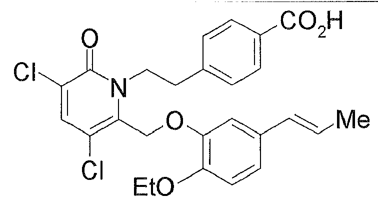 |
| 85 | 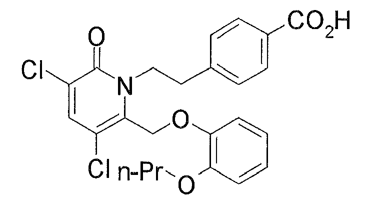 | | 86 | 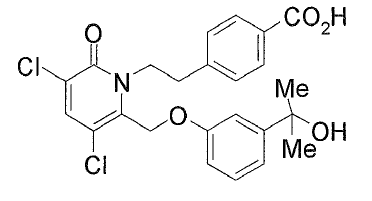 |
| 87 | 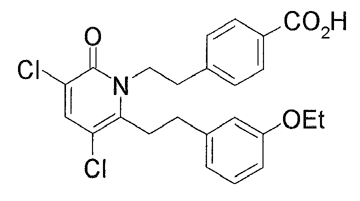 | | 88 | 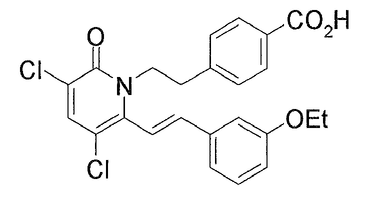 |
| 89 | 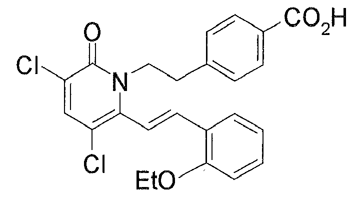 | | 90 | 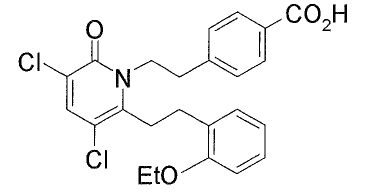 |
| 91 | 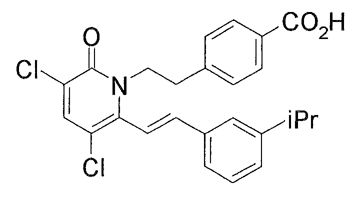 | | 92 | 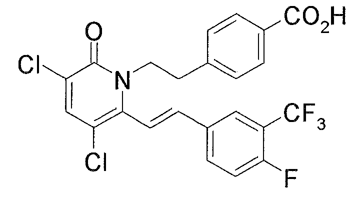 |

**[Table 33]**

| | | | | |
|---|---|---|---|---|
| 93 | 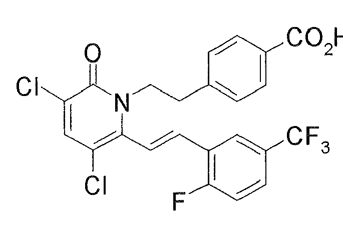 | | 94 | 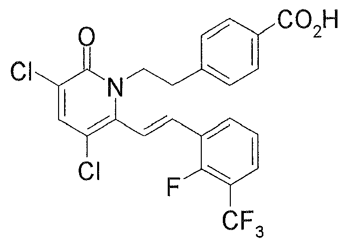 |
| 95 | 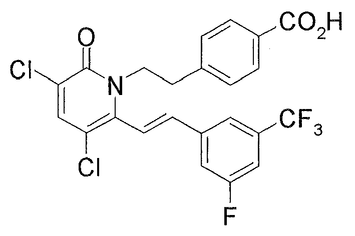 | | 96 | 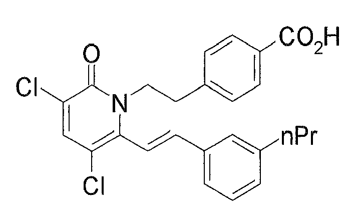 |
| 97 | 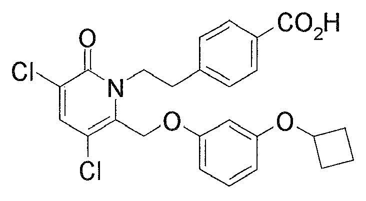 | | 98 | 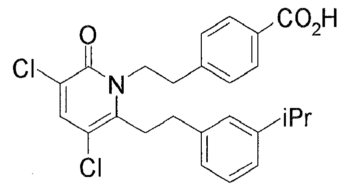 |
| 99 | 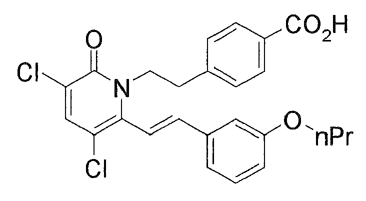 | | 100 | 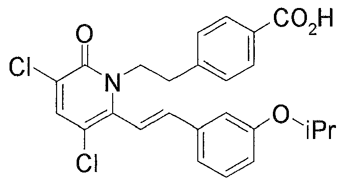 |
| 101 | 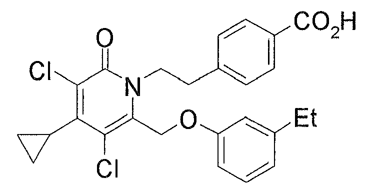 | | 102 | 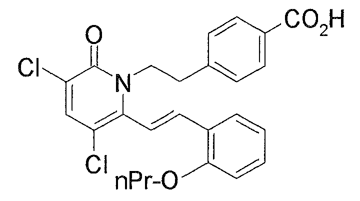 |

**[Table 34]**

| | | | | |
|---|---|---|---|---|
| 103 | 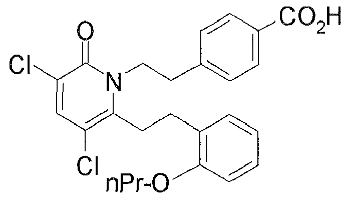 | | 104 | 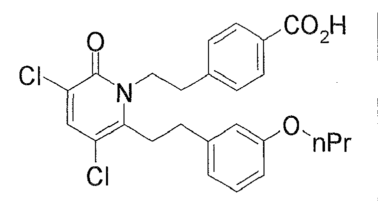 |
| 105 | 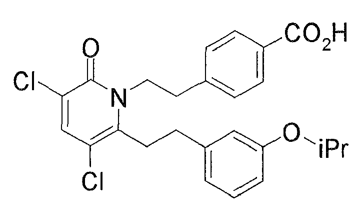 | | 106 | 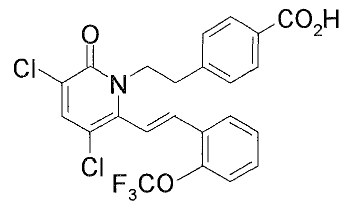 |
| 107 | 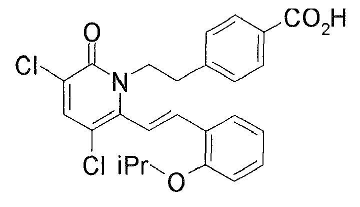 | | 108 | 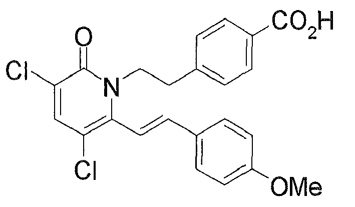 |
| 109 | 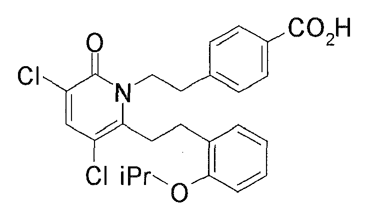 | | 110 | 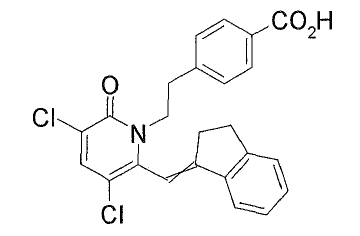 |
| 111 | 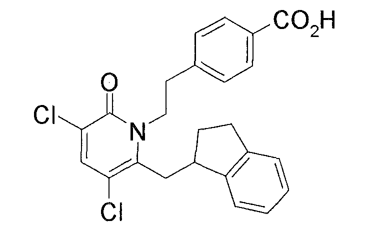 | | 112 | 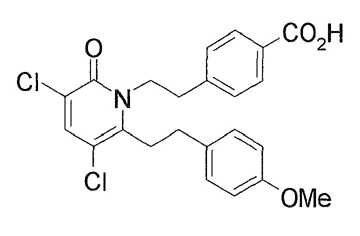 |

**[Table 35]**

| | | | | |
|---|---|---|---|---|
| 113 | 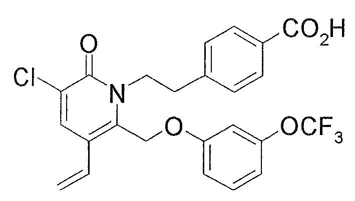 | | 114 | 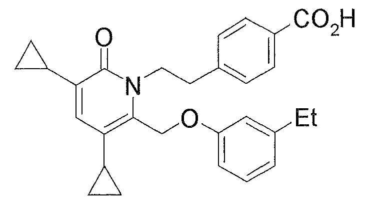 |
| 115 | 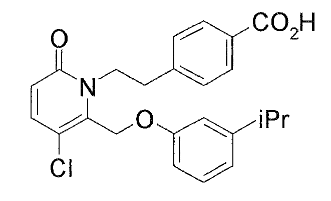 | | 116 | 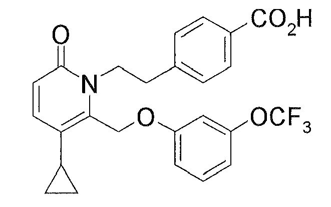 |
| 117 | 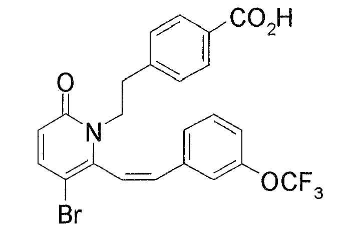 | | 118 | 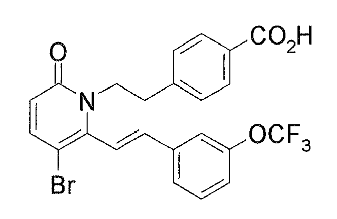 |
| 119 | 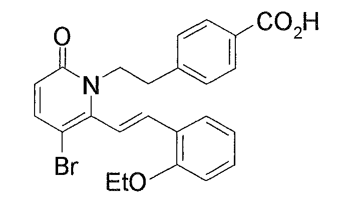 | | 120 | 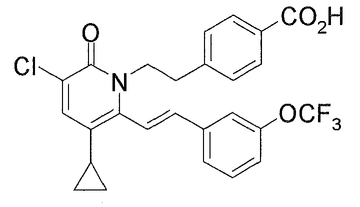 |
| 121 | 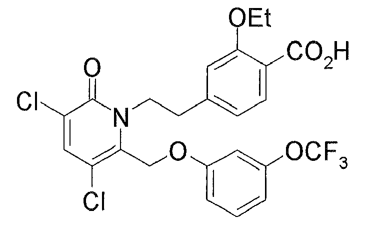 | | 122 | 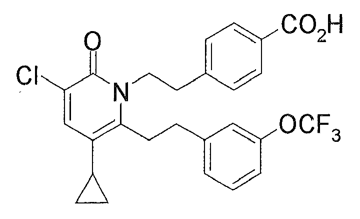 |

**[Table 36]**

| | | | | |
|---|---|---|---|---|
| 123 | 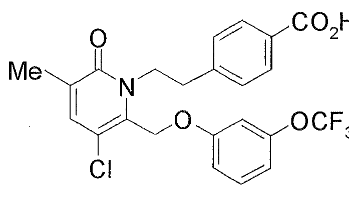 | | 124 | 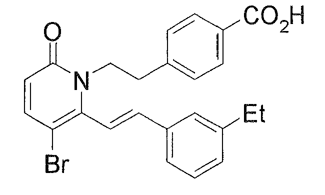 |
| 125 | 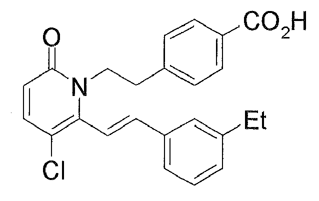 | | 126 | 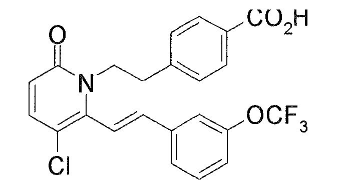 |
| 127 | 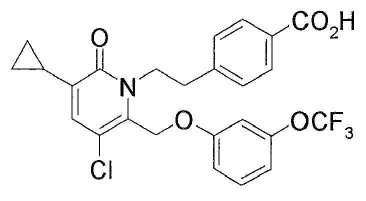 | | 128 | 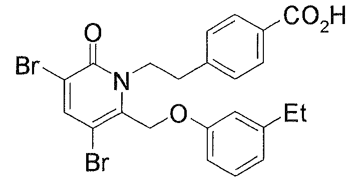 |
| 129 | 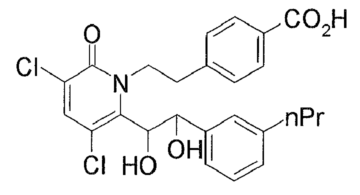 | | 130 | 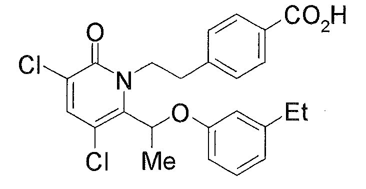 |
| 131 | 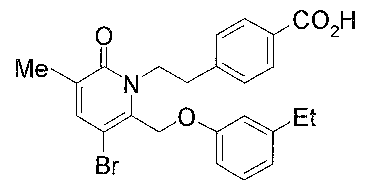 | | 132 | 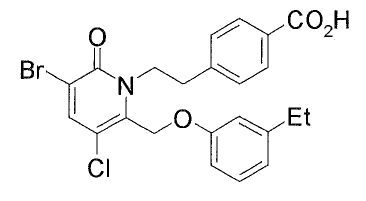 |

**[Table 37]**

| | | | | |
|---|---|---|---|---|
| 133 | 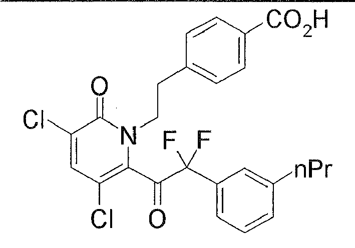 | | 134 | 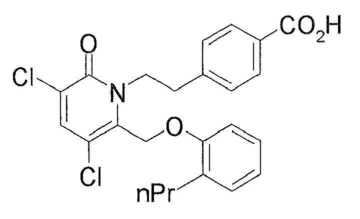 |
| 135 | 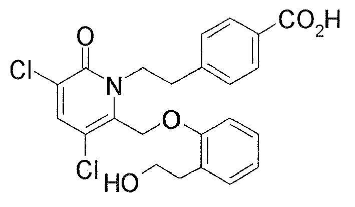 | | 136 | 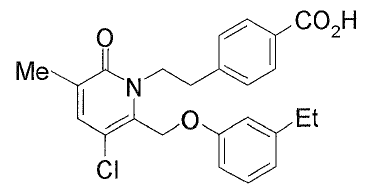 |
| 137 | 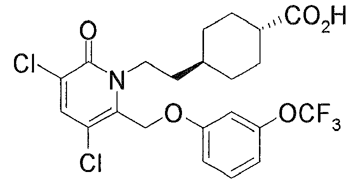 | | 138 | 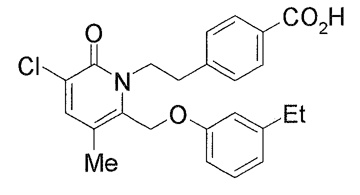 |
| 139 | 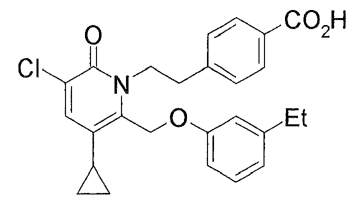 | | 140 | 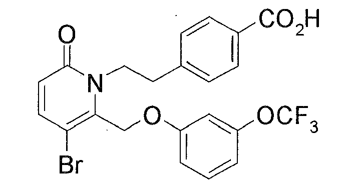 |
| 141 | 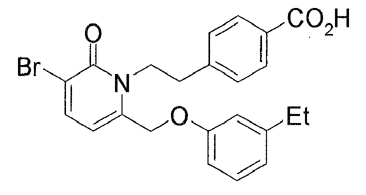 | | 142 | 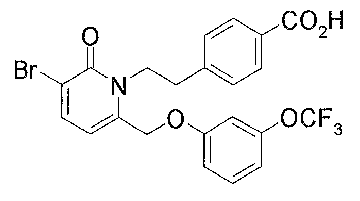 |

**[Table 38]**

| | | | | |
|---|---|---|---|---|
| 143 | 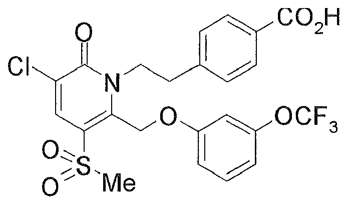 | | 144 | 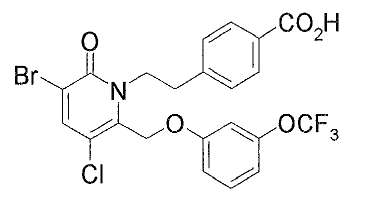 |
| 145 | 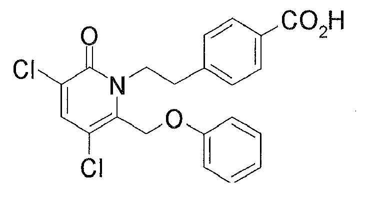 | | 146 | 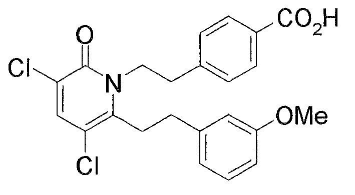 |
| 147 | 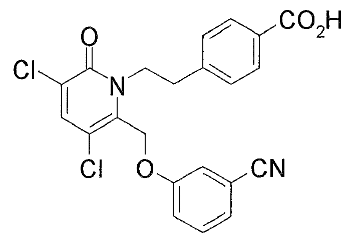 | | 148 | 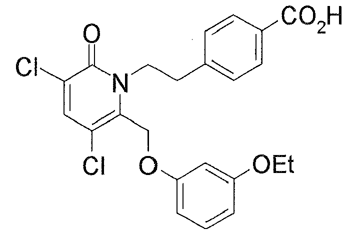 |
| 149 | 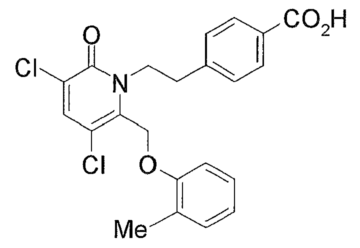 | | 150 | 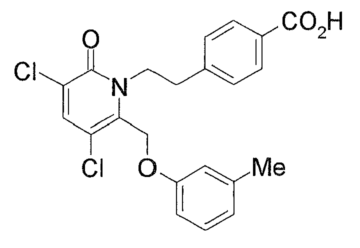 |
| 151 | 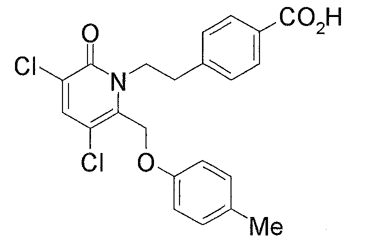 | | 152 | 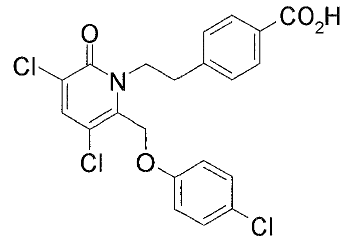 |

**[Table 39]**

| | | | | |
|---|---|---|---|---|
| 153 | 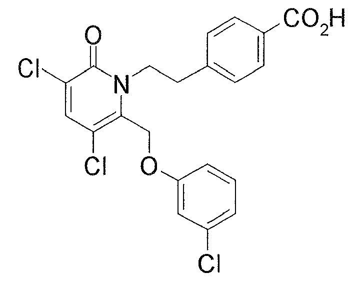 | | 154 | 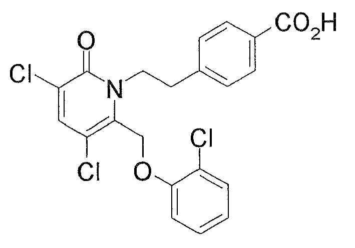 |
| 155 | 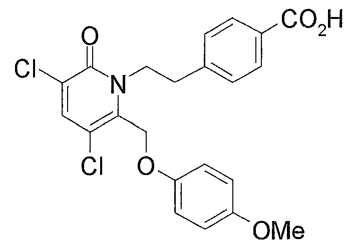 | | 156 | 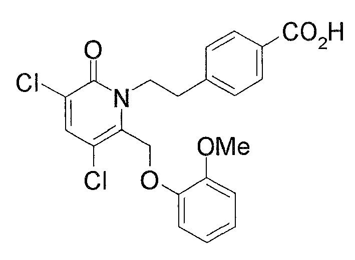 |
| 157 | 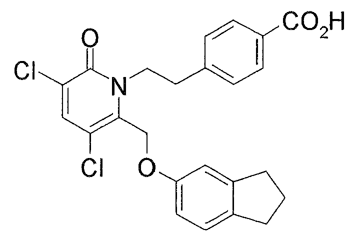 | | 158 | 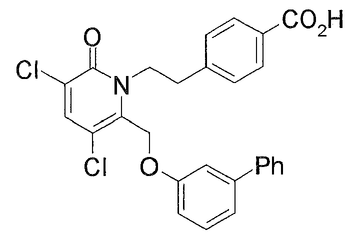 |
| 159 | 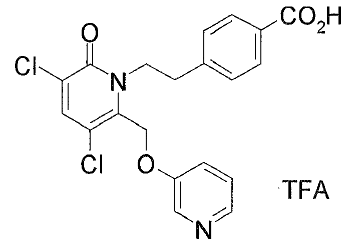 | | 160 | 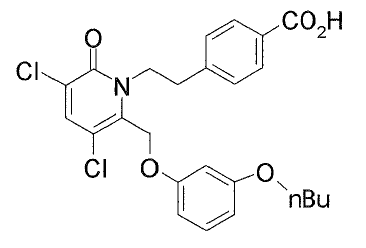 |
| 161 | 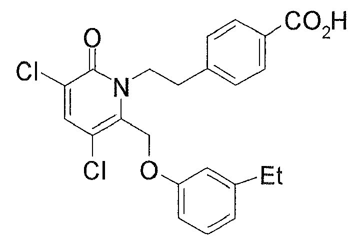 | | 162 | 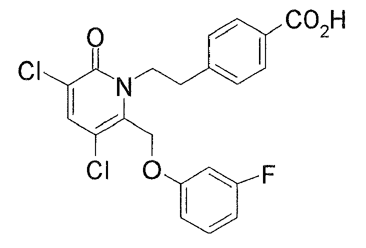 |

**[Table 40]**

| | | | | |
|---|---|---|---|---|
| 163 | 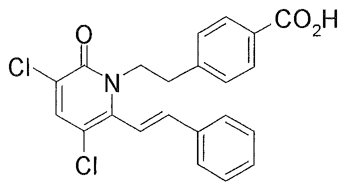 | | 164 | 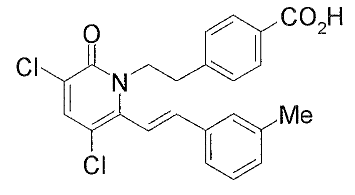 |
| 165 | 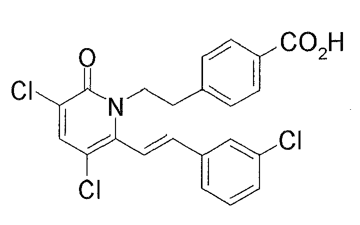 | | 166 | 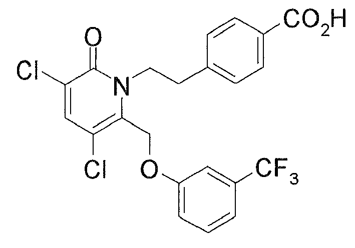 |
| 167 | 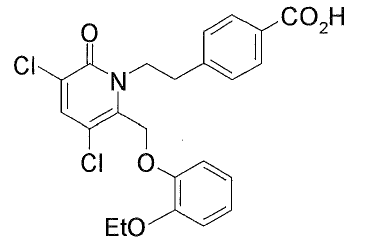 | | 168 | 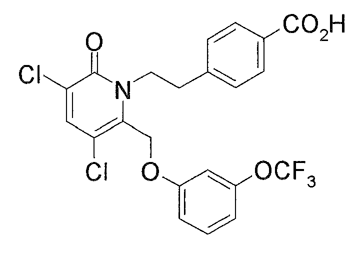 |
| 169 | 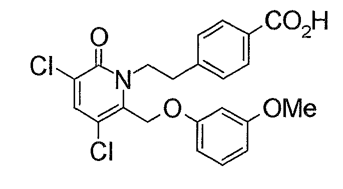 | | 170 | 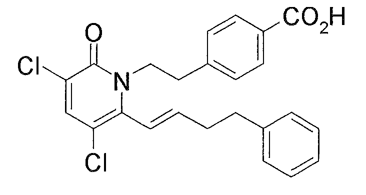 |
| 171 | 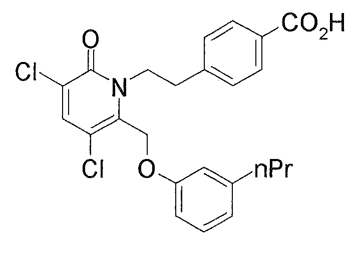 | | 172 | 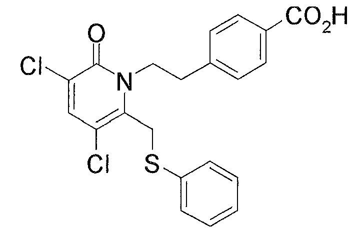 |

**[Table 41]**

| | | | | |
|---|---|---|---|---|
| 173 | 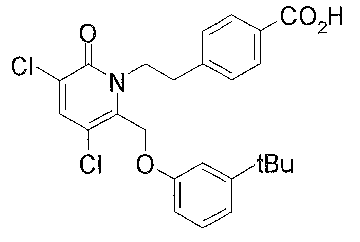 | | 174 | 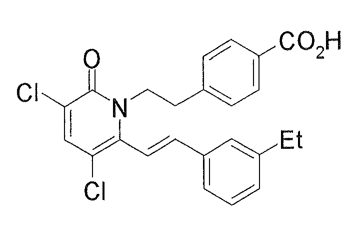 |
| 175 | 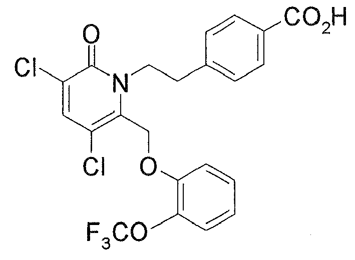 | | 176 | 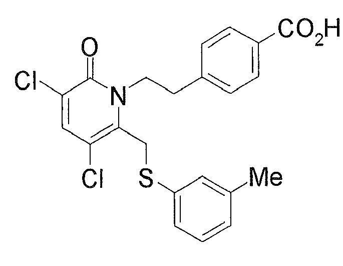 |
| 177 | 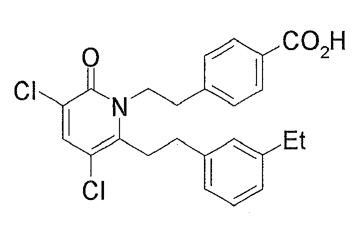 | | 178 | 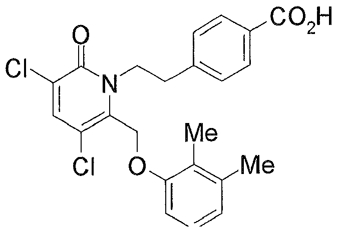 |
| 179 | 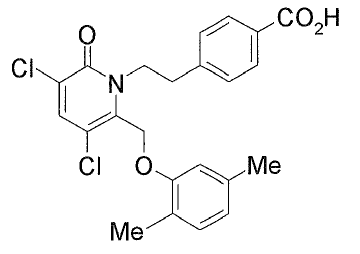 | | 180 | 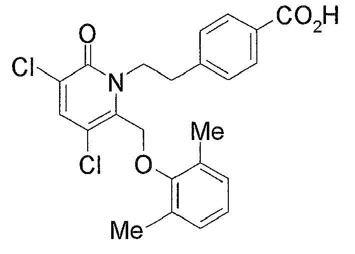 |

**[Table 42]**

| | | | | |
|---|---|---|---|---|
| 181 | 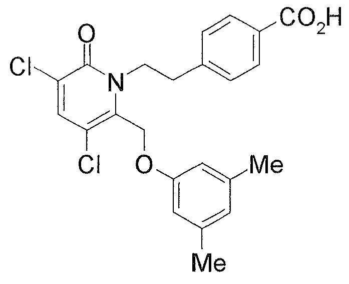 | | 182 | 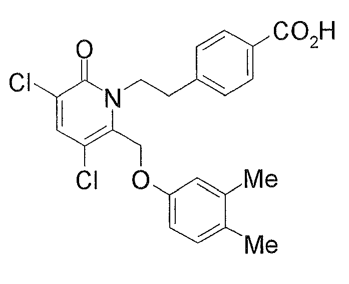 |
| 183 | 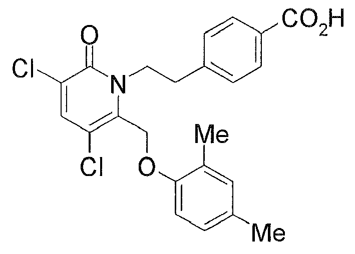 | | 184 | 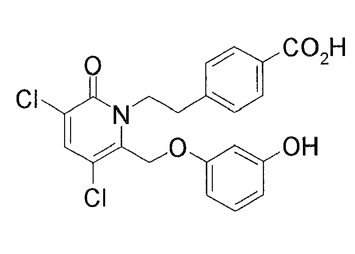 |
| 185 | 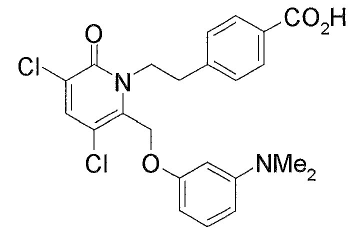 | | 186 | 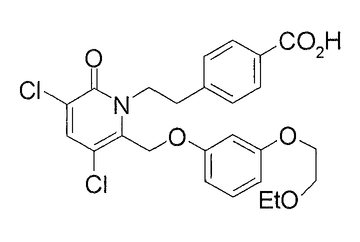 |
| 187 | 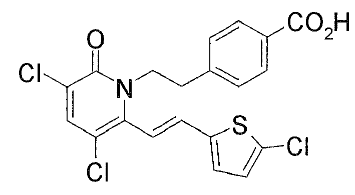 | | 188 | 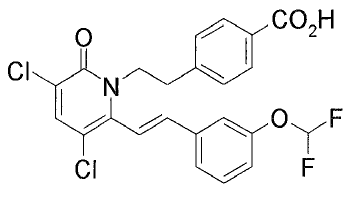 |
| 189 | 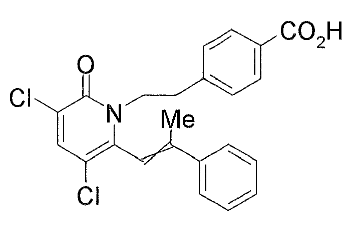 | | 190 | 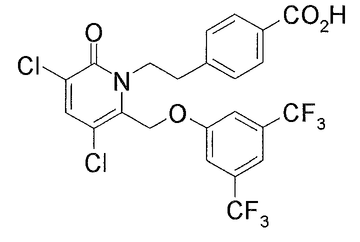 |

**[Table 43]**

| | | | | |
|---|---|---|---|---|
| 191 | 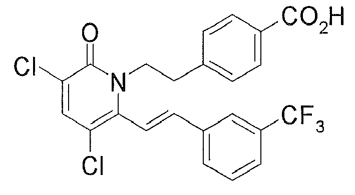 | | 192 | 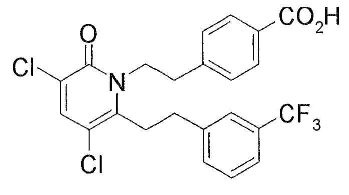 |
| 193 | 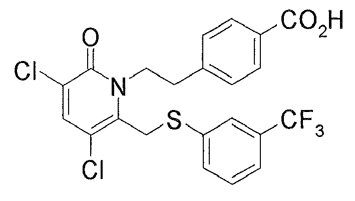 | | 194 | 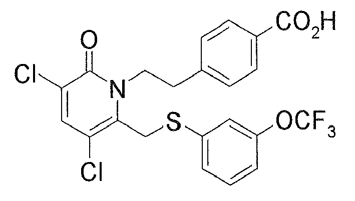 |
| 195 | 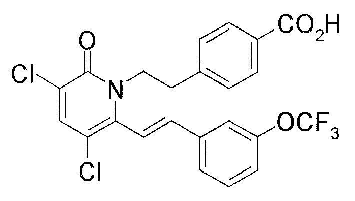 | | 196 | 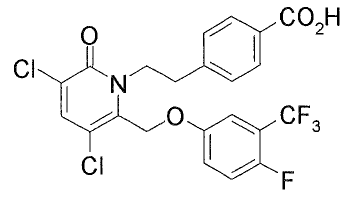 |
| 197 | 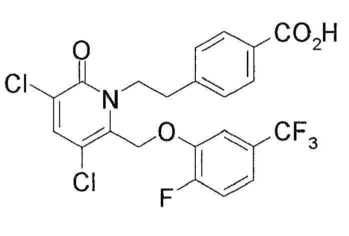 | | 198 | 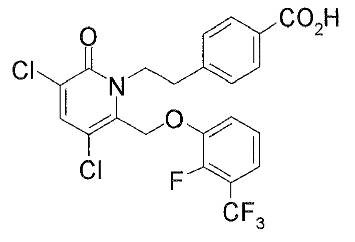 |
| 199 | 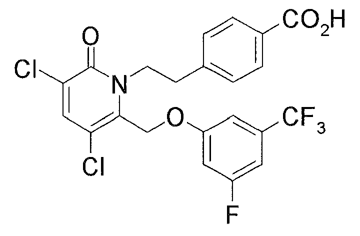 | | 200 | 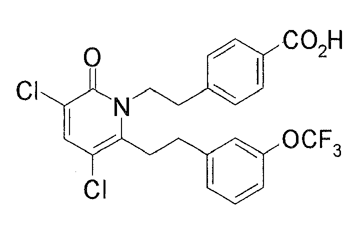 |

**[Table 44]**

| | | | | |
|---|---|---|---|---|
| 201 | 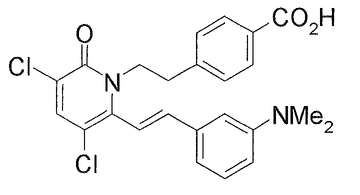 | | 202 | 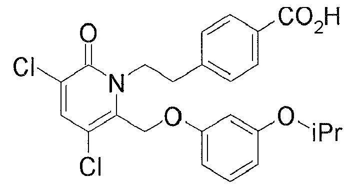 |
| 203 | 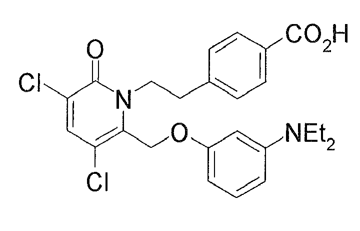 | | 204 | 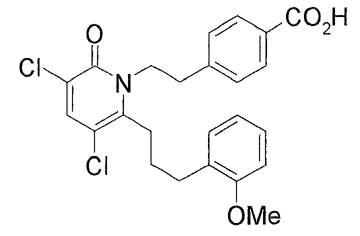 |
| 205 | 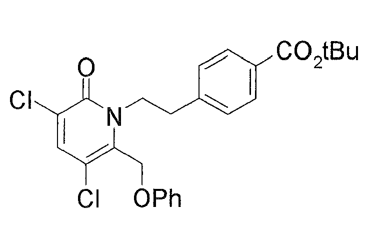 | | 206 | 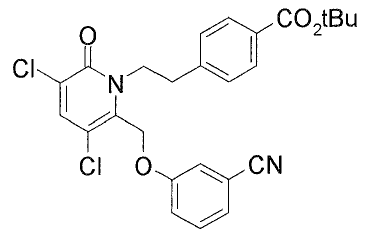 |
| 207 | 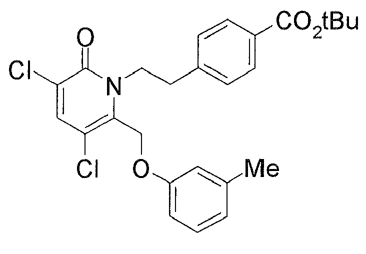 | | 208 | 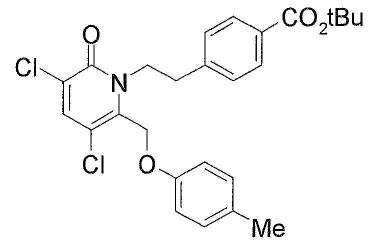 |
| 209 | 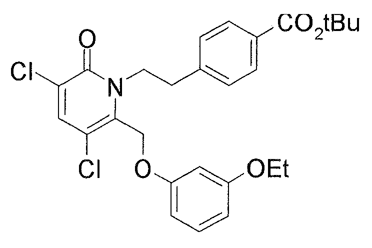 | | 210 | 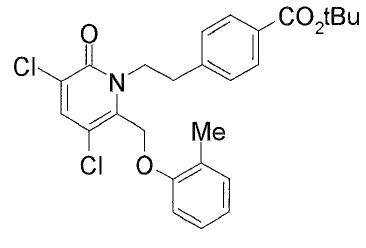 |

**[Table 45]**

| | | | | |
|---|---|---|---|---|
| 211 | 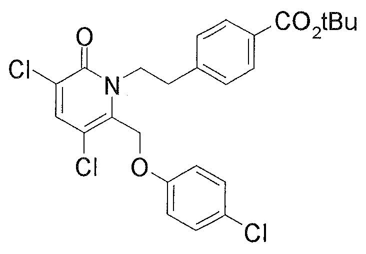 | | 212 | 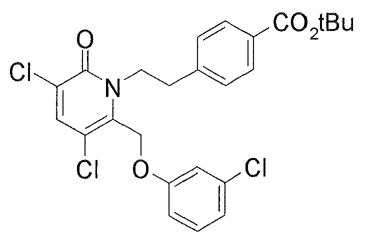 |
| 213 | 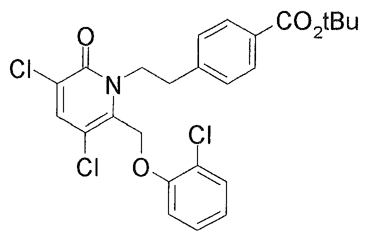 | | 214 | 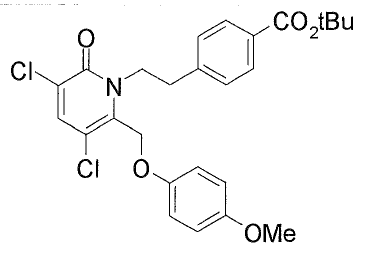 |
| 215 | 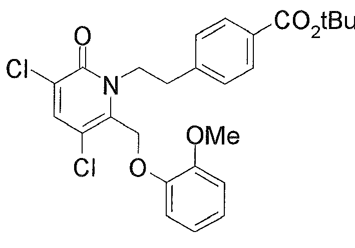 | | 216 | 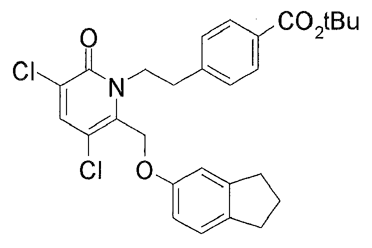 |
| 217 | 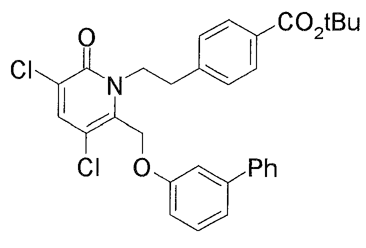 | | 218 | 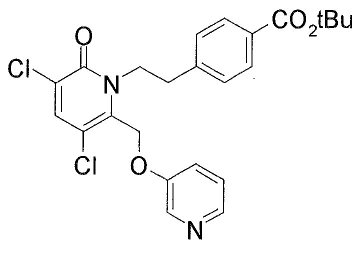 |

**[Table 46]**

| | | | | |
|---|---|---|---|---|
| 219 | 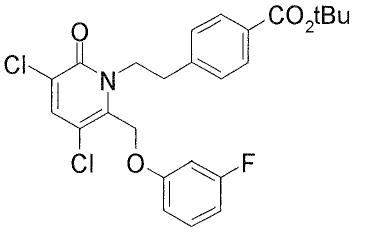 | | 220 | 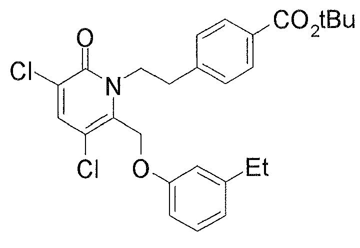 |
| 221 | 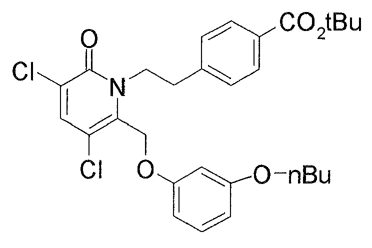 | | 222 | 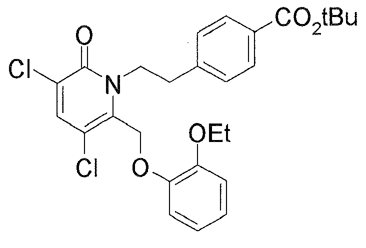 |
| 223 | 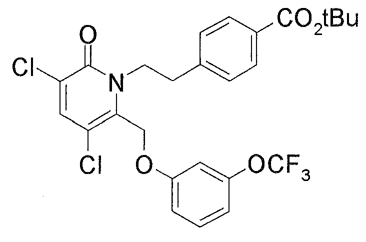 | | 224 | 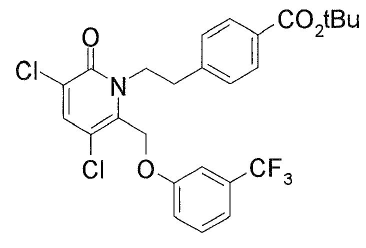 |
| 225 | 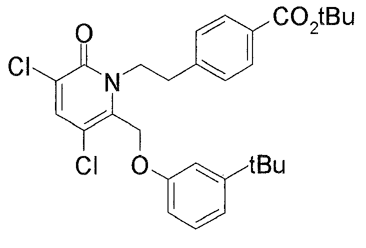 | | 226 | 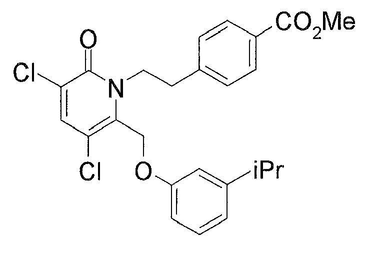 |
| 227 | 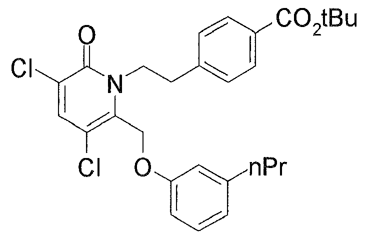 | | 228 | 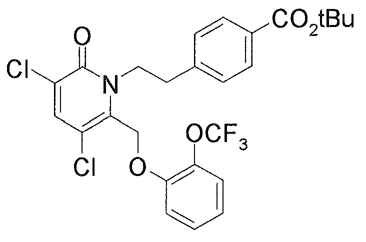 |

**[Table 47]**

| | | | | |
|---|---|---|---|---|
| 229 | 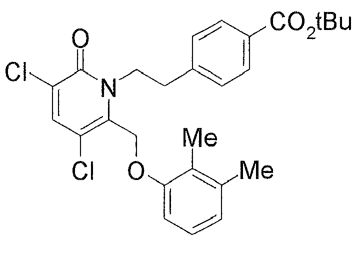 | | 230 | 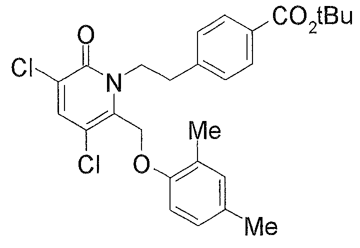 |
| 231 | 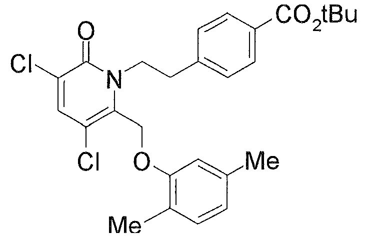 | | 232 | 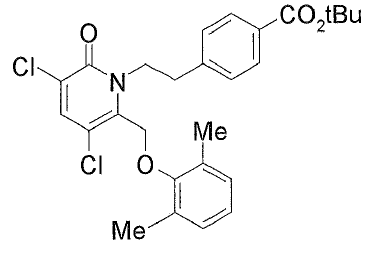 |
| 233 | 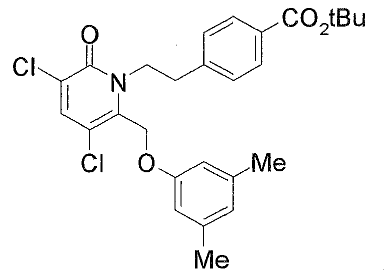 | | 234 | 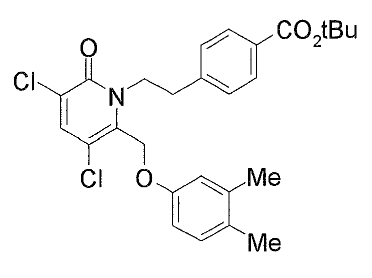 |
| 235 | 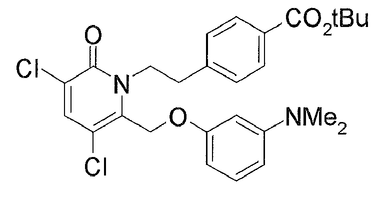 | | 236 | 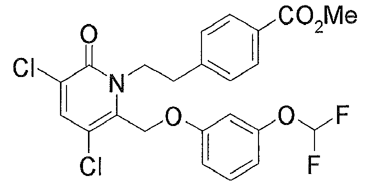 |
| 237 | 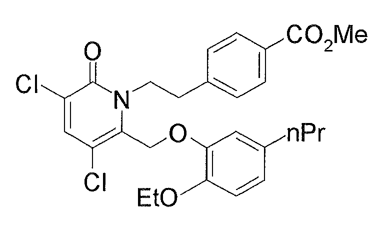 | | 238 | 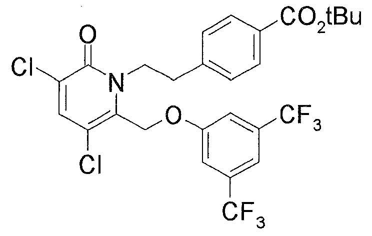 |

**[Table 48]**

| | | | | |
|---|---|---|---|---|
| 239 | 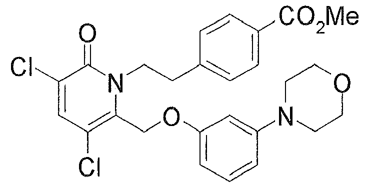 | | 240 | 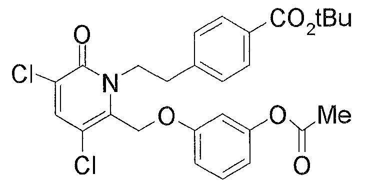 |
| 241 | 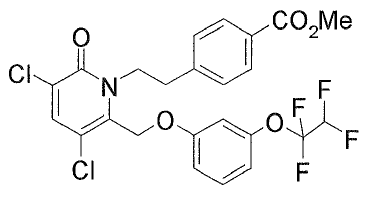 | | 242 | 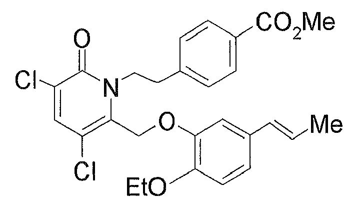 |
| 243 | 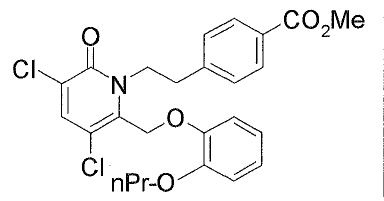 | | 244 | 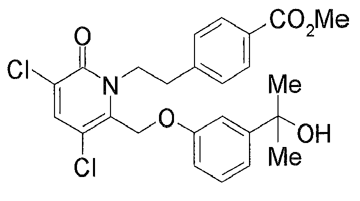 |
| 245 | 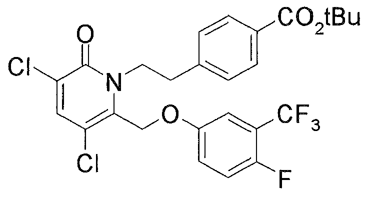 | | 246 | 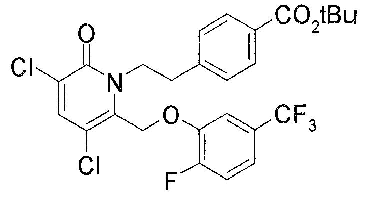 |
| 247 | 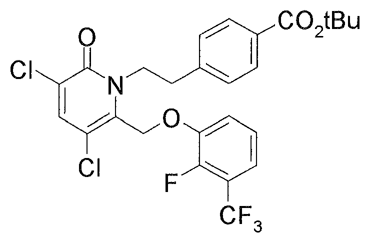 | | 248 | 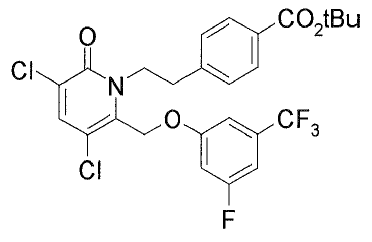 |

**[Table 49]**

| | | | | |
|---|---|---|---|---|
| 249 | 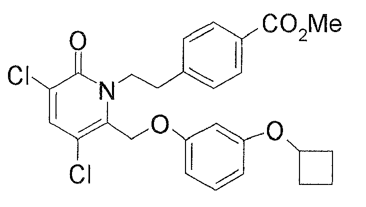 | | 250 | 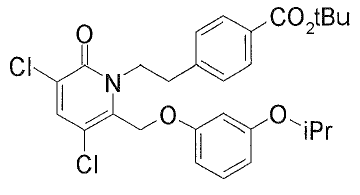 |
| 251 | 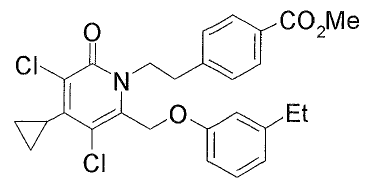 | | 252 | 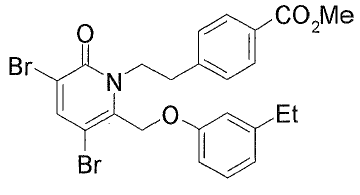 |
| 253 | 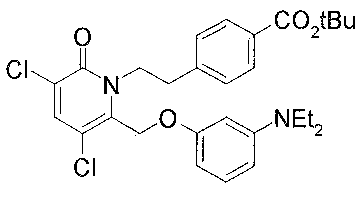 | | 254 | 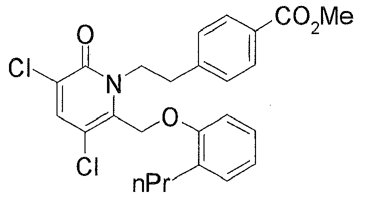 |
| 255 | 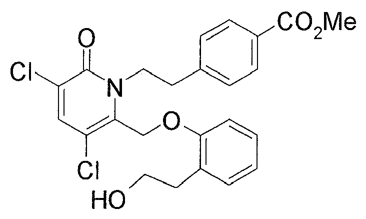 | | 256 | 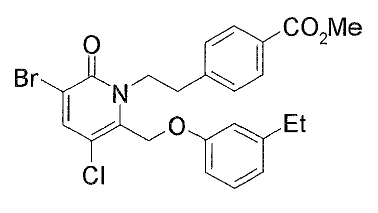 |
| 257 | 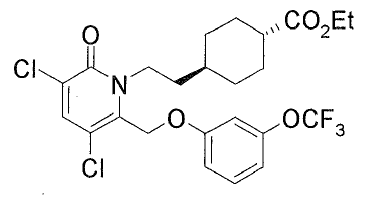 | | 258 | 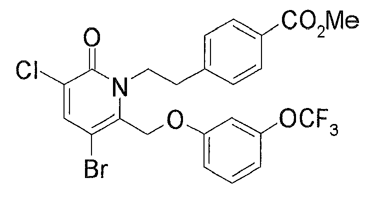 |
| 259 | 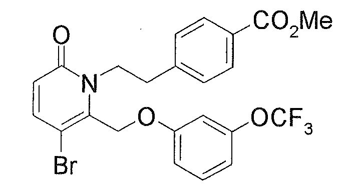 | | 260 | 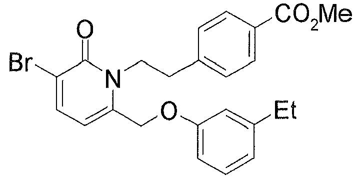 |

**[Table 50]**

| | | | | |
|---|---|---|---|---|
| 261 | 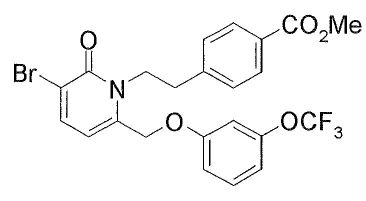 | | 262 | 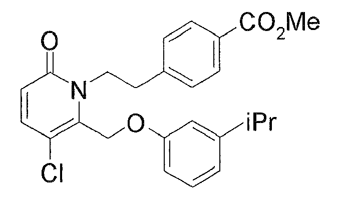 |
| 263 | 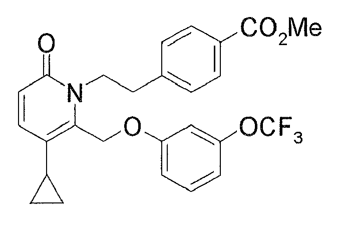 | | 264 | 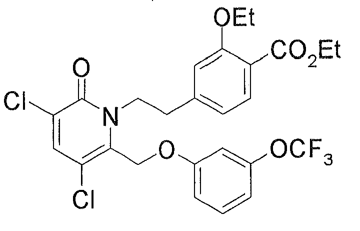 |
| 265 | 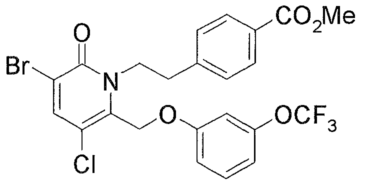 | | 266 | 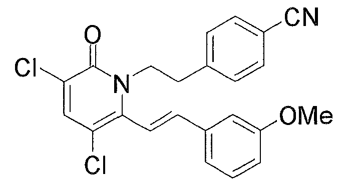 |
| 267 | 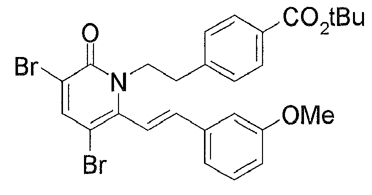 | | 268 | 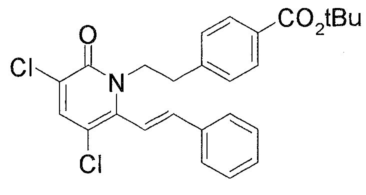 |
| 269 | 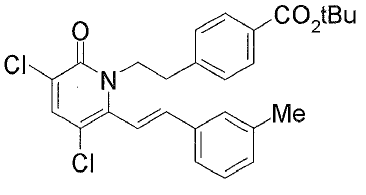 | | 270 | 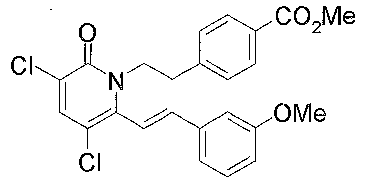 |

**[Table 51]**

| | | | | |
|---|---|---|---|---|
| 271 | 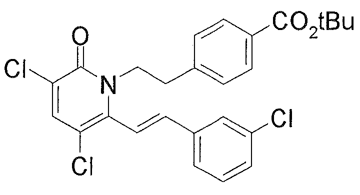 | | 272 | 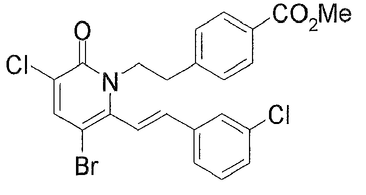 |
| 273 | 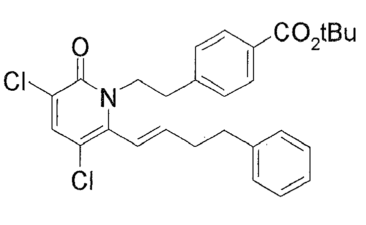 | | 274 | 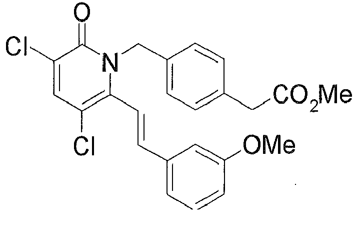 |
| 275 | 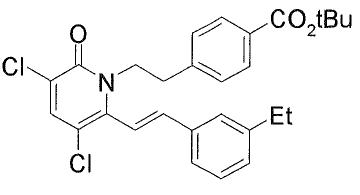 | | 276 | 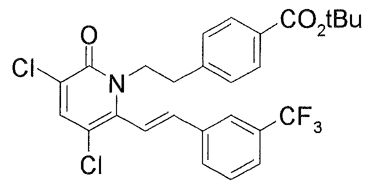 |
| 277 | 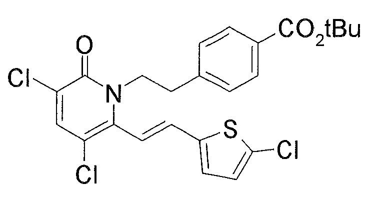 | | 278 | 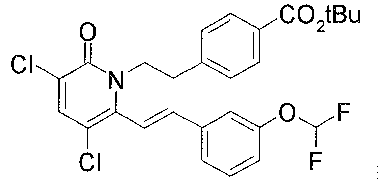 |
| 279 | 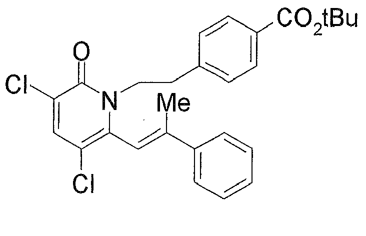 | | 280 | 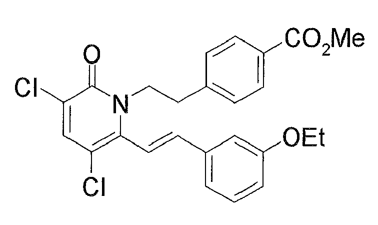 |

**[Table 52]**

| | | | | |
|---|---|---|---|---|
| 281 | 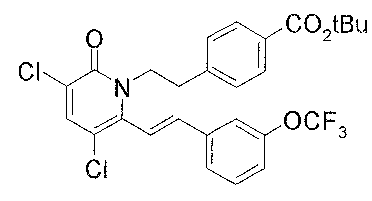 | | 282 | 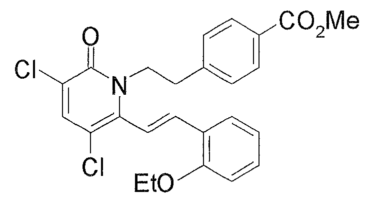 |
| 283 | 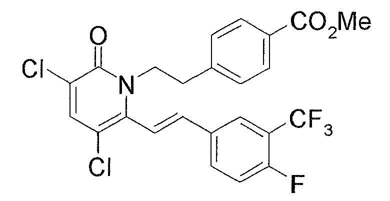 | | 284 | 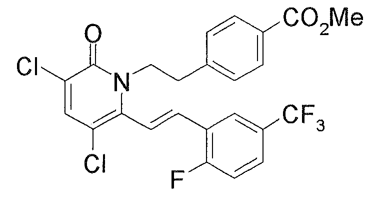 |
| 285 | 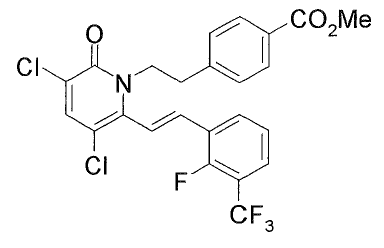 | | 286 | 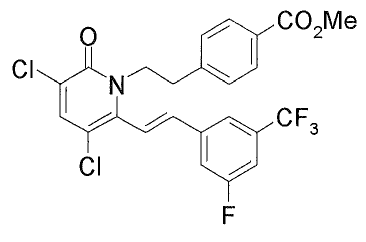 |
| 287 | 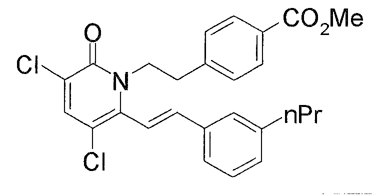 | | 288 | 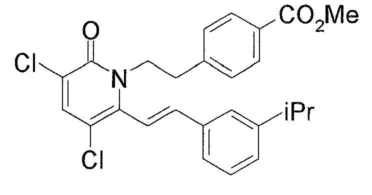 |
| 289 | 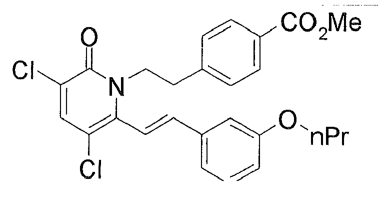 | | 290 | 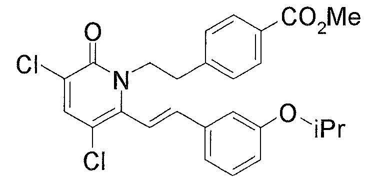 |

**[Table 53]**

| | | | | |
|---|---|---|---|---|
| 291 | 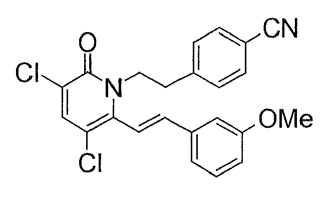 | | 292 | 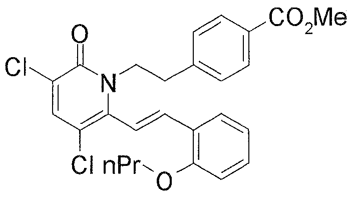 |
| 293 | 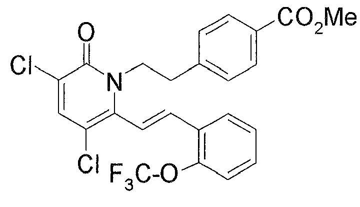 | | 294 | 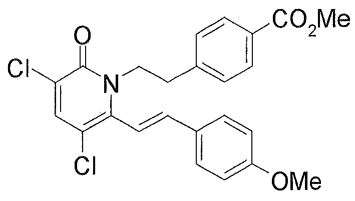 |
| 295 | 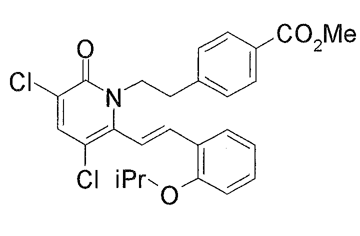 | | 296 | 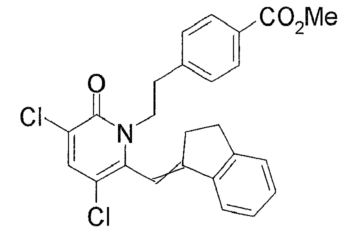 |
| 297 | 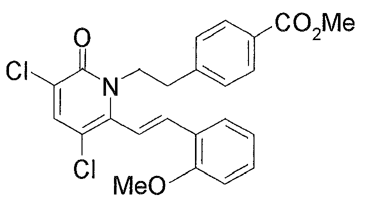 | | 298 | 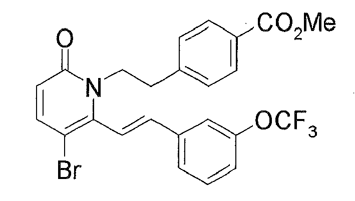 |
| 299 | 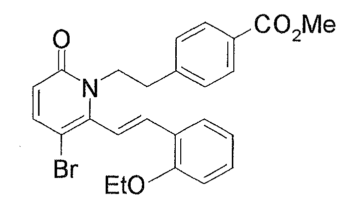 | | 300 | 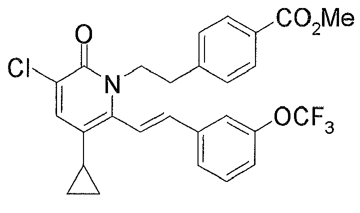 |

**[Table 54]**

| | | | | |
|---|---|---|---|---|
| 301 | 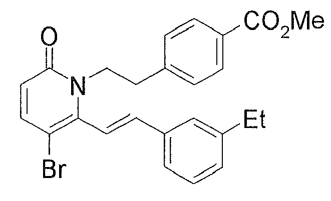 | | 302 | 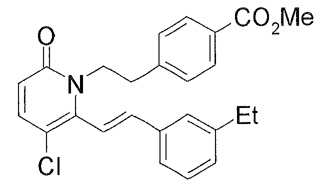 |
| 303 | 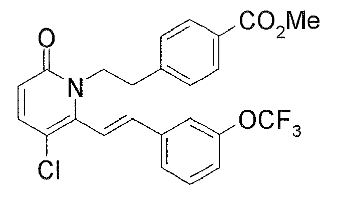 | | 304 | 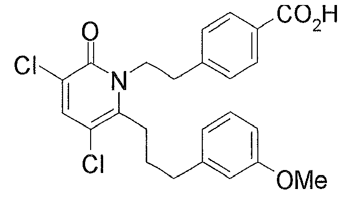 |
| 305 | 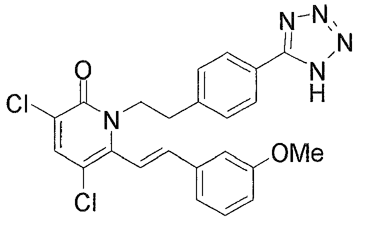 | | 306 | 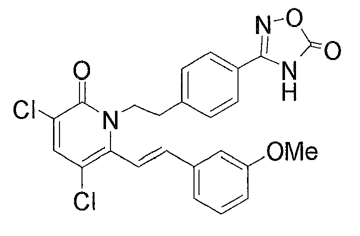 |
| 307 | 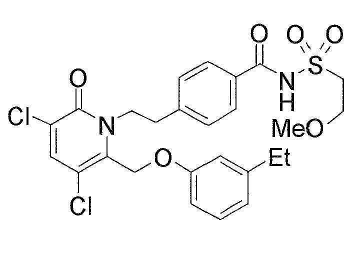 | | 308 | 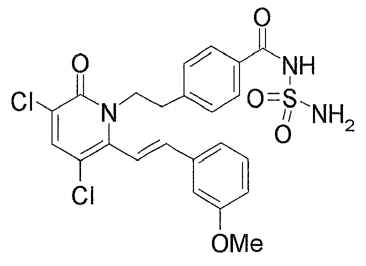 |
| 309 | 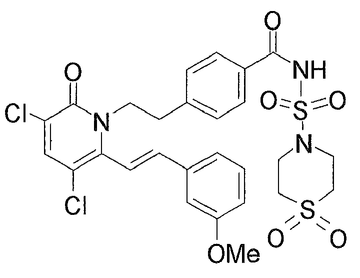 | | 310 | 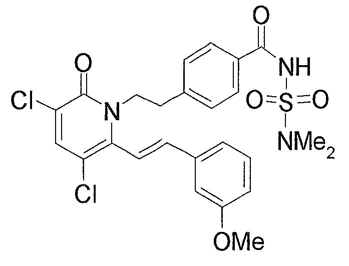 |

**[Table 55]**

| | | | | |
|---|---|---|---|---|
| 311 | 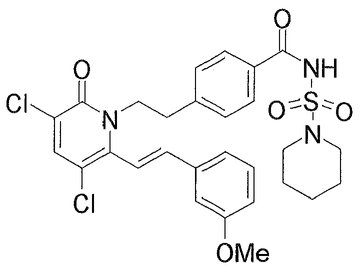 | | 312 | 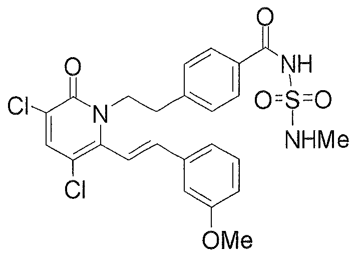 |
| 313 | 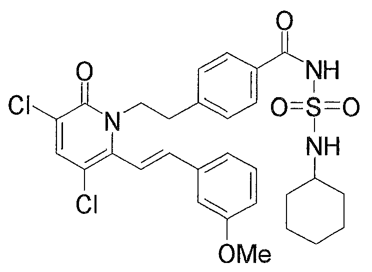 | | 314 | 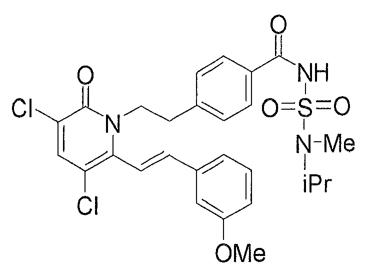 |
| 315 | 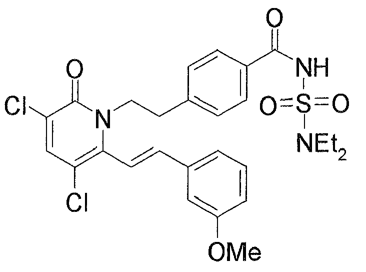 | | 316 | 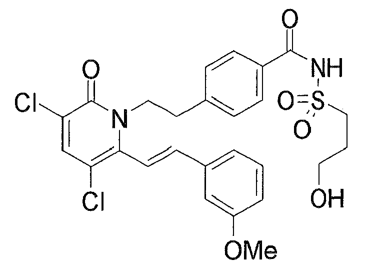 |
| 317 | 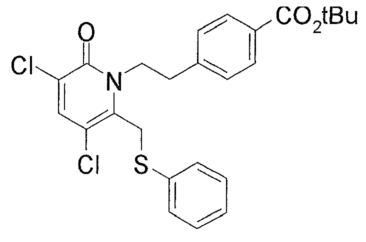 | | 318 | 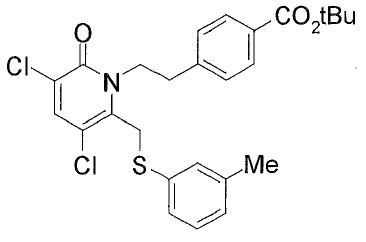 |

**[Table 56]**

| | | | | |
|---|---|---|---|---|
| 319 | 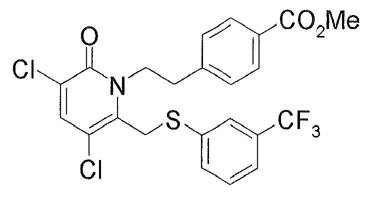 | | 320 | 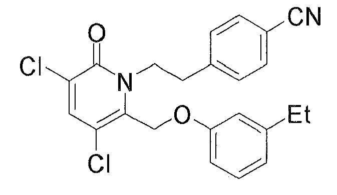 |
| 321 | 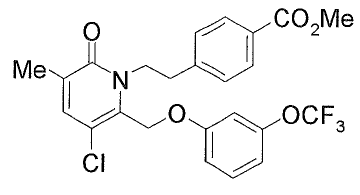 | | 322 | 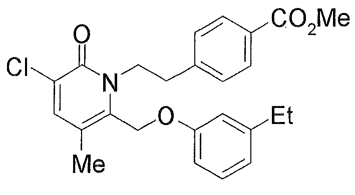 |
| 323 | 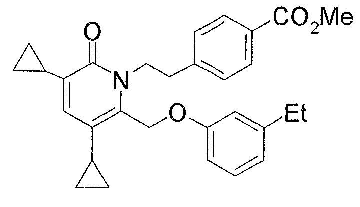 | | 324 | 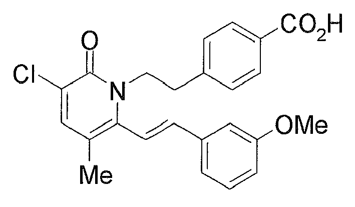 |
| 325 | 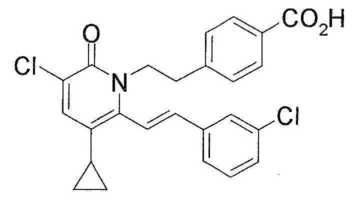 | | 326 | 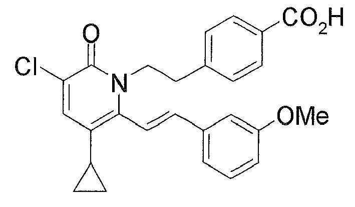 |
| 327 | 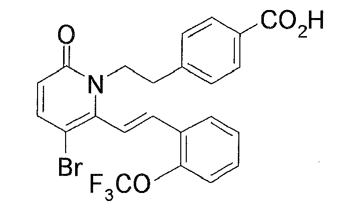 | | 328 | 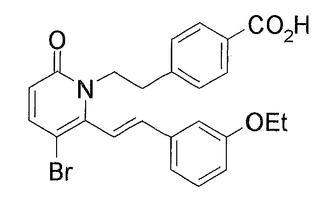 |

**[Table 57]**

| | | | | |
|---|---|---|---|---|
| 329 | 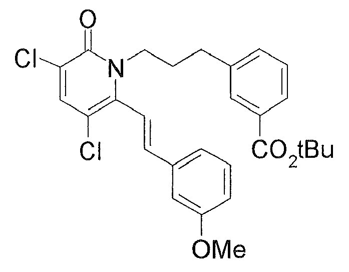 | | 330 | 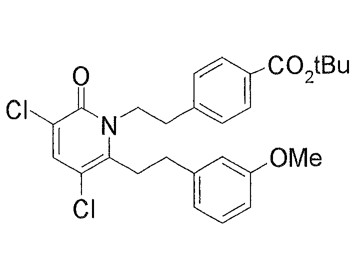 |
| 331 | 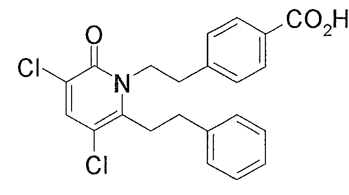 | | 332 | 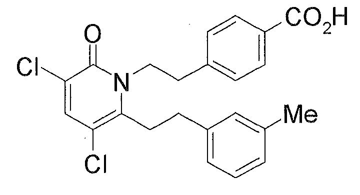 |
| 333 | 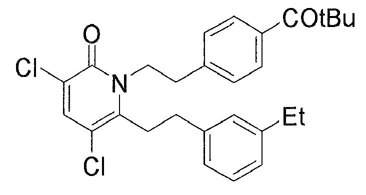 | | 334 | 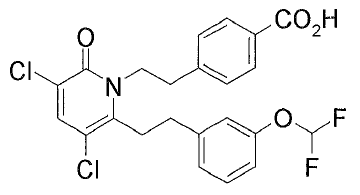 |
| 335 | 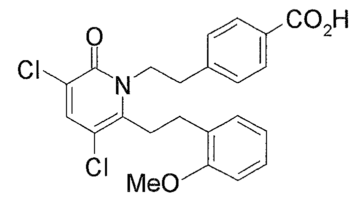 | | 336 | 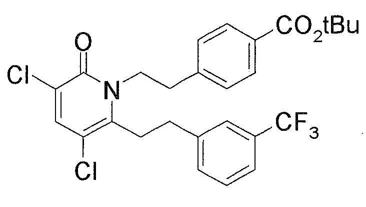 |
| 337 | 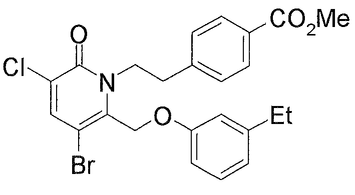 | | 338 | 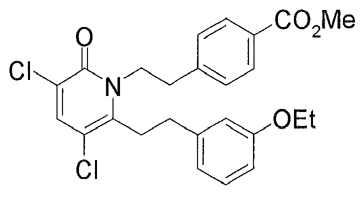 |

**[Table 58]**

| | | | | |
|---|---|---|---|---|
| 339 | 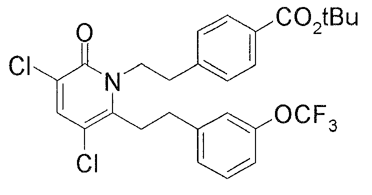 | | 340 | 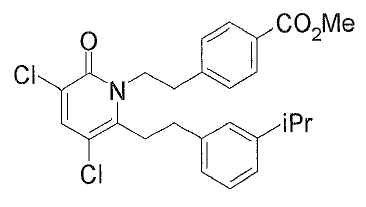 |
| 341 | 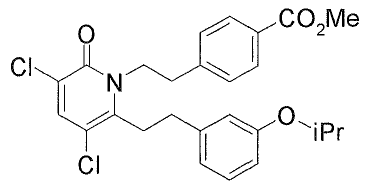 | | 342 | 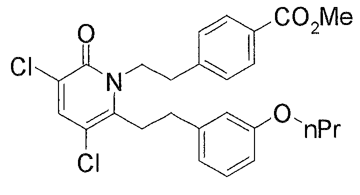 |
| 343 | 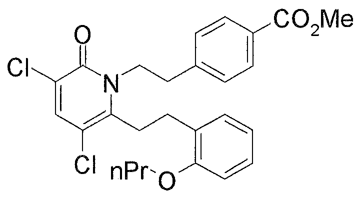 | | 344 | 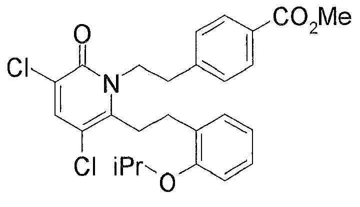 |
| 345 | 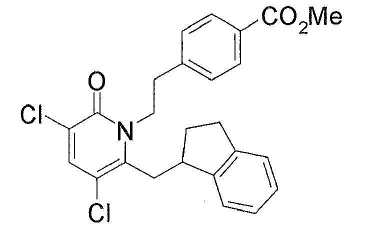 | | 346 | 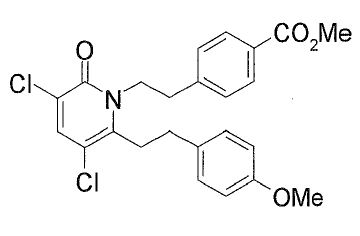 |
| 347 | 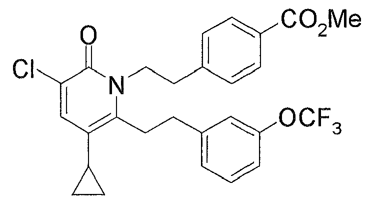 | | 348 | 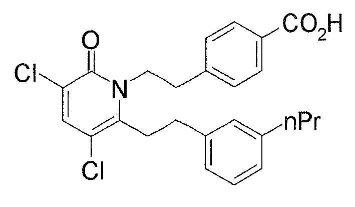 |

**[Table 59]**

| | | | | |
|---|---|---|---|---|
| 349 | 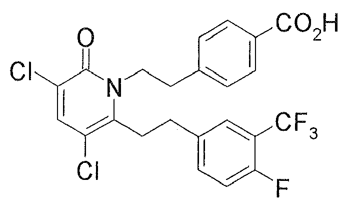 | | 350 | 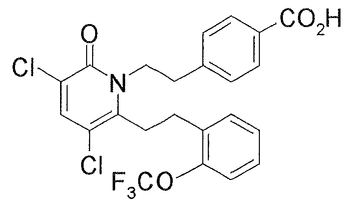 |
| 351 | 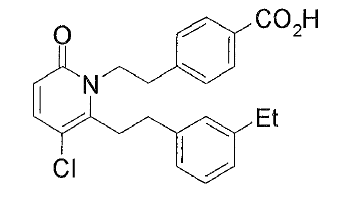 | | 352 | 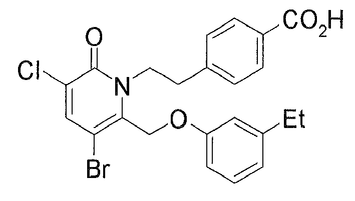 |
| 353 | 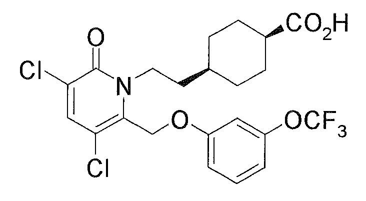 | | 354 | 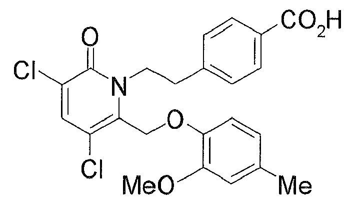 |
| 355 | 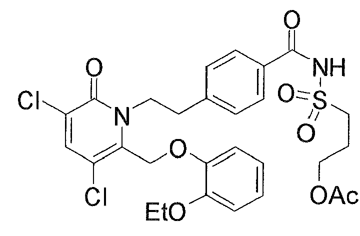 | | 356 | 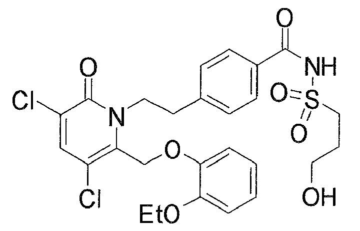 |
| 357 | 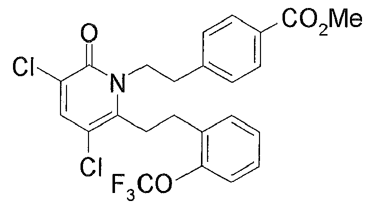 | | | |

**[Table 60]**

| Ex | Syn | Data |
|---|---|---|
| 27 | 27 | ESI+: 504, 506, 508 |
| 28 | 28 | ESI+: 522, 524 |
| 29 | 29 | ESI+: 486, 488 |
| 30 | 30 | ESI+: 516, 518, 520 |
| 31 | 31 | ESI-: 428, 430, 432 |
| 32 | 32 | FAB+: 470,472 |
| 33 | 33 | ESI+: 486, 488 |
| 34 | 34 | FAB-:500 |
| 35 | 35 | FAB+: 506 |
| 36 | 36 | ESI+: 609, 611 |
| 37 | 37 | ESI+: 532, 525, 526 |
| 38 | 38 | FAB+: 445 |
| 39 | 39 | FAB+: 607 |
| 40 | 40 | FAB+: 567 |
| 41 | 41 | FAB+: 553 |
| 42 | 42 | FAB+: 531 |
| 43 | 43 | FAB+: 503 |
| 44 | 44 | ESI-: 470, 472, 473 |
| 45 | 45 | FAB+: 502 |
| 46 | 46 | FAB+: 536 |
| 47 | 47 | ESI+: 532, 534 |
| 48 | 48 | ESI+: 532, 534, 536 |
| 49 | 49 | ESI+: 474, 476, 478 |
| 50 | 50 | ESI+: 562, 565 |
| 51 | 51 | FAB+: 478 |
| 52 | 52 | ESI+: 484, 486, 487 |
| 53 | 53 | ESI+: 440, 442, 443 |
| 54 | 54 | ESI+: 522, 524 |
| 55 | 55 | ESI+: 508, 510 |
| 56 | 56 | ESI+: 560, 562 |
| 57 | 57 | ESI+: 466 |
| 58 | 58 | ESI+: 406 |
| 59 | 59 | ESI+: 490, 492, 494 |

**[Table 61]**

| | | |
|---|---|---|
| 61 | 61 | NMR(400MHz): 1.26 (6H, d, J = 6.8 Hz), 2.88-3.04 (3H, m), 4.19-4.29 (2H, m), 6.41 (1H, d, J = 9.6 Hz), 6.77 (1H, d, J = 16.4 Hz), 6.85 (1H, d, J = 16.4 Hz), 7.23 (2H, d, J = 8.0 Hz), 7.28, (1H, d, J = 7.6 Hz), 7.35 (1H, dd, J = 7.6, 7.6 Hz), 7.40 (1H, d, J = 7.6 Hz), 7.42-7.46 (1H, m), 7.67 (1H, d, J = 9.6 Hz), 7.80 (2H, d, J = 8.0 Hz), 12.90 (1H, s); ESI+: 466 |
| 62 | 62 | ESI+: 472 |
| 63 | 63 | ESI+: 513, 515 |
| 64 | 64 | ESI+: 474, 476, 478 |
| 65 | 65 | FAB-: 500 |
| 66 | 66 | FAB+: 490 |
| 67 | 67 | ESI+: 485, 487 |
| 68 | 68 | ESI+: 490, 492 |
| 69 | 69 | ESI-: 470, 472 |
| 70 | 70 | ESI+: 443, 446 |
| 71 | 68 | ESI+: 492, 494 |
| 72 | 68 | ESI+: 488, 490 |
| 73 | 2 | ESI+: 476, 478, 480 |
| 74 | 5 | NMR(400MHz): 1.11 (6H, d, J = 6.0 Hz), 3.07-3.19 (2H, m), 4.32-4.42 (2H, m), 4.50 (1H, septet, J = 6.0 Hz), 5.20 (2H, s), 6.87-6.94 (1H, m), 6.98-7.05 (2H, m), 7.14 (1H, d, J = 7.6 Hz), 7.30 (2H, d, J = 8.0 Hz), 7.84 (2H, d, J = 8.0 Hz), 8.05 (1H, s), 12.87 (1H, s); ESI+: 476, 478; ESI+: 476, 478 |
| 75 | 1 | ESI+:492, 494, 496, 498 |
| 76 | 1 | FAB+: 444 |
| 77 | 1 | FAB+: 458 |
| 78 | 5 | ESI+: 458, 460 |
| 79 | 1 | ESI+: 484, 486 |
| 80 | 1 | ESI+: 444, 446 |
| 81 | 1 | ESI+: 504, 506 |
| 82 | 1 | ESI-: 532, 534 |
| 83 | 1 | ESI-: 501, 503 |
| 84 | 1 | ESI+: 502, 504 |
| 85 | 1 | ESI+: 476, 478 |
| 86 | 1 | ESI-: 474, 476 |
| 87 | 1 | ESI+: 460 |

**[Table 62]**

| | | |
|---|---|---|
| 88 | 1 | NMR(400MHz): 1.36 (3H, t, J = 6.8Hz), 2.93-3.09 (2H,m), 4.08 (2H, q, J = 6.8 Hz), 4.23-4.37 (2H, m), 6.83 (1H, d, J = 16.4 Hz), 6.89 (1H, d, J = 16.4 Hz), 6.96 (1H, dd, J = 2.0, 8.0 Hz), 7.13 (1H, d, J = 8.0 Hz), 7.16-7.19 (1H, m), 7.24 (2H, d, J = 8.0 Hz), 7.33 (1H, dd, J = 8.0, 8.0 Hz), 7.82 (2H, d, J = 8.0 Hz), 8.06 (1H, s), 12.89 (1H, s); ESI+: 458, 460, 462 |
| 89 | 1 | ESI-: 456, 458 |
| 90 | 1 | NMR(400MHz): 1.12 (3H, t, J = 6.8 Hz), 2.75-2.84 (2H, m), 2.88-3.03 (4H, m), 3.91 (2H, q, J = 6.8 Hz), 4.21-4.28 (2H, m), 6.86 (1H, dd, J = 7.6, 7.6 Hz), 6.94 (1H, d, J = 7.6 Hz), 7.13 (2H, dd, J = 1.6, 7.6 Hz), 7.17-7.23 (1H, m), 7.33 (2H, d, J = 8.0 Hz), 7.89 (2H, d, J = 8.0 Hz), 7.98 (1H, s), 12.92 (1H, s); ESI+: 460, 462, 464 |
| 91 | 1 | NMR(400MHz): 1.25 (6H, d, J = 6.8 Hz), 2.87-2.99 (1H, m), 3.03 (2H, t, J = 7.2 Hz), 4.30 (2H, t, J = 7.2 Hz), 6.82 (1H, d, J = 16.0 Hz), 6.86 (1H, d, J = 16.0 Hz), 7.25 (2H, d, J = 8.0 Hz), 7.28 (1H, ddd, J = 1.6, 1.6, 7.6 Hz), 7.35 (1H, dd, J = 7.6, 7.6 Hz), 7.40 (1H, ddd, J = 1.6, 1.6, 7.6 Hz), 7.43-7.46 (1H, m), 7.81 (2H, d, J = 8.0 Hz), 8.06 (1H, s), 12.89 (1H, s); ESI+: 456, 458, 460 |
| 92 | 1 | ESI+: 500, 502 |
| 93 | 1 | ESI+: 500, 502 |
| 94 | 1 | ESI+: 500, 502 |
| 95 | 1 | ESI+: 500, 502 |
| 96 | 1 | ESI+: 456, 458, 460 |
| 97 | 1 | FAB+: 488 |
| 98 | 1 | ESI+: 472, 474, 476 |
| 99 | 1 | NMR(300MHz): 1.01 (3H, t, J = 7.2 Hz), 1.68-1.84 (2H, m), 2.96-3.07 (2H, m), 3.99 (2H, t, J = 6.3 Hz), 4.24-4.37 (2H, m), 6.84 (1H, d, J = 16.5 Hz), 6.91 (1H, d, J = 16.5 Hz), 6.97 (1H, dd, J = 1.8, 7.8 Hz), 7.13 (1H, d, J = 7.8 Hz), 7.16-7.21 (1H, m), 7.25 (2H, d, J = 8.1 Hz), 7.33 (1H, dd, J = 7.8, 7.8 Hz), 7.81 (2H, d, J = 8.1 Hz), 8.07 (1H, s), 12.90 (1H, s); ESI+: 472, 474, 476 |
| 100 | 1 | ESI+:474,476 |
| 101 | 1 | FAB+: 474 |
| 102 | 1 | ESI+: 498, 500 |
| 103 | 1 | ESI+: 472, 474 |
| 104 | 1 | ESI+: 472, 474, 476 |
| 105 | 1 | ESI+: 474, 476 |
| 106 | 1 | NMR(400 MHz): 3.00 (2H, t, J = 7.2 Hz), 4.32 (2H, t, J = 7.2 Hz), 6.98 (1H, d, J = 16.4 Hz), 7.03 (1H, d, J = 16.4 Hz), 7.22 (2H, d, J = 8.0 Hz), 7.42-7.47 (1H, m), 7.49-7.60 (2H, m), 7.78 (2H, d, J = 8.0 Hz), 7.96 (1H, dd, J = 2.0, 7.6 Hz), 8.07 (1H, s), 12.84 (1H, s); ESI+: 498, 500 |
| 107 | 1 | FAB+: 474 |

**[Table 63]**

| | | |
|---|---|---|
| 109 | 1 | ESI+: 474, 476, 478 |
| 110 | 1 | ESI+: 440, 442 |
| 111 | 1 | ESI+: 442, 444, 446 |
| 112 | 1 | ESI+: 446, 448 |
| 113 | 1 | ESI+: 494, 496 |
| 114 | 1 | ESI+: 458 |
| 115 | 1 | FAB+: 426 |
| 116 | 1 | ESI+: 474 |
| 117 | 1 | ESI+: 508, 510 |
| 118 | 1 | NMR(400 MHz): 2.94-3.03 (2H, m), 4.19-4.30 (2H, m), 6.43 (1H, d, J = 9.6 Hz), 6.89 (1H, d, J = 16.4 Hz), 7.07 (1H, d, J = 16.4 Hz), 7.22 (2H, d, J = 8.0 Hz), 7.40 (1H, d, J = 7.6 Hz), 7.55-7.72 (4H, m), 7.79 (2H, d, J = 8.0 Hz), 12.87 (1H, s); ESI-: 506, 508 |
| 119 | 1 | APCI-: 466, 488 |
| 120 | 1 | NMR(400 MHz): 0.55-0.63 (2H, m), 0.74-0.83 (2H, m), 1.80-1.90 (1H, m), 3.00 (2H, t, J = 7.6 Hz), 4.28 (2H, t, J = 7.6 Hz), 6.92 (1H, d, J = 16.4 Hz), 7.13 (1H, d, J = 16.4 Hz), 7.24 (2H, d, J = 8.0 Hz), 7.38 (1H, d, J = 8.0 Hz), 7.47 (1H, s), 7.57 (1H, dd, J = 8.0, 8.0 Hz), 7.64 (1H, d, J = 8.0 Hz), 7.67-7.71 (1H, m), 7.80 (2H, d, J = 8.0 Hz), 12.87 (1H, s); ESI+: 504, 506 |
| 121 | 1 | ESI+: 546, 548 |
| 122 | 1 | NMR(400 MHz): 0.49-0.56 (2H, ,m), 0.74-0.83 (2H, m), 1.57-1.67 (1H, m), 2.87-3.06 (6H, m), 4.21-4.32 (2H, m), 7.19-7.29 (3H, m), 7.32 (2H, d, J = 8.0 Hz), 7.40-7.48 (2H, m), 7.87 (2H, d, J = 8.0 Hz), 12.90 (1H, s); ESI+: 506, 508 |
| 123 | 1 | ESI+: 482, 484 |
| 124 | 1 | NMR(300 MHz): 1.23 (3H, t, J = 7.5 Hz), 2.65 (2H, q, J = 7.5 Hz), 2.94-3.03 (2H, m), 4.17-4.32 (2H, m), 6.41 (1H, d, J = 9.6 Hz), 6.80 (1H, d, J = 16.5 Hz), 6.83 (1H, d, J = 16.5 Hz), 7.17-7.28 (3H, m), 7.43-7.44 (3H, m), 7.67 (1H, d, J = 9.6 Hz), 7.80 (2H, d, J = 8.1 Hz), 12.90 (1H, s); ESI+: 452, 454 |
| 125 | 1 | NMR(400 MHz): 1.23 (3H, t, J = 7.6 Hz), 2.66 (2H, q, J = 7.6 Hz), 2.95-3.04 (2H, m), 4.18-4.29 (2H, m), 6.47 (1H, d, J = 9.6 Hz), 6.77-6.88 (2H), 7.20-7.27 (3H, m), 7.31-7.44 (3H, m), 7.58 (1H, d, J = 9.6 Hz), 7.81 (2H, d, J = 8.0 Hz), 12.90 (1H, s); ESI+: 408, 410 |
| 126 | 1 | NMR(400 MHz): 2.94-3.03 (2H, m), 4.19-4.29 (2H, m), 6.49 (1H, d, J = 9.6 Hz), 6.93 (1H, d, J = 16.4 Hz), 7.08 (1H, d, J = 16.4 Hz), 7.23 (2H, d, J = 8.0 Hz), 7.40 (1H, d, J = 7.6 Hz), 7.55-7.66 (3H, m), 7.67-7.71 (1H, m), 7.79 (2H, d, J = 8.0 Hz), 12.88 (1H, s); ESI+: 464, 466 |
| 127 | 1 | ESI+: 508, 510, 511 |
| 128 | 2 | FAB+: 536 |
| 129 | 2 | ESI+: 490, 492, 494 |
| 130 | 2 | ESI+: 460, 462 |

**[Table 64]**

| | | |
|---|---|---|
| 131 | 2 | ESI+: 47, 472, 473 |
| 132 | 2 | ESI+: 490, 492 |
| 133 | 2 | ESI+: 508, 510, 512 |
| 134 | 2 | ESI+: 460, 462 |
| 135 | 2 | ESI-: 460, 462 |
| 136 | 2 | ESI+: 426, 428 |
| 137 | 2 | ESI+: 508, 510, 512 |
| 138 | 2 | ESI+: 426, 428 |
| 139 | 2 | NMR(400 MHz): 0.58-0.66 (2H, m), 0.79-0.89 (2H, m), 1.18 (3H, t, J = 7.6Hz), 1.82-1.95 (1H, m), 2.60 (2H, q, J = 7.6Hz), 3.00-3.12 (2H, m), 4.13-4.25 (2H, m), 5.24 (2H, s), 6.86-6.96 (3H, m), 7.19-7.30 (3H, m), 7.60(1H, s), 7.80 (2H, d, J = 8.0 Hz), 12.88 (1H, s); ESI+ 452, 454 |
| 140 | 2 | APCI-: 510, 512 |
| 141 | 2 | ESI+: 456, 458 |
| 142 | 2 | ESI-: 510 |
| 143 | 2 | FAB-: 544 |
| 144 | 2 | ESI+: 546, 548 |
| 145 | 5 | FAB+: 418 |
| 146 | 5 | ESI+: 446, 448, 450 |
| 147 | 5 | FAB+: 443 |
| 148 | 5 | ESI+: 462, 464, 466 |
| 149 | 5 | ESI+: 432, 434, 436 |
| 150 | 5 | ESI+: 432, 434 |
| 151 | 5 | ESI+: 432, 434, 436 |
| 152 | 5 | FAB+: 452 |
| 153 | 5 | FAB+: 452 |
| 154 | 5 | FAB+: 452 |
| 155 | 5 | ESI+: 448, 450 |
| 156 | 5 | ESI+: 448, 450 |
| 157 | 5 | FAB+: 458 |
| 158 | 5 | ESI+: 494, 496 |
| 159 | 5 | ESI+:419, 421 |
| 160 | 5 | FAB+: 490 |
| 161 | 1 | NMR(400 MHz): 1.17 (3H, t, J = 7.6Hz), 2.59 (2H, q, J = 7.6Hz), 3.02-3.12 (2H, m), 4.20-4.30 (2H, m), 5.20 (2H, s), 6.87-6.95 (3H, m), 7.22-7.29 (3H, m), 7.82 (2H, d, J = 8.0 Hz), 8.10 (1H, s), 12.88 (1H, s); FAB+: 446 |
| 162 | 5 | FAB+: 436 |

**[Table 65]**

| | | |
|---|---|---|
| 161 | 1 | NMR(400 MHz): 1.17 (3H, t, J = 7.6Hz), 2.59 (2H, q, J = 7.6Hz), 3.02-3.12 (2H, m), 4.20-4.30 (2H, m), 5.20 (2H, s), 6.87-6.95 (3H, m), 7.22-7.29 (3H, m), 7.82 (2H, d, J = 8.0 Hz), 8.10 (1H, s), 12.88 (1H, s); FAB+: 446 |
| 162 | 5 | FAB+: 436 |
| 163 | 5 | ESI+: 414 |
| 164 | 5 | ESI-: 426, 428 |
| 165 | 5 | ESI-: 446, 448, 450 |
| 166 | 5 | ESI+: 486, 488 |
| 167 | 5 | NMR(400 MHz): 1.1 (3H, t, J = 6.8 Hz), 3.08-3.19 (2H, m), 3.94 (2H, q, J = 6.8 Hz), 4.28-4.40 (2H, m), 5.22 (2H, s), 6.87-6.95 (1H, m), 6.90-7.01 (2H, m), 7.14 (1H, d, J = 7.6 Hz), 7.29 (2H, d, J = 8.0 Hz), 7.84 (2H, d, J = 8.0 Hz), 8.06 (1H, s), 12.87 (1H, s); ESI+: 462, 464 |
| 168 | 5 | ESI+: 502, 504 |
| 169 | 1 | NMR(400 MHz): 3.01-3.11 (2H, m),3.75 (3H, s), 4.22-4.30 (2H, m), 5.21 (2H, s), 6.60-6.70 (3H, m), 7.24-7.31 (3H, m), 7.83 (2H, d, J = 8.4 Hz), 8.10 (1H, s), 12.87 (1H, s); ESI+: 448, 450, 452 |
| 170 | 5 | ESI-: 440, 442, 444 |
| 171 | 1 | NMR(400 MHz): 0.88 (3H, t, J = 7.2Hz), 1.58 (2H, qt, J = 7.2, 7.2 Hz), 2.44-2.60 (2H, m), 3.03-3.11 (2H, m), 4.16-4.34 (2H, m), 5.20 (2H, s), 6.85-6.93 (3H, m), 7.21-7.29 (3H, m), 7.81 (2H, d, J = 8.0 Hz), 8.10 (1H, s), 12.87 (1H, s); ESI+: 460, 462, 464 |
| 172 | 5 | ESI+: 434 |
| 173 | 5 | ESI-: 472, 474, 476 |
| 174 | 5 | NMR(400 MHz): 1.23 (3H, t, J = 7.2 Hz), 2.65 (2H, q, J = 7.2 Hz), 2.97-3.07 (2H,m), 4.25-4.35 (2H, m), 6.79 (1H, d, J = 16.4 Hz), 6.84 (1H, d, J = 16.4 Hz), 7.21-7.28 (3H, m), 7.35 (1H, dd, J = 7.6, 7.6 Hz), 7.34-7.42 (2H, m), 7.82 (2H, d, J = 8.0 Hz), 8.07 (1H, s), 12.91 (1H, s); ESI+: 442, 444, 446 |
| 175 | 5 | ESI+: 502 |
| 176 | 5 | ESI-: 446, 448 |
| 177 | 5 | ESI+: 444, 446 |
| 178 | 5 | ESI+: 446, 448 |
| 179 | 5 | ESI+: 446, 448 |
| 180 | 5 | ESI+: 446, 448 |
| 181 | 5 | ESI+: 446, 448 |
| 182 | 5 | ESI+: 446, 448 |
| 183 | 5 | FAB+: 446 |
| 184 | 5 | ESI+: 434, 436 |
| 185 | 5 | FAB+: 461 |

**[Table 66]**

| | | |
|---|---|---|
| 190 | 5 | FAB+:554 |
| 191 | 5 | FAB+: 482, 484, 486 |
| 192 | 5 | FAB+: 484, 486, 488 |
| 193 | 1 | ESI-: 500, 502 |
| 194 | 2 | NMR(400 MHz): 3.02-3.12 (2H, m), 4.26-4.33 (2H, m), 4.36 (2H, s), 7.31-7.39 (3H, m), 7.47-7.52 (3H, m), 7.87 (2H, d, J = 8.0Hz), 7.93 (1H, s), 12.92 (1H, s); ESI+: 518, 520, 522 |
| 195 | 5 | NMR(400 MHz): 2.96-3.06 (2H,m), 4.26-4.34 (2H, m), 6.94 (1H, d, J = 16.4 Hz), 7.06 (1H, d, J = 16.4 Hz), 7.24 (2H, d, J = 8.0 Hz), 7.41 (1H, d, J = 8.0 Hz), 7.58 (1H, dd, J = 8.0, 8.0 Hz), 7.64 (1H, d, J = 8.0 Hz), 7.68-7.72 (1H, m), 7.79 (2H, d, J = 8.0 Hz), 8.08 (1H, s), 12.88 (1H, s);ESI+: 498, 500, 502 |
| 196 | 5 | FAB+: 504 |
| 197 | 5 | FAB+: 504 |
| 198 | 5 | FAB+: 504 |
| 199 | 5 | FAB+: 504 |
| 200 | 5 | NMR(400 MHz): 2.86-3.04 (6H, m), 4.16-4.28 (2H, m), 7.20-7.28 (3H, m), 7.36 (2H, d, J = 8.0Hz), 7.40-7.41 (1H, m), 7.88 (2H, d, J = 8.0 Hz), 7.96 (1H, s), 12.90 (1H, s); ESI+: 500, 502, 504 |
| 201 | 5 | ESI+: 457, 459, 461 |
| 202 | 5 | ESI+: 476, 478 |
| 203 | 5 | ESI+: 489, 491 |
| 204 | 5 | ESI-: 458, 460 |
| 205 | 6 | ESI+: 474 |
| 206 | 6 | ESI+: 499 |
| 207 | 6 | ESI+: 488 |
| 208 | 6 | ESI+: 488 |
| 209 | 6 | ESI+: 518 |
| 210 | 6 | ESI+: 488, 490, 492 |
| 211 | 6 | ESI+: 508, 510 |
| 212 | 6 | ESI+: 508, 510 |
| 213 | 6 | ESI+: 508, 510 |
| 214 | 6 | ESI+: 504 |
| 215 | 6 | ESI+: 504 |
| 216 | 6 | ESI+: 514, 516, 518 |
| 217 | 6 | ESI+: 550 |
| 218 | 6 | ESI+: 475 |
| 219 | 6 | ESI+: 492 |

**[Table 67]**

| | | |
|---|---|---|
| 220 | 6 | ESI+: 502 |
| 221 | 6 | ESI+: 546 |
| 222 | 6 | ESI+: 518 |
| 223 | 6 | ESI+: 558 |
| 224 | 6 | ESI+:542 |
| 225 | 6 | ESI+: 530 |
| 226 | 6 | ESI+: 474, 476, 478 |
| 227 | 6 | ESI+: 516 |
| 228 | 6 | ESI+:558 |
| 229 | 6 | ESI+: 502, 504, 506 |
| 230 | 6 | ESI+: 502, 504, 506 |
| 231 | 6 | ESI+: 502 |
| 232 | 6 | ESI+ 502, 504, 506 |
| 233 | 6 | ESI+: 502, 504, 506 |
| 234 | 6 | ESI+: 502 |
| 235 | 6 | ESI+: 517, 519, 521 |
| 236 | 6 | ESI+: 498, 500 |
| 237 | 6 | ESI+: 518, 520, 522 |
| 238 | 6 | ESI+: 610 |
| 239 | 6 | ESI+: 517 |
| 240 | 6 | FAB+: 532 |
| 241 | 6 | ESI+: 548, 550, 552 |
| 242 | 6 | ESI+: 516, 518, 520 |
| 243 | 6 | ESI+:490 |
| 244 | 6 | ESI-: 488, 490 |
| 245 | 6 | FAB-: 558 |
| 246 | 6 | FAB-: 558 |
| 247 | 6 | FAB+: 560 |
| 248 | 6 | FAB+: 560 |
| 249 | 6 | APCI+: 502 |
| 250 | 6 | ESI+: 532, 534 |
| 251 | 6 | ESI+: 500, 502, 503, 505 |
| 252 | 6 | ESI+: 548, 550, 552 |
| 253 | 6 | ESI+: 545 |
| 254 | 6 | ESI+: 474, 476, 478 |

**[Table 68]**

| | | |
|---|---|---|
| 255 | 6 | ESI+: 476, 478, 480 |
| 256 | 6 | ESI+: 504, 506, 508 |
| 257 | 6 | ESI+: 536, 538 |
| 258 | 6 | ESI+: 560, 562, 564 |
| 259 | 6 | ESI+: 526, 528 |
| 260 | 6 | APCI+: 470, 472 |
| 261 | 6 | ESI-: 524, 526 |
| 262 | 6 | ESI+: 440, 442 |
| 263 | 6 | APCI/ESI+: 488 |
| 264 | 6 | FAB+:574 |
| 265 | 6 | ESI+: 560, 562 |
| 266 | 7 | ESI+: 425, 427 |
| 267 | 7 | ESI+: 588, 590, 592 |
| 268 | 7 | ESI+: 470, 472 |
| 269 | 7 | ESI+: 484, 486, 488 |
| 270 | 7 | FAB: 458, 460, 462 |
| 271 | 7 | ESI+: 504, 506, 508 |
| 272 | 7 | FAB+:508 |
| 273 | 7 | ESI+: 498, 500 |
| 274 | 7 | ESI+: 458 |
| 275 | 7 | ESI+: 498, 500, 502 |
| 276 | 7 | FAB+: 538, 540, 542 |
| 277 | 7 | ESI+: 510, 512, 514 |
| 278 | 7 | ESI+: 536, 538, 539 |
| 279 | 7 | ESI+: 484, 486 |
| 280 | 7 | ESI+: 472, 474, 476 |
| 281 | 7 | FAB+: 554, 556, 558 |
| 282 | 7 | ESI+: 472, 474, 476 |
| 283 | 7 | FAB+: 514 |
| 284 | 7 | FAB+: 514 |
| 285 | 7 | ESI+: 514, 516 |
| 286 | 7 | ESI+: 514, 516 |
| 287 | 7 | ESI+: 470, 472, 474 |
| 288 | 7 | ESI+: 470, 472, 474 |
| 289 | 7 | ESI+: 486, 488, 490 |

**[Table 69]**

| | | |
|---|---|---|
| 290 | 7 | ESI+: 486, 488, 490 |
| 291 | 7 | FAB+: 515 |
| 292 | 7 | ESI+: 486, 488 |
| 293 | 7 | APCI+: 512, 514 |
| 294 | 7 | APCI+: 458, 460, 462 |
| 295 | 7 | ESI+: 486, 488, 490 |
| 296 | 7 | ESI+: 454, 456 |
| 297 | 7 | ESI+: 458, 460, 462 |
| 298 | 7 | ESI+: 522, 524 |
| 299 | 7 | ESI+: 482, 484 |
| 300 | 7 | ESI+: 518, 520 |
| 301 | 7 | ESI+: 466, 468 |
| 302 | 7 | ESI+: 422, 424 |
| 303 | 7 | ESI+: 478, 500 |
| 304 | 31 | FAB+: 460 |
| 305 | 32 | ESI+: 468, 470 |
| 306 | 33 | ESI+: 484, 486 |
| 307 | 36 | ESI-: 565, 567, 569 |
| 308 | 36 | FAB+: 522 |
| 309 | 36 | ESI+: 640, 642 |
| 310 | 36 | FAB+:550 |
| 311 | 36 | FAB+: 590 |
| 312 | 36 | FAB+: 536 |
| 313 | 36 | FAB+:604 |
| 314 | 36 | FAB+:578 |
| 315 | 36 | FAB+:578 |
| 316 | 40 | FAB+: 565 |
| 317 | 48 | ESI+: 434,436,438 (-tBu) |
| 318 | 48 | ESI+: 504 |
| 319 | 48 | ESI+: 516 |
| 320 | 49 | ESI+: 427, 429, 431 |
| 321 | 54 | ESI+: 496, 498, 499 |
| 322 | 57 | ESI+: 440, 442 |
| 323 | 57 | ESI+: 472 |

**[Table 70]**

| | | |
|---|---|---|
| 324 | 61 | ESI+: 424, 426 |
| 325 | 61 | ESI+: 454, 456 |
| 326 | 61 | ESI+: 450 |
| 327 | 61 | ESI+: 508 |
| 328 | 61 | ESI+: 468 |
| 329 | 62 | ESI+: 514 |
| 330 | 64 | ESI+: 502, 504, 506 |
| 331 | 64 | ESI+: 416, 418 |
| 332 | 64 | ESI+: 430, 432 |
| 333 | 64 | ESI+: 500, 502, 504 |
| 334 | 64 | FAB+: 482 |
| 335 | 64 | FAB+: 446 |
| 336 | 64 | ESI+: 540, 542, 544 |
| 337 | 6 | FAB+: 504, 506 |
| 338 | 64 | ESI+: 474 |
| 339 | 64 | ESI+: 556, 558, 560 |
| 340 | 64 | APCI+: 472, 474 |
| 341 | 64 | ESI+: 488, 490, 492 |
| 342 | 64 | ESI+: 488 |
| 343 | 64 | ESI+: 488, 490, 492 |
| 344 | 64 | APCI+: 488, 490, 492 |
| 345 | 64 | APCI+: 456 |
| 346 | 64 | ESI+: 460, 462, 464 |
| 347 | 64 | ESI+: 520, 522 |
| 348 | 65 | ESI+: 458, 460 |
| 349 | 65 | FAB+: 502 |
| 350 | 1 | NMR(400 MHz): 2.86-3.04 (6H, ,m), 4.20-4.28 (2H, m), 7.30-7.45 (6H, m), 7.89 (2H, d, J = 8.0 Hz), 7.97 (1H, s), 12.90 (1H, s); ESI+: 500, 502, 504 |
| 351 | 65 | ESI+: 410, 412 |
| 352 | 68 | ESI-: 488, 490, 492 |
| 353 | 68 | ESI+: 508, 510 |
| 354 | 68 | FAB+: 462 |
| 355 | 36 | ESI+: 625, 627 |
| 356 | 40 | ESI+: 583, 585 |
| 357 | 64 | ESI+: 514, 516, 518 |

### Industrial Availability

Since the compound of the present invention has an EP4 receptor agonistic action, it is useful as an agent for preventing and/or treating peripheral arterial occlusive disease and the like.

## Claims

1. A compound of the formula (I) or a pharmaceutically acceptable salt thereof: 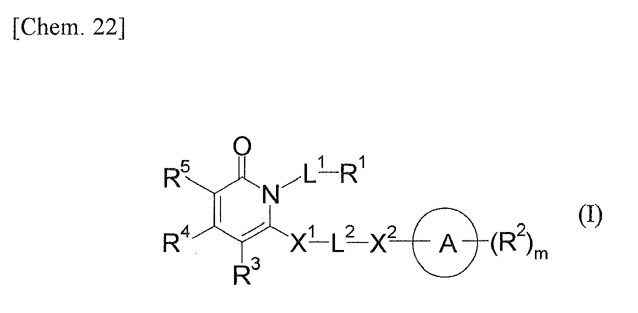 [wherein
Ring A represents aryl or heteroaryl,
X¹, and X² are the same as or different from each other, and represent a single bond, -O-, or -S-,
L¹ represents lower alkylene which may be substituted,
L² represents lower alkylene or lower alkenylene, which may be each substituted,
R¹ represents R⁶ or a group represented by the following formula (II): 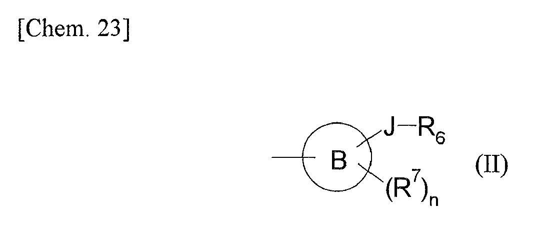 Ring B represents aryl or heteroaryl,
R⁶ represents -CO₂R⁰, -CN, -C(O)-N(R⁰)-S(O)₂-R⁸, -C(O)-N(R⁰)-S(O)₂-N(R⁰)-R⁸, -N(R⁰)-C(O)-N(R⁰)-S(O)₂-R⁸, -C(O)-N(R⁰)-R⁸, or a group represented by the following formula (III) or (IV): 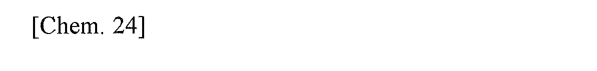 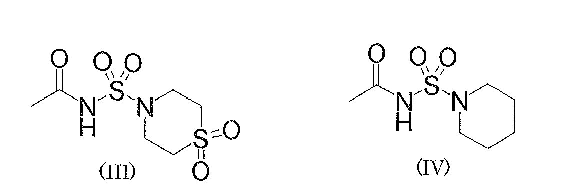 or a group represented by any one of the following formulae (V) to (XIV): 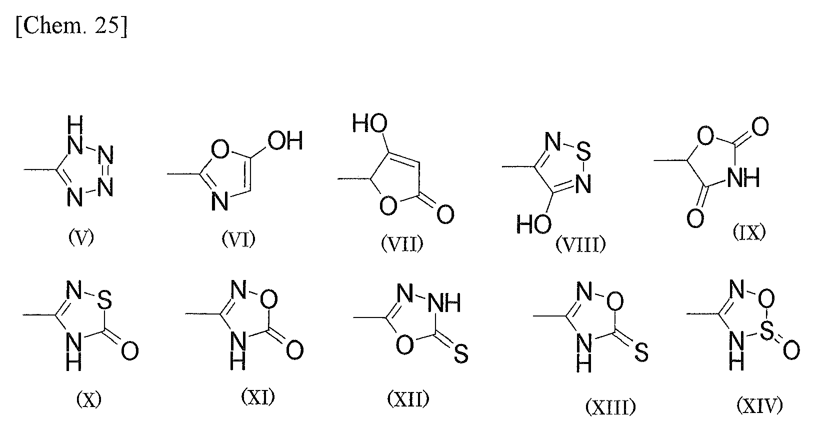 R⁰ are the same as or different from each other, and represent H or lower alkyl,
R⁸ represents H, lower alkyl, halogeno-lower alkyl, cycloalkyl, -(lower alkylene)-OR⁰, -(lower alkylene)-O-C(O)-R⁰, or -(lower alkylene)-CO₂R⁰,
J represents a single bond, lower alkylene, or lower alkenylene,
R² and R⁷ are the same as or different from each other, and represent lower alkyl, halogen, cyano, nitro, halogeno-lower alkyl, -OR⁰, -O-(halogeno-lower alkyl), -O-(cycloalkyl), -O-(lower alkylene)-OR⁰, -N(R⁰)₂, morpholyl, -(lower alkylene)-OR⁰, - (lower alkenylene)-R⁰, or -O-C(O)-R⁰,
m and n are the same as or different from each other, and represent an integer of 0 to 3,
R³, R⁴, and R⁵ are the same as or different from each other, and represent H, halogen, -CN, lower alkyl, lower alkenyl, cycloalkyl, halogeno-lower alkyl, -OR⁰, -O-halogeno-lower alkyl, -CO₂R⁰, -S(O)₂R⁰, or -C(O)N(R⁰)₂,
provided that methyl {6-[(3-methylphenoxy)methyl]-2-oxopyridin-1(2H)-yl}acetate is excluded].

2. The compound or a pharmaceutically acceptable salt thereof as described in claim 1, wherein Ring A is phenyl, -X¹-L²-X²- is a group selected from the group consisting of lower alkylene, lower alkenylene, -(lower alkylene)-O-, and -(lower alkylene)-S-, R¹ is a group represented by the formula (II), R⁴ is H, R³ and R⁵ are each the same as or different from each other, and represent H, Cl, Br, or cyclopropyl, Ring B is phenyl, J is a single bond, and R⁶ is -CO₂H.

3. A compound selected from the group consisting of:
4-(2-{3,5-dichloro-6-[(3-isopropylphenoxy)methyl]-2-oxopyridin-1(2H)-yl}ethyl)benzoic acid,
4-(2-{3,5-dichloro-6-[2-(2-ethoxyphenyl)ethyl]-2-oxopyridin-1(2H)-yl}ethyl)benzoic acid,
4-(2-{3,5-dichloro-6-[(E)-2-(3-isopropylphenyl)vinyl]-2-oxopyridin-1(2H)-yl}ethyl)benzoic acid,
4-{2-[3,5-dichloro-2-oxo-6-{(E)-2-[2-(trifluoromethoxy)phenyl]vinyl}pyridin-1(2H)-yl]ethyl}benzoic acid,
4-{2-[3,5-dichloro-2-oxo-6-{2-[2-(trifluoromethoxy)phenyl]ethyl}pyridin-1(2H)-yl]ethyl}benzoic acid,
4-(2-{3,5-dichloro-2-oxo-6-[(3-propyl phenoxy)methyl]pyridin-1(2H)-yl}ethyl)benzoic acid,
4-(2-{3,5-dichloro-6-[(2-isopropoxyphenoxy)methyl]-2-oxopyridin-1(2H)-yl}ethyl)benzoic acid,
4-(2-{3,5-dichloro-2-oxo-6-[(E)-2-(3-propoxyphenyl)vinyl]pyridin-1(2H)-yl}ethyl)benzoic acid,
4-(2-{3-chloro-5-cyclopropyl-6-[(3-ethylphenoxy)methyl]-2-oxopyridin-1(2H)-yl}ethyl)benzoic acid,
4-{2-[3-chloro-5-cyclopropyl-2-oxo-6-{(E)-2-[3-(trifluoromethoxy)phenyl]vinyl}pyridin-1(2H)-yl]ethyl}benzoic acid,
4-{2-[3-chloro-5-cyclopropyl-2-oxo-6-{2-[3-(trifluoromethoxy)phenyl]ethyl}pyridin-1(2H)-yl]ethyl}benzoin acid,
4-(2-{5-bromo-6-[(E)-2-(3-ethylphenyl)vinyl]-2-oxopyridin-1(2H)-yl}ethyl)benzoic acid,
4-(2-{5-chloro-6-[(E)-2-(3-ethylphenyl)vinyl]-2-oxopyridin-1(2H)-yl}ethyl)benzoic acid, and
4-{2-[5-chloro-2-oxo-6-{(E)-2-[3-(trifluoromethoxy)phenyl]vinyl}pyridin-1(2H)-yl]ethyl}benzoic acid,
or a pharmaceutically acceptable salt thereof.

4. A compound selected from the group consisting of:
4-(2-{3,5-dichloro-6-[(E)-2-(3-methoxyphenyl)vinyl]-2-oxopyridin-1(2H)-yl}ethyl)benzoic acid,
4-(2-{3,5-dichloro-6-[(3-ethylphenoxy)methyl]-2-oxopyridin-1(2H)-yl}ethyl)benzoic acid,
4-(2-{3,5-dichloro-2-oxo-6-({[3-(trifluoromethoxy)phenyl]sulfanyl}methyl)pyridin-1(2H)-yl]ethyl}benzoic acid,
4-{2-[3,5-dichloro-2-oxo-6-{2-[3-(trifluoromethoxy)phenyl]ethyl}pyridin-1(2H)-yl]ethyl}benzoic acid,
4-{2-[5-bromo-2-oxo-6-{(E)-2-[3-(trifluoromethoxy)phenyl]vinyl}pyridin-1(2H)-yl]ethyl}benzoic acid,
4-(2-{5-bromo-6-[(E)-2-(3-ethylphenyl)vinyl]-2-oxopyridin-1(2H)-yl}ethyl)benzoic acid,
4-(2-{3,5-dichloro-6-[(2-ethoxyphenoxy)methyl]-2-oxopyridin-1(2H)-yl}ethyl)benzoic acid,
4-(2-{3,5-dichloro-6-[(E)-2-(3-ethylphenyl)vinyl]-2-oxopyridin-1(2H)-yl}ethyl)benzoic acid,
4-{2-[3,5-dichloro-2-oxo-6-{(E)-2-[3-(trifluoromethoxy)phenyl]vinyl}pyridin-1(2H)-yl]ethyl}benzoic acid, and
4-(2-{3,5-dichloro-6-[(E)-2-(3-ethoxyphenyl)vinyl]-2-oxopyridin-1(2H)-yl}ethyl)benzoic acid,
or a pharmaceutically acceptable salt thereof.

5. A pharmaceutical composition comprising the compound or a pharmaceutically acceptable salt thereof as described in claim 1, and a pharmaceutically acceptable excipient.

6. A pharmaceutical composition for preventing or treating peripheral arterial occlusive disease, comprising the compound or a pharmaceutically acceptable salt thereof as described in claim 1.

7. Use of the compound or a pharmaceutically acceptable salt thereof as described in claim 1 for the manufacture of an agent for preventing or treating peripheral arterial occlusive disease.

8. A method for preventing or treating peripheral arterial occlusive disease, comprising administering to a patient an effective amount of the compound or a pharmaceutically acceptable salt thereof as described in claim 1.
